# EUROPEAN PATENT APPLICATION

(11) **EP 2 420 251 A2**
(43) Date of publication of application: **22.02.2012**
(21) Application number: 11161978.9
(22) Date of filing: 10.11.2005
(51) Int. Cl.: A61K 39/395, C07K 16/24, C07K 16/26

(54) **Ligands that enhance endogenous compounds**

(30) Priority: 10.11.2004 US 985847
(62) Divisional of application: 05801585.0
(71) Applicant: Domantis Limited, Brentford, Middlesex TW8 9GS (GB)
(72) Inventor: Tomlinson, Ian M, Cambridge, CB4 0WG (GB)
(74) Representative: Easeman, Richard Lewis

(57) **Abstract**

The invention relates to ligands that comprise a moiety (e.g., a dAb) that has a binding site with binding specificity for an endogenous target compound but do not substantially inhibit the activity of said endogenous target compound. Preferably, the ligand does not bind to the active site of an endogenous target compound. The invention relates to the use of such a ligand for the manufacture of a medicament for increasing the half-life, bioavailability, activity or amount of an endogenous target compound to which the ligand binds.

SEQUENCE LISTING

<110> Domantis Limited
      Tomlinson, Ian M.

<120> LIGANDS THAT ENHANCE ENDOGENOUS
  COMPOUNDS

<130> 3440.1014001

<140> PCT/GB2005/004319
<141> 2005-11-10

<150> 10/985,847
<151> 2004-11-10

<160> 38

<170> FastSEQ for Windows Version 4.0

<210> 1
<211> 546
<212> DNA
<213> Artificial Sequence

<220>
<223> Encodes Human TNFR1 Extracellular Region

<400> 1
ctggtccctc acctagggga cagggagaag agagatagtg tgtgtcccca aggaaaatat 60
atccaccctc aaaataattc gatttgctgt accaagtgcc acaaaggaac ctacttgtac 120
aatgactgtc caggcccggg gcaggatacg gactgcaggg agtgtgagag cggctccttc 180
accgcttcag aaaaccacct cagacactgc ctcagctgct ccaaatgccg aaaggaaatg 240
ggtcaggtgg agatctcttc ttgcacagtg gaccgggaca ccgtgtgtgg ctgcaggaag 300
aaccagtacc ggcattattg gagtgaaaac cttttccagt gcttcaattg cagcctctgc 360
ctcaatggga ccgtgcacct ctcctgccag gagaaacaga acaccgtgtg cacctgccat 420
gcaggtttct ttctaagaga aaacgagtgt gtctcctgta gtaactgtaa gaaaagcctg 480
gagtgcacga agttgtgcct accccagatt gagaatgtta agggcactga ggactcaggc 540
accaca                                                            546

<210> 2
<211> 182
<212> PRT
<213> Artificial Sequence

<220>
<223> Human TNFR1 Extracellular Region

<400> 2
Leu Val Pro His Leu Gly Asp Arg Glu Lys Arg Asp Ser Val Cys Pro
 1               5                  10                  15
Gln Gly Lys Tyr Ile His Pro Gln Asn Asn Ser Ile Cys Cys Thr Lys
            20                  25                  30
Cys His Lys Gly Thr Tyr Leu Tyr Asn Asp Cys Pro Gly Pro Gly Gln
        35                  40                  45
Asp Thr Asp Cys Arg Glu Cys Glu Ser Gly Ser Phe Thr Ala Ser Glu
    50                  55                  60
Asn His Leu Arg His Cys Leu Ser Cys Ser Lys Cys Arg Lys Glu Met
65                  70                  75                  80
Gly Gln Val Glu Ile Ser Ser Cys Thr Val Asp Arg Asp Thr Val Cys
                85                  90                  95
Gly Cys Arg Lys Asn Gln Tyr Arg His Tyr Trp Ser Glu Asn Leu Phe
            100                 105                 110
Gln Cys Phe Asn Cys Ser Leu Cys Leu Asn Gly Thr Val His Leu Ser
        115                 120                 125
Cys Gln Glu Lys Gln Asn Thr Val Cys Thr Cys His Ala Gly Phe Phe
    130                 135                 140

Leu Arg Glu Asn Glu Cys Val Ser Cys Ser Asn Cys Lys Lys Ser Leu
145                 150                 155                 160
Glu Cys Thr Lys Leu Cys Leu Pro Gln Ile Glu Asn Val Lys Gly Thr
                165                 170                 175
Glu Asp Ser Gly Thr Thr
            180

<210> 3
<211> 549
<212> DNA
<213> Artificial Sequence

<220>
<223> Encodes Murine TNFR1 Extracellular Region

<400> 3
ctagtccctt ctcttggtga ccgggagaag agggatagct tgtgtcccca aggaaagtat 60
gtccattcta agaacaattc catctgctgc accaagtgcc acaaaggaac ctacttggtg 120
agtgactgtc cgagcccagg gcgggataca gtctgcaggg agtgtgaaaa gggcaccttt 180
acggcttccc agaattacct caggcagtgt ctcagttgca agacatgtcg gaaagaaatg 240
tcccaggtgg agatctctcc ttgccaagct gacaaggaca cggtgtgtgg ctgtaaggag 300
aaccagttcc aacgctacct gagtgagaca cacttccagt gcgtggactg cagcccctgc 360
ttcaacggca ccgtgacaat cccctgtaag gagactcaga acaccgtgtg taactgccat 420
gcagggttct ttctgagaga aagtgagtgc gtcccttgca gccactgcaa gaaaaatgag 480
gagtgtatga agttgtgcct acctcctccg cttgcaaatg tcacaaaccc ccaggactca 540
ggtactgcg                                                         549

<210> 4
<211> 183
<212> PRT
<213> Artificial Sequence

<220>
<223> Murine TNFR1 Extracellular Region

<400> 4
Leu Val Pro Ser Leu Gly Asp Arg Glu Lys Arg Asp Ser Leu Cys Pro
1               5                   10                  15
Gln Gly Lys Tyr Val His Ser Lys Asn Asn Ser Ile Cys Cys Thr Lys
            20                  25                  30
Cys His Lys Gly Thr Tyr Leu Val Ser Asp Cys Pro Ser Pro Gly Arg
        35                  40                  45
Asp Thr Val Cys Arg Glu Cys Glu Lys Gly Thr Phe Thr Ala Ser Gln
    50                  55                  60
Asn Tyr Leu Arg Gln Cys Leu Ser Cys Lys Thr Cys Arg Lys Glu Met
65                  70                  75                  80
Ser Gln Val Glu Ile Ser Pro Cys Gln Ala Asp Lys Asp Thr Val Cys
                85                  90                  95
Gly Cys Lys Glu Asn Gln Phe Gln Arg Tyr Leu Ser Glu Thr His Phe
            100                 105                 110
Gln Cys Val Asp Cys Ser Pro Cys Phe Asn Gly Thr Val Thr Ile Pro
        115                 120                 125
Cys Lys Glu Thr Gln Asn Thr Val Cys Asn Cys His Ala Gly Phe Phe
    130                 135                 140
Leu Arg Glu Ser Glu Cys Val Pro Cys Ser His Cys Lys Lys Asn Glu
145                 150                 155                 160
Glu Cys Met Lys Leu Cys Leu Pro Pro Pro Leu Ala Asn Val Thr Asn
                165                 170                 175
Pro Gln Asp Ser Gly Thr Ala
            180

<210> 5
<211> 21
<212> DNA
<213> Artificial Sequence

<220>
<223> Primer

<400> 5
gccagcattg ctgctaaaga a                                                21

<210> 6
<211> 19
<212> DNA
<213> Artificial Sequence

<220>
<223> Primer

<400> 6
ggtcgacggc gctattcag                                                   19

<210> 7
<211> 22
<212> DNA
<213> Artificial Sequence

<220>
<223> Primer

<400> 7
ctgcagggag tgtgagagcg gc                                               22

<210> 8
<211> 21
<212> DNA
<213> Artificial Sequence

<220>
<223> Primer

<400> 8
gtgtgtggct gcaggaagaa c                                                21

<210> 9
<211> 20
<212> DNA
<213> Artificial Sequence

<220>
<223> Primer

<400> 9
ctgccatgca ggtttctttc                                                  20

<210> 10
<211> 21
<212> DNA
<213> Artificial Sequence

<220>
<223> Primer

<400> 10
ctgcagggag tgtgaaaagg g                                                21

<210> 11
<211> 22
<212> DNA
<213> Artificial Sequence

<220>
<223> Primer

<400> 11
gtgtgtggct gtaaggagaa cc                                                22

<210> 12
<211> 20
<212> DNA
<213> Artificial Sequence

<220>
<223> Primer

<400> 12
ctgccatgca gggttctttc                                                   20

<210> 13
<211> 19
<212> DNA
<213> Artificial Sequence

<220>
<223> Primer

<400> 13
tcacactccc tgcagtccg                                                    19

<210> 14
<211> 21
<212> DNA
<213> Artificial Sequence

<220>
<223> Primer

<400> 14
cagccacaca cggtgtcccg g                                                 21

<210> 15
<211> 22
<212> DNA
<213> Artificial Sequence

<220>
<223> Primer

<400> 15
cctgcatggc aggtgcacac gg                                                22

<210> 16
<211> 19
<212> DNA
<213> Artificial Sequence

<220>
<223> Primer

<400> 16
tcacactccc tgcagactg                                                    19

<210> 17
<211> 21
<212> DNA
<213> Artificial Sequence

<220>
<223> Primer

<400> 17

cagccacaca ccgtgtcctt g                                              21

<210> 18
<211> 22
<212> DNA
<213> Artificial Sequence

<220>
<223> Primer

<400> 18
cctgcatggc agttacacac gg                                             22

<210> 19
<211> 495
<212> DNA
<213> Artificial Sequence

<220>
<223> Encodes Chimeric TNFR1

<400> 19
agtgtgtgtc cccaaggaaa atatatccac cctcaaaata attcgatttg ctgtaccaag 60
tgccacaaag gaacctactt gtacaatgac tgtccaggcc cggggcagga tacggactgc 120
agggagtgtg aaaagggcac ctttacggct tcccagaatt acctcaggca gtgcctcagt 180
tgcaagacat gtcggaaaga aatgtcccag gtggagatct ctccttgcca agctgacaag 240
gacacggtgt gtggctgtaa ggagaaccag ttccaacgct acctgagtga gacacacttc 300
cagtgcgtgg actgcagccc ctgcttcaac ggcaccgtga caatcccctg taaggagact 360
cagaacaccg tgtgtaactg ccatgcaggg ttctttctga gagaaagtga gtgcgtccct 420
tgcagccact gcaagaaaaa tgaggagtgt atgaagttgt gcctaagcgc tcatcatcat 480
catcatcatt aatga                                                     495

<210> 20
<211> 495
<212> DNA
<213> Artificial Sequence

<220>
<223> Encodes Chimeric TNFR1

<400> 20
agtgtgtgtc cccaaggaaa atatatccac cctcaaaata attcgatttg ctgtaccaag 60
tgccacaaag gaacctactt gtacaatgac tgtccaggcc cggggcagga tacggactgc 120
agggagtgtg agagcggctc cttcaccgct tcagaaaacc acctcagaca ctgcctcagc 180
tgctccaaat gccgaaagga aatgggtcag gtggagatct cttcttgcac agtggaccgg 240
gacaccgtgt gtggctgcag gaagaaccag taccggcatt attggagtga aaaccttttc 300
cagtgcttca attgcagcct ctgcctcaat gggaccgtgc acctctcctg ccaggagaaa 360
cagaacaccg tgtgcacctg ccatgcaggg ttctttctga gagaaagtga gtgcgtccct 420
tgcagccact gcaagaaaaa tgaggagtgt atgaagttgt gcctaagcgc tcatcatcat 480
catcatcatt aatga                                                     495

<210> 21
<211> 594
<212> DNA
<213> Artificial Sequence

<220>
<223> Encodes Chimeric TNFR1

<400> 21
agtgtgtgtc cccaaggaaa atatatccac cctcaaaata attcgatttg ctgtaccaag 60
tgccacaaag gaacctactt gtacaatgac tgtccaggcc cggggcagga tacggactgc 120
agggagtgtg agagcggctc cttcaccgct tcagaaaacc acctcagaca ctgcctcagc 180
tgctccaaat gccgaaagga aatgggtcag gtggagatct cttcttgcac agtggaccgg 240
gacaccgtgt gtggctgtaa ggagaaccag ttccaacgct acctgagtga gacacacttc 300
cagtgcgtgg actgcagccc ctgcttcaac ggcaccgtga caatcccctg taaggagact 360
cagaacaccg tgtgtaactg ccatgcaggt ttctttctaa gagaaaacga gtgtgtctcc 420
tgtagtaact gtaagaaaag cctggagtgc acgaagttgt gcctacccca gattgagaat 480

gttaagggca ctgaggactc aggcaccaca gcggccgcca gctttctaga acaaaaactc 540
atctcagaag aggatctgaa tagcgccgtc gaccatcatc atcatcatca ttga 594

<210> 22
<211> 594
<212> DNA
<213> Artificial Sequence

<220>
<223> Encodes Chimeric TNFR1

<400> 22
agtgtgtgtc cccaaggaaa atatatccac cctcaaaata attcgatttg ctgtaccaag 60
tgccacaaag gaacctactt gtacaatgac tgtccaggcc cggggcagga tacggactgc 120
agggagtgtg aaaagggcac ctttacggct tcccagaatt acctcaggca gtgtctcagt 180
tgcaagacat gtcggaaaga aatgtcccag gtggagatct ctccttgcca agctgacaag 240
gacacggtgt gtggctgcag gaagaaccag taccggcatt attggagtga aaaccttttc 300
cagtgcttca attgcagcct ctgcctcaat gggaccgtgc acctctcctg ccaggagaaa 360
cagaacaccg tgtgcacctg ccatgcaggt ttctttctaa gagaaaacga gtgtgtctcc 420
tgtagtaact gtaagaaaag cctggagtgc acgaagttgt gcctacccca gattgagaat 480
gttaagggca ctgaggactc aggcaccaca gcggccgcca gctttctaga acaaaaactc 540
atctcagaag aggatctgaa tagcgccgtc gaccatcatc atcatcatca ttga 594

<210> 23
<211> 594
<212> DNA
<213> Artificial Sequence

<220>
<223> Encodes Chimeric TNFR1

<400> 23
agcttgtgtc cccaaggaaa gtatgtccat tctaagaaca attccatctg ctgcaccaag 60
tgccacaaag gaacctactt ggtgagtgac tgtccgagcc cagggcggga tacagtctgc 120
agggagtgtg agagcggctc cttcaccgct tcagaaaacc acctcagaca ctgcctcagc 180
tgctccaaat gccgaaagga aatgggtcag gtggagatct cttcttgcac agtggaccgg 240
gacaccgtgt gtggctgcag gaagaaccag taccggcatt attggagtga aaaccttttc 300
cagtgcttca attgcagcct ctgcctcaat gggaccgtgc acctctcctg ccaggagaaa 360
cagaacaccg tgtgcacctg ccatgcaggt ttctttctaa gagaaaacga gtgtgtctcc 420
tgtagtaact gtaagaaaag cctggagtgc acgaagttgt gcctacccca gattgagaat 480
gttaagggca ctgaggactc aggcaccaca gcggccgcca gctttctaga acaaaaactc 540
atctcagaag aggatctgaa tagcgccgtc gaccatcatc atcatcatca ttga 594

<210> 24
<211> 120
<212> PRT
<213> Mouse

<220>
<223> Anti-Mouse TNFR1 dab

<400> 24
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Asn Arg Tyr
            20                  25                  30
Ser Met Gly Trp Leu Arg Gln Ala Pro Gly Lys Gly Leu Glu Trp Val
        35                  40                  45
Ser Arg Ile Asp Ser Tyr Gly Arg Gly Thr Tyr Tyr Glu Asp Pro Val
    50                  55                  60
Lys Gly Arg Phe Ser Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Ile Ser Gln Phe Gly Ser Asn Ala Phe Asp Tyr Trp Gly Gln
            100                 105                 110
Gly Thr Gln Val Thr Val Ser Ser
        115                 120

<210> 25
<211> 119
<212> PRT
<213> Artificial Sequence

<220>
<223> Anti-Human TNFR1 dAb

<400> 25
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Val Lys Tyr
            20                  25                  30
Ser Met Gly Trp Val Arg Gln Ala Pro Gly Lys Gly Leu Glu Trp Val
        35                  40                  45
Ser Gln Ile Ser Asn Thr Gly Gly His Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys Tyr Thr Gly Arg Trp Glu Pro Phe Asp Tyr Trp Gly Gln Gly
            100                 105                 110
Thr Leu Val Thr Val Ser Ser
        115


<210> 26
<211> 22
<212> PRT
<213> Artificial Sequence

<220>
<223> Amino Acids 59-80 of Human TNF

<400> 26
Tyr Ser Gln Val Leu Phe Lys Gly Gln Gly Cys Pro Ser Thr His Val
1               5                   10                  15
Leu Leu Thr His Thr Ile
            20


<210> 27
<211> 22
<212> PRT
<213> Artificial Sequence

<220>
<223> Amino Acids 87-108 of Human TNF

<400> 27
Tyr Gln Thr Lys Val Asn Leu Leu Ser Ala Ile Lys Ser Pro Cys Gln
1               5                   10                  15
Arg Glu Thr Pro Glu Gly
            20


<210> 28
<211> 38
<212> PRT
<213> Artificial Sequence

<220>
<223> PLAD Domain of TNFR1

<400> 28

Cys Pro Gln Gly Lys Tyr Ile His Pro Gln Asn Asn Ser Ile Cys Cys
1               5                   10                  15
Thr Lys Cys His Lys Gly Thr Tyr Leu Tyr Asn Asp Cys Pro Gly Pro
            20                  25                  30
Gly Gln Asp Thr Asp Cys
        35

<210> 29
<211> 36
<212> PRT
<213> Artificial Sequence

<220>
<223> PLAD Domain of TNFR2

<400> 29
Cys Arg Leu Arg Glu Tyr Tyr Asp Gln Thr Ala Gln Met Cys Cys Ser
1               5                   10                  15
Lys Cys Ser Pro Gly Gln His Ala Lys Val Phe Cys Thr Lys Thr Ser
            20                  25                  30
Asp Thr Val Cys
        35

<210> 30
<211> 43
<212> PRT
<213> Artificial Sequence

<220>
<223> PLAD Domain of FAS

<400> 30
Arg Leu Ser Ser Lys Ser Val Asn Ala Gln Val Thr Asp Ile Asn Ser
1               5                   10                  15
Lys Gly Leu Glu Leu Arg Lys Thr Val Thr Thr Val Glu Thr Gln Asn
            20                  25                  30
Leu Glu Gly Leu His His Asp Gly Gln Phe Cys
        35                  40

<210> 31
<211> 62
<212> PRT
<213> Artificial Sequence

<220>
<223> PLAD Domain of FAS

<400> 31
Arg Leu Ser Ser Lys Ser Val Asn Ala Gln Val Thr Asp Ile Asn Ser
1               5                   10                  15
Lys Gly Leu Glu Leu Arg Lys Thr Val Thr Thr Val Glu Thr Gln Asn
            20                  25                  30
Leu Glu Gly Leu His His Asp Gly Gln Phe Cys His Lys Pro Cys Pro
        35                  40                  45
Pro Gly Glu Arg Lys Ala Arg Asp Cys Thr Val Asn Gly Asp
    50                  55                  60

<210> 32
<211> 38
<212> PRT
<213> Artificial Sequence

<220>
<223> PLAD Domain of LTbetaR

<400> 32
Cys Arg Asp Gln Glu Lys Glu Tyr Tyr Glu Pro Gln His Arg Ile Cys
1               5                   10                  15
Cys Ser Arg Cys Pro Pro Gly Thr Tyr Val Ser Ala Lys Cys Ser Arg
            20                  25                  30
Ile Arg Asp Thr Val Cys
        35


<210> 33
<211> 34
<212> PRT
<213> Artificial Sequence

<220>
<223> PLAD Domain of CD40

<400> 33
Cys Arg Glu Lys Gln Tyr Leu Ile Asn Ser Gln Cys Cys Ser Leu Cys
1               5                   10                  15
Gln Pro Gly Gln Lys Leu Val Ser Asp Cys Thr Glu Phe Thr Glu Thr
            20                  25                  30
Glu Cys


<210> 34
<211> 41
<212> PRT
<213> Artificial Sequence

<220>
<223> PLAD Domain of CD30

<400> 34
Cys His Gly Asn Pro Ser His Tyr Tyr Asp Lys Ala Val Arg Arg Cys
1               5                   10                  15
Cys Tyr Arg Cys Pro Met Gly Leu Phe Pro Thr Gln Gln Cys Pro Gln
            20                  25                  30
Arg Pro Thr Asp Cys Arg Lys Gln Cys
        35                  40


<210> 35
<211> 36
<212> PRT
<213> Artificial Sequence

<220>
<223> PLAD Domain of CD27

<400> 35
Trp Trp Leu Cys Val Leu Gly Thr Leu Val Gly Leu Ser Ala Thr Pro
1               5                   10                  15
Ala Pro Lys Ser Cys Pro Glu Arg His Tyr Trp Ala Gln Gly Lys Leu
            20                  25                  30
Cys Cys Gln Met
        35


<210> 36
<211> 34
<212> PRT
<213> Artificial Sequence

<220>
<223> PLAD Domain of HVEM

<400> 36
Cys Lys Glu Asp Glu Tyr Pro Val Gly Ser Glu Cys Cys Pro Lys Cys
1               5                   10                  15
Ser Pro Gly Tyr Arg Val Lys Glu Ala Cys Gly Glu Leu Thr Gly Thr
            20                  25                  30
Val Cys

<210> 37
<211> 34
<212> PRT
<213> Artificial Sequence

<220>
<223> PLAD Domain of OX40

<400> 37
Val Gly Ala Arg Arg Leu Gly Arg Gly Pro Cys Ala Ala Leu Leu Leu
1               5                   10                  15
Leu Gly Leu Gly Leu Ser Thr Val Thr Gly Leu His Cys Val Gly Asp
            20                  25                  30
Thr Tyr

<210> 38
<211> 30
<212> PRT
<213> Artificial Sequence

<220>
<223> PLAD Domain of DR4

<400> 38
Ala Thr Ile Lys Leu His Asp Gln Ser Ile Gly Thr Gln Gln Trp Glu
1               5                   10                  15
His Ser Pro Leu Gly Glu Leu Cys Pro Pro Gly Ser His Arg
            20                  25                  30

## Description

### RELATED APPLICATIONS

This application is a continuation-in-part of U.S. Patent Application No. 10/985,847, filed on November 10, 2004, the entire teachings of which are incorporated herein by reference.

### BACKGROUND OF THE INVENTION

Animals, including mammals, and humans produce a variety of endogenous compounds that have activities that help maintain the normal heath and physiology of the animal. Such compounds are involved in many physiological processes such as hemostasis, immune function, metabolism, regulation of cellular proliferation and differentiation and the like. Many endogenous compounds have been isolated and synthetic or recombinant forms can be made and administered to patients to combat disease. Current therapeutic approaches often involve administering exogenous or exogenously produced forms of endogenous compounds to patients. Such therapeutic approaches can produce undesirable effects due to the high doses that are normally administered, and certain therapeutic agents, such as recombinant forms of human proteins generally must be produced by expression in mammalian cells which is expensive and produces lower yields than expression in bacterial or yeast systems.

These problems could be overcome by the development of therapeutic agents and methods that can harness and exploit the beneficial activities of endogenous compounds for producing therapeutic effects. A need exists for such therapeutic agents and methods.

### SUMMARY OF THE INVENTION

The invention relates to the use of a ligand comprising a moiety that has a binding site for an endogenous target compound wherein said ligand binds said endogenous target compound and does not substantially inhibit the activity of said endogenous target compound, for the manufacture of a medicament. Preferably, the ligand does not bind to the active site of the endogenous target compound.

The medicament can be for increasing the amount of an endogenous target compound in a subject, for increasing the bioavailability an endogenous target compound in a subject, for increasing the *in vivo* half-life of an endogenous target compound. The medicament can also be for increasing the activity (e.g., binding activity) of an endogenous target compound. The medicament can be for local or systemic delivery.

In some embodiments, the amount of endogenous target ligand in the subject about 4 hours after administration of the medicament is increased by at least 1.5 times, relative to the amount before administration of the medicament. In some embodiments, the *in vivo* half life of the endogenous target compound is increased by at least 1.5 times after administration of the medicament.

Generally, the ligand inhibits the activity of said endogenous target compound by no more than about 10%, or has an inhibitory concentration 50 (IC50) of at least 1 micromolar.

The ligand can comprise two or more copies of said moiety that has a binding site for an endogenous target. For example, the ligand can be a dimer of a moiety that has a binding site for an endogenous target, such as a dAb dimer.

The moiety that has a binding site for an endogenous target compound can be an affibody, an SpA domain, an LDL receptor class A domain, an EGF domain, an avimer, antibody or an antibody fragment (e.g., a Fv fragment, a single chain Fv fragment, a disulfide bonded Fv fragment, a Fab fragment, a Fab' fragment, a F(ab')₂ fragment, a diabody, an immunoglobulin single variable domain (e.g., a human V_{H}, and a human V_{L}, a V_{HH}).

If desired, the ligand can further comprise a half-life extending moiety as described herein. For example, the ligand can comprise a half-life extending moiety selected from the group consisting of a polyalkylene glycol moiety, serum albumin or a fragment thereof, transferrin receptor or a transferrin-binding portion thereof, or a moiety comprising a binding site for a polypeptide that enhances half-life *in vivo.* Suitable a moiety comprising a binding site for a polypeptide that enhances half-life *in vivo* include an affibody, an SpA domain, an LDL receptor class A domain, an EGF domain, an avimer, an antibody or antibody fragment, such as a Fv fragment, a single chain Fv fragment, a disulfide bonded Fv fragment, a Fab fragment, a Fab' fragment, a F(ab')₂ fragment, a diabody, and an immunoglobulin single variable domain (e.g., human V_{H}, human V_{L}, V_{HH}).

The medicaments and ligands of the invention are substantially non-immunogenic. In some embodiments, the medicament is a depot formulation.

In some embodiments, the endogenous target compound is a soluble agonist (e.g., cytokines, growth factors, hormones), soluble receptor (e.g., soluble cytokine receptors, such as soluble TNFR1, soluble TNFR2, soluble IL-1 receptor, soluble IL-4 receptor, soluble IL-13 receptor), endogenous receptor antagonist (e.g., IL-1 receptor antagonist (IL-1ra)) or an enzyme. In particular embodiments, the endogenous target compound is a soluble cytokine receptor, such as soluble TNFR1. Preferably, the ligand binds the endogenous target compound with high affinity, such as a Kd of 1 x 10⁻⁷ M or less.

In certain embodiments, the medicaments or ligands of the invention do not include an antibody, fragment or region thereof disclosed in published US patent application publication no. 2003/0144484. In certain embodiments, the medicaments or ligands of the invention do not inhibit binding of antibody A2 or chimeric antibody A2 (cA2) to human TNF-alpha. In particular embodiments, the medicaments or ligands of the invention do not bind to an epitope included in amino acids 87-108 or an epitope included in both amino acids 59-80 and 87-108 of human TNF (tumour necrosis factor). These amino acids are:
59-80: Tyr-Ser-Gln-Val-Leu-Phe-Lys-Gly-Gln-Gly-Cys-Pro-Ser-Thr-His-Val-Leu-Leu-Thr-His-Thr-Ile (SEQ ID NO:26); and
87-108: Tyr-Gln-Thr-Lys-Val-Asn-Leu-Leu-Ser-Ala-Ile-Lys-Ser-Pro-Cys-Gln-Arg-Glu-Thr-Pro-Glu-Gly (SEQ ID NO:27).

The invention also relates to the use of endogenous target compound for the manufacture of a medicament for increasing the activity of said endogenous target, wherein said ligand binds said endogenous target and does not substantially inhibit the activity of said endogenous target compound. Preferably, the ligand does not bind to the active site of said endogenous target compound. In some embodiments, the binding activity of the endogenous target ligand is increased, for example the binding activity (e.g., affinity, avidity) can be increased by a factor of at least 10.

The invention also relates to a ligand that binds an endogenous target compound having activity suitable for treating a disease in a subject, wherein said ligand does not bind the active site of said endogenous target compound or substantially inhibit the activity of said endogenous target compound, for use in therapy of a disease amenable to treatment with said endogenous target compound.

The invention also relates to a pharmaceutical composition comprising a ligand that binds an endogenous target compound having activity suitable for treating a disease in a subject and a physiologically acceptable carrier, wherein said ligand does not bind the active site of said endogenous target compound or substantially inhibit the activity of said endogenous target compound.

The invention also relates to a drug delivery device comprising a pharmaceutical composition of the invention. In some embodiments that drug delivery device is a parenteral delivery device, intravenous delivery device, intramuscular delivery device, intraperitoneal delivery device, transdermal delivery device, pulmonary delivery device, intraarterial delivery device, intrathecal delivery device, intraarticular delivery device, subcutaneous delivery device, intranasal delivery device, vaginal delivery device, and rectal delivery device. For example, the drug delivery device can be a syringe, a transdermal delivery device, a capsule, a tablet, a nebulizer, an inhaler, an atomizer, an aerosolizer, a mister, a dry powder inhaler, a metered dose inhaler, a metered dose sprayer, a metered dose mister, a metered dose atomizer, a catheter.

The invention relates to a method for increasing the half-life of an endogenous target compound in a subject, comprising administering to a subject in need thereof an effective amount of a ligand comprising a moiety that has a binding site with binding specificity for said endogenous target compound.

The invention relates to a method for increasing the amount of an endogenous target compound in a subject, comprising administering to a subject in need thereof an effective amount of a ligand comprising a moiety that has a binding site with binding specificity for said endogenous target compound.

The invention relates to a method for increasing the bioavailability of an endogenous target compound in a subject, comprising administering to a subject in need thereof an effective amount of a ligand comprising a moiety that has a binding site with binding specificity for said endogenous target compound.

The invention relates to a method for increasing the activity (e.g., binding activity) of an endogenous target compound in a subject, comprising administering to a subject in need thereof an effective amount of a ligand comprising a moiety that has a binding site with binding specificity for said endogenous target compound.

The invention relates to a method for treating a subject having a disease that is amenable to treatment with an endogenous target compound in a subject, comprising administering to a subject in need thereof an effective amount of a ligand comprising a moiety that has a binding site with binding specificity for said endogenous target compound.

The invention also relates to novel ligands (e.g., dAbs) described herein.

### BRIEF DESCRIPTION OF THE DRAWINGS

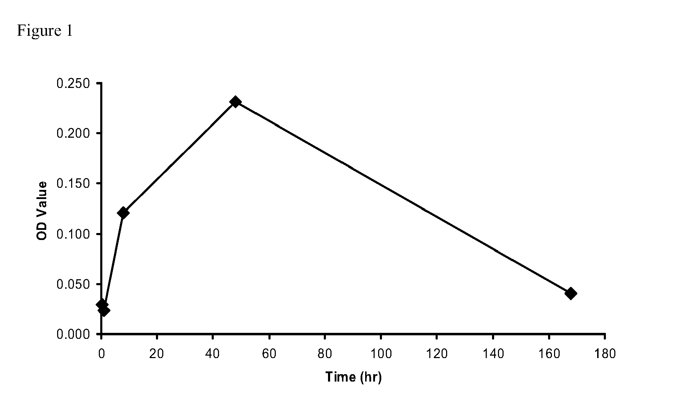

Figure 1 is a graph of the relative concentration of soluble TNFR1 detected by ELISA in the serum over time (concentration time curve) following a single intravenous administration of a PEGylated dAb monomer that binds TNFR1 (TAR2m-21-23/40K branched PEG; also referred to as PEG anti-TNFR1 dAb). The plotted data points are were obtained by ELISA using a 1:5 dilution of serum. The graph clearly shows that the bioavailability of TNFR1 in the systemic circulation was increased by the administration of a PEGylated dAb monomer that binds TNFR1. As shown by the concentration time curve, the level of soluble TNFR1 in serum increased after the PEGylated dAb monomer was administered, reach a peak concentration, and then decreased over time to basal levels as the PEGylated dAb was cleared. The results demonstrate that administering a dAb that binds a soluble receptor can increase the bioavailability of the receptor and increase the level or amount of soluble receptor in the systemic circulation, and that the increased level or amount of soluble receptor in the systemic circulation is cleared and returns to basal levels. The results indicate that the level of soluble receptor in the systemic circulation can be controlled by administering a PEGylated dAb monomer that binds the soluble receptor.

### DETAILED DESCRIPTION OF THE INVENTION

Within this specification embodiments have been described in a way which enables a clear and concise specification to be written, but it is intended and will be appreciated that embodiments may be variously combined or separated without parting from the invention.

As used herein, the term "ligand" refers to a compound that comprises at least one peptide, polypeptide or protein moiety that has a binding site with binding specificity for a desired endogenous target compound. For example, the moiety that has a binding site for a desired endogenous target compound can be an immunoglobulin single variable domain (*e.g*., V_{H}, V_{L}, V_{HH}) that has binding specificity for a desired endogenous target compound (*e.g*., a soluble receptor protein, endogenous receptor antagonist, cytokine or growth factor). The moiety that has a binding site for an endogenous target compound can also comprises one or more complementarity determining regions (CDRs) of an immunoglobulin single variable domain that has binding specificity for a desired endogenous target compound in a suitable format, such that the moiety has binding specificity for the endogenous target compound. For example, the CDRs can be grafted onto a suitable protein scaffold or skeleton, such as an affibody, an SpA scaffold, an LDL receptor class A domain, or an EGF domain. Further, the ligand can be monovalent (e.g., a dAb monomer), bivalent (homobivalent, heterobivalent) or multivalent (homomultivalent, heteromultivalent) as described herein. Thus, "ligands" include polypeptides that consist of a dAb, include polypeptides that consist essentially of such a dAb, polypeptides that comprise a dAb (or the CDRs of a dAb) in a suitable format, such as an antibody format (*e.g*., IgG-like format, scFv, Fab, Fab', F(ab')₂) or a suitable protein scaffold or skeleton, such as an affibody, an SpA scaffold, an LDL receptor class A domain or an EGF domain, dual specific ligands that comprise a dAb that binds a first endogenous target compound and a second dAb that binds another endogenous target compound (*e.g*., serum albumin), and multispecific ligands as described herein. The moiety that has a binding site for an endogenous target compound can also be a protein domain comprising a binding site for a desired target, *e.g*., a protein domain is selected from an affibody, an SpA domain, an LDL receptor class A domain, an EGF domain, an avimer (see, *e.g*., U.S. Patent Application Publication Nos. 2005/0053973, 2005/0089932, 2005/0164301). If desired, a "ligand" can further comprise one or more additional moieties, that can each independently be a peptide, polypeptide or protein moiety or a non-peptidic moiety (*e.g*., a polyalkylene glycol, a lipid, a carbohydrate). For example, the ligand can further comprise a half-life extending moiety as described herein (*e.g*., a polyalkylene glycol moiety, a moiety comprising albumin, an albumin fragment or albumin variant, a moiety comprising transferrin, a transferrin fragment or transferrin variant, a moiety that binds albumin, a moiety that binds neonatal Fc receptor).

As used herein, the term "endogenous target compound" refers to soluble compound that is produced by a subject (e.g., a mammal, a human) of interest. Endogenous target compounds include, for example, soluble agonists (e.g., cytokines, growth factors, hormones), soluble receptors (e.g., soluble cytokine receptors, such as soluble TNFR1, soluble, soluble TNFR2, soluble IL-1 receptor, soluble IL-4 receptor, soluble IL-13 receptor), endogenous receptor antagonists (e.g., IL-1 receptor antagonist (IL-1ra)), enzymes, and the like.

As used herein, the term "active site" refers to the site or domain of an endogenous target compound (e.g., protein) that interacts with (e.g., binds) an endogenous binding partner of an endogenous target compound (e.g., site or domain of a receptor protein that makes contact with the ligand of the receptor, site or domain of an agonist protein (e.g., a cytokine) that makes contact with the receptor of the agonist, the catalytic domain of an enzyme). For example, as used herein, the "active site" of a soluble endogenous compound such as a cytokine, growth factor or hormone is the site on the cytokine, growth factor or hormone that makes contact with the endogenous receptor that binds the cytokine, growth factor or hormone.

As used herein, "bioavailability" refers to the extent to which an endogenous target compound or ligand-endogenous target compound complex is present or gains access to a desired site of action (*e.g*., the systemic circulation, the central nervous system, a local site of action (*e.g.,* a particular organ, a particular area of tissue (*e.g*., muscle, skin))). Bioavailability of an endogenous target compound can be increased independently of any change is the *in vivo* half-life of the endogenous target compound, or of any change in the amount of the endogenous target compound in a subject. For example, a ligand may bind an endogenous target compound that has beneficial activity in the systemic circulation but is rapidly distributed into the tissues, and slow the rate of distribution into the tissues. Such a ligand would increase bioavailability of the endogenous target compound in the systemic circulation where the beneficial activity of the endogenous target compound can have therapeutic effect.

As used herein, "binding activity" refers to the capacity of binding partners to bind to each other. For example, the capacity of a receptor or soluble receptor to bind its cognate ligand. Binding activity can be expressed, for example, as affinity (K_{d}; K_{d}=K_{off}/Kₒₙ) or avidity. Binding activity can be increased, for example, by increasing affinity (e.g., increasing the rate of binding (Kₒₙ) or decreasing the rate of dissociation (K_{off})) or by increasing avidity. Avidity can be increased, for example, by increasing the number of sites at which a first binding partner and a second binding partner make contact.

As used herein, the phrase "substantially non-immunogenic" means high affinity antibodies that bind ligand, ligand-endogenous target compound or endogenous target compound are not produced with a ligand is administered to a subject. High affinity antibodies bind the ligand, ligand-endogenous target compound complex or endogenous target compound with an affinity of 500 nM or lower, or 300 nM or lower, or 100 nM or lower, or 10 nM or lower, or 1 nM or lower. Antibodies that bind ligand, ligand-endogenous target compound complex or endogenous target compound and the affinity of such antibodies can be identified using any suitable method. Several such methods are well-known in the art. For example, a serum sample can be obtained from a human to whom a ligand has been administered (*e.g*., about 1 week, 2 weeks, 3 weeks, 4 weeks after administration) and the serum can be tested for presence of antibodies that bind ligand, ligand-endogenous target compound or endogenous target compound using, for example an ELISA or other suitable assay. The affinity of antibodies can be determined using any suitable method, such as by equilibrium dialysis or by surface plasmon resonance.

The phrase "immunoglobulin single variable domain" refers to an antibody variable region (V_{H}, V_{HH}, V_{L}) that specifically binds an antigen or epitope independently of other V regions or domains; however, as the term is used herein, an immunoglobulin single variable domain can be present in a format (e.g., homo- or hetero-multimer) with other variable regions or variable domains where the other regions or domains are not required for antigen binding by the single immunoglobulin variable domain (i.e., where the immunoglobulin single variable domain binds antigen independently of the additional variable domains). "Immunoglobulin single variable domain" encompasses not only an isolated antibody single variable domain polypeptide, but also larger polypeptides that comprise one or more monomers of an antibody single variable domain polypeptide sequence. A "domain antibody" or "dAb" is the same as an "immunoglobulin single variable domain" polypeptide as the term is used herein. An immunoglobulin single variable domain polypeptide, as used herein refers to a mammalian immunoglobulin single variable domain polypeptide, preferably human, but also includes rodent (for example, as disclosed in WO 00/29004, the contents of which are incorporated herein by reference in their entirety) or camelid V_{HH} dAbs. Camelid dAbs are immunoglobulin single variable domain polypeptides which are derived from species including camel, llama, alpaca, dromedary, and guanaco, and comprise heavy chain antibodies naturally devoid of light chain: V_{HH}. V_{HH} molecules are about ten times smaller than IgG molecules, and as single polypeptides, they are very stable, resisting extreme pH and temperature conditions.

"Complementary" Two immunoglobulin domains are "complementary" where they belong to families of structures which form cognate pairs or groups or are derived from such families and retain this feature. For example, a V_{H} domain and a V_{L} domain of an antibody are complementary; two V_{H} domains are not complementary, and two V_{L} domains are not complementary. Complementary domains may be found in other members of the immunoglobulin superfamily, such as the V_{α} and V_{β} (or γ and δ) domains of the T-cell receptor. Domains which are artificial, such as domains based on protein scaffolds which do not bind epitopes unless engineered to do so, are non-complementary. Likewise, two domains based on (for example) an immunoglobulin domain and a fibronectin domain are not complementary.

"Immunoglobulin family" This refers to a family of polypeptides which retain the immunoglobulin fold characteristic of antibody molecules, which contains two β sheets and, usually, a conserved disulphide bond. Members of the immunoglobulin superfamily are involved in many aspects of cellular and non-cellular interactions *in vivo,* including widespread roles in the immune system (for example, antibodies, T-cell receptor molecules and the like), involvement in cell adhesion (for example the ICAM molecules) and intracellular signaling (for example, receptor molecules, such as the PDGF receptor). The present invention is applicable to all immunoglobulin superfamily molecules which possess binding domains. Preferably, the present invention relates to antibodies.

"Domain" A domain is a folded protein structure which retains its tertiary structure independently of the rest of the protein. Generally, domains are responsible for discrete functional properties of proteins, and in many cases may be added, removed or transferred to other proteins without loss of function of the remainder of the protein and/or of the domain. By single antibody variable domain is meant a folded polypeptide domain comprising sequences characteristic of antibody variable domains. It therefore includes complete antibody variable domains and modified variable domains, for example in which one or more loops have been replaced by sequences which are not characteristic of antibody variable domains, or antibody variable domains which have been truncated or comprise N- or C-terminal extensions, as well as folded fragments of variable domains which retain at least in part the binding activity and specificity of the full-length domain.

"Repertoire" A collection of diverse variants, for example polypeptide variants which differ in their primary sequence. A library used in the present invention will encompass a repertoire of polypeptides comprising at least 1000 members.

"Library" The term library refers to a mixture of heterogeneous polypeptides or nucleic acids. The library is composed of members, each of which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. Preferably, each individual organism or cell contains only one or a limited number of library members. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

"Antibody" An antibody (for example IgG, IgM, IgA, IgD or IgE) or fragment (such as a Fab , F(ab')₂, Fv, disulphide linked Fv, scFv, closed conformation multispecific antibody, disulphide-linked scFv, diabody) whether derived from any species naturally producing an antibody, or created by recombinant DNA technology; whether isolated from serum, B-cells, hybridomas, transfectomas, yeast or bacteria).

"Dual-specific ligand" A ligand comprising a first immunoglobulin single variable domain and a second immunoglobulin single variable domain as herein defined, wherein the variable regions are capable of binding to two different antigens or two epitopes on the same antigen which are not normally bound by a monospecific immunoglobulin. For example, the two epitopes may be on the same hapten, but are not the same epitope or sufficiently adjacent to be bound by a monospecific ligand. The dual specific ligands according to the invention are composed of variable domains which have different specificities, and do not contain mutually complementary variable domain pairs which have the same specificity. Dual-specific ligands and suitable methods for preparing dual-specific ligands are disclosed in WO 2004/058821, WO 2004/003019, and WO 03/002609, the entire teachings of each of these published international applications are incorporated herein by reference.

"Antigen" A molecule that is bound by a ligand according to the present invention. Typically, antigens are bound by antibody ligands and are capable of raising an antibody response *in vivo.* It may be a polypeptide, protein, nucleic acid or other molecule. Generally, the dual specific ligands according to the invention are selected for target specificity against a particular antigen. In the case of conventional antibodies and fragments thereof, the antibody binding site defined by the variable loops (L1, L2, L3 and H1, H2, H3) is capable of binding to the antigen.

"Epitope" A unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation.

"Universal framework" A single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917. The invention provides for the use of a single framework, or a set of such frameworks, which has been found to permit the derivation of virtually any binding specificity though variation in the hypervariable regions alone.

"Half-life" The time taken for the serum concentration of the ligand to reduce by 50%, *in vivo,* for example due to degradation of the ligand and/or clearance or sequestration of the ligand by natural mechanisms. The ligands of the invention are stabilised *in vivo* and their half-life increased by binding to molecules which resist degradation and/or clearance or sequestration. Typically, such molecules are naturally occurring proteins which themselves have a long half life *in vivo.* The half-life of a ligand is increased if its functional activity persists, *in vivo,* for a longer period than a similar ligand which is not specific for the half-life increasing molecule. Thus, a ligand specific for HSA and a target molecule is compared with the same ligand wherein the specificity for HSA is not present, that it does not bind HSA but binds another molecule. For example, it may bind a second epitope on the target molecule. Typically, the half life is increased by 10%, 20%, 30%, 40%, 50% or more. Increases in the range of 2x, 3x, 4x, 5x, 10x, 20x, 30x, 40x, 50x or more of the half life are possible. Alternatively, or in addition, increases in the range of up to 30x, 40x, 50x, 60x, 70x, 80x, 90x, 100x, 150x of the half life are possible.

"Substantially identical (or "substantially homologous")" A first amino acid or nucleotide sequence that contains a sufficient number of identical or equivalent (*e.g*., with a similar side chain, *e.g*., conserved amino acid substitutions) amino acid residues or nucleotides to a second amino acid or nucleotide sequence such that the first and second amino acid or nucleotide sequences have similar activities. In the case of antibodies, the second antibody has the same binding specificity and has at least 50% of the affinity of the same.

As referred to herein, the term "about" is preferably interpreted to mean plus or minus 20%, more preferably plus or minus 10%, even more preferably plus or minus 5%, even more preferably plus or minus 2%, most preferably plus or minus 1%.

As used herein, the terms "low stringency," "medium stringency," "high stringency," or "very high stringency conditions" describe conditions for nucleic acid hybridization and washing. Guidance for performing hybridization reactions can be found in Current Protocols in Molecular Biology, John Wiley & Sons, N.Y. (1989), 6.3.1-6.3.6, which is incorporated herein by reference in its entirety. Aqueous and nonaqueous methods are described in that reference and either can be used. Specific hybridization conditions referred to herein are as follows: (1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); (2) medium stringency hybridization conditions in 6X SSC at about 45°C, followed by one or more washes in 0.2X SSC, 0.1% SDS at 60°C; (3) high stringency hybridization conditions in 6X SSC at about 45°C, followed by one or more washes in 0.2X SSC, 0.1% SDS at 65°C; and preferably (4) very high stringency hybridization conditions are 0.5M sodium phosphate, 7% SDS at 65°C, followed by one or more washes at 0.2X SSC, 1% SDS at 65°C. Very high stringency conditions (4) are the preferred conditions and the ones that should be used unless otherwise specified.

As referred to herein, the term "competes" means that the binding of a first epitope to its cognate epitope binding domain is inhibited when a second epitope is bound to its cognate epitope binding domain. For example, binding may be inhibited sterically, for example by physical blocking of a binding domain or by alteration of the structure or environment of a binding domain such that its affinity or avidity for an epitope is reduced.

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences.

Amino acid and nucleotide sequence alignments and homology, similarity or identity, as defined herein are preferably prepared and determined using the algorithm BLAST 2 Sequences, using default parameters (Tatusova, T. A. et al., FEMS Microbiol Lett, 174:187-188 (1999)). Alternatively, advantageously, the BLAST algorithm (version 2.0) is employed for sequence alignment, with parameters set to default values. The BLAST algorithm is described in detail at the world wide web site ("www") of the National Center for Biotechnology Information (".ncbi") of the National Institutes of Health ("nih") of the U.S. government (".gov"), in the "/Blast/" directory, in the "blast_help.html" file. The search parameters are defined as follows, and are advantageously set to the defined default parameters.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, Proc. Natl. Acad. Sci. USA 87(6):2264-8 (see the "blast_help.html" file, as described above) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul et al. (1994).

The five BLAST programs available at the National Center for Biotechnology Information web site perform the following tasks:
"blastp" compares an amino acid query sequence against a protein sequence database;
"blastn" compares a nucleotide query sequence against a nucleotide sequence database;
"blastx" compares the six-frame conceptual translation products of a nucleotide query sequence (both strands) against a protein sequence database;
"tblastn" compares a protein query sequence against a nucleotide sequence database dynamically translated in all six reading frames (both strands).
"tblastx" compares the six-frame translations of a nucleotide query sequence against the six-frame translations of a nucleotide sequence database.

BLAST uses the following search parameters:
HISTOGRAM Display a histogram of scores for each search; default is yes. (See parameter H in the BLAST Manual).
DESCRIPTIONS Restricts the number of short descriptions of matching sequences reported to the number specified; default limit is 100 descriptions. (See parameter V in the manual page). See also EXPECT and CUTOFF.
ALIGNMENTS Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).
EXPECT The statistical significance threshold for reporting matches against database sequences; the default value is 10, such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).
CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.
MATRIX Specify an alternate scoring matrix for BLASTP, BLASTX, TBLASTN and TBLASTX. The default matrix is BLOSUM62 (Henikoff & Henikoff, 1992, Proc. Natl. Acad. Sci. USA 89(22):10915-9). The valid alternative choices include: PAM40, PAM120, PAM250 and IDENTITY. No alternate scoring matrices are available for BLASTN; specifying the MATRIX directive in BLASTN requests returns an error response.
STRAND Restrict a TBLASTN search to just the top or bottom strand of the database sequences; or restrict a BLASTN, BLASTX or TBLASTX search to just reading frames on the top or bottom strand of the query sequence.
FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States, 1993, Computers and Chemistry 17:191-201, or, for BLASTN, by the DUST program of Tatusov and Lipman (see the world wide web site of the NCBI). Filtering can eliminate statistically significant but biologically uninteresting reports from the blast output (e.g., hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "N" repeated 13 times) and the letter "X" in protein sequences (e.g., "X" repeated 9 times).

Filtering is only applied to the query sequence (or its translation products), not to database sequences. Default filtering is DUST for BLASTN, SEG for other programs.

It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect. NCBI-gi Causes NCBI gi identifiers to be shown in the output, in addition to the accession and/or locus name. Most preferably, sequence comparisons are conducted using the simple BLAST search algorithm provided at the NCBI world wide web site described above, in the "/BLAST" directory.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. which are incorporated herein by reference) and chemical methods.

The invention relates to ligands that comprise a binding moiety that has binding specificity for a desired endogenous target compound, to such ligands for use in therapy and to the use of such ligands for the manufacture of a medicament and to therapeutic methods that comprise administering such ligands.

It has been discovered that agents that bind soluble endogenous target compounds but do not substantially alter the activity of such compounds, such as antibodies and antibody fragments (e.g., Fab fragment, Fab' fragment, F(ab')₂ fragment, Fv fragment (e.g., scFv, disulfide bonded Fv), dAb, diabody), can be used to alter certain properties of the endogenous target compound, and thereby make the endogenous target compound more effective for treating, suppressing or mitigating a disease, or for diagnostic purposes. Accordingly, the invention relates to ligands, as described herein, that bind a soluble endogenous target compound but do not substantially inhibit the activity of the endogenous target compound (*e.g.*, do not bind the active site of the endogenous target compound), but that are useful for altering certain properties of the endogenous target compounds. For example, a ligand that comprises a binding moiety that has binding specificity for a desired endogenous target compound can bind the endogenous target compound, thereby increasing the hydrodynamic size of the endogenous target compound and increasing the serum half-life of the endogenous target compound. Beneficially, the endogenous target compound retains its biological activity when bound by the ligand (e.g., is active in a complex consisting of ligand and endogenous target compound), and can produce beneficial effects for therapeutic and/or diagnostic purposes.

The ligand, as described herein, can increase the serum half-life of an endogenous target compound by, for example, reducing the rate of clearance of the endogenous target compound. In such a situation, the amount of endogenous target compound in a subject (e.g., in the systemic circulation) can increase to a amount that is greater than the amount normally present in a subject, and the increased amount of endogenous target ligand can produce beneficial therapeutic effects that are not produce by lower or normal amounts of endogenous target compound.

A ligand that comprise a binding moiety that has binding specificity for a desired endogenous target compound can bind the endogenous target compound and increase the bioavailability of the endogenous target compound independent of any change is the *in vivo* half-life of the endogenous target compound, or of any change in the amount of the endogenous target compound in a subject. For example, a ligand may bind an endogenous target compound that has beneficial activity in the systemic circulation but is rapidly distributed into the tissues or cleared (e.g., a steroid or peptide hormone), and slow the rate of distribution or clearance. Such a ligand can increase bioavailability of the endogenous target compound in the systemic circulation where the activity of the endogenous target compound can have a beneficial (e.g., therapeutic) effect.

A ligand that comprise a binding moiety that has binding specificity for a desired endogenous target compound can bind the endogenous target compound and increase the activity (e.g., binding activity) of the endogenous target compound. For example, a ligand may bind an endogenous target compound such as an endogenous agonist (e.g., a cytokine) or a soluble cytokine receptor and improve the binding activity of the endogenous target compound by increasing the affinity and/or avidity of the endogenous target compound for its endogenous binding partner. For example, a ligand that comprises two or more moieties that have binding sites with binding specificity for a desired cytokine can bind two or more individual cytokine molecules thereby producing a cytokine dimer, trimer, oligomer or multimer. Such a dimer, trimer, oligomer or multimer will have improved binding activity toward, for example, a cell that expresses the receptor for the cytokine due to higher avidity. Similarly, the binding activity of soluble receptors (e.g., soluble TNFR1) can be increased by a ligand that comprises two or more binding sites for soluble TNFR1. Such a ligand can, for example, bind two soluble TNFR1 chains forming a soluble TNFR1 dimer that will bind trimeric TNF with higher avidity that a single TNFR1 chain.

The ligands described herein can be administered systemically, for example, to bind endogenous target ligands in the systemic circulation and produce beneficial effects in the systemic circulation. Additionally, the ligands can be administered locally (e.g., by subcutaneous injection, intramuscular injection, intra-articular injection, intradermal injection, inhalation, and the like) and thereby improve local bioavailability of an endogenous target compound or to increase the amount of endogenous target compound at the site where the ligand is locally administered. For example, a depot formulation of a ligand can be administered locally by subcutaneous injection, and the administered ligand can recruit endogenous target ligand to the site of the depot and bind the endogenous target ligand to improve local bioavailability or to produce a locally high amount of endogenous target ligand. Such an approach is advantageous, for example, to promote wound healing, or to inhibit local inflammatory reactions (e.g., in the skin, in the lung, in a joint).

The invention provides several advantages over conventional therapeutic agents and approaches. For example, the ligands described herein are generally small polypeptides that can be produced economically and in large quantities using bacterial or yeast expression systems, rather than more expensive mammalian expression systems which produce lower yields. In addition, the ligands described herein are administered to harness and exploit the beneficial activities of compounds (e.g., proteins) that are endogenous to the patient, and generally the ligand is composed of protein domains (e.g. a dAb) that are of the same origin as the patient (e.g., human origin) and therefore are substantially non-immunogenic. Advantageously, this significantly reduces or eliminates the risk that the patient will produce high affinity neutralizing antibodies against the ligand, ligand-endogenous target compound complex or endogenous target compound. This is a distinct advantage over therapeutic approaches that comprise administering exogenous compounds, or even recombinantly produced endogenous proteins, which frequently induce the production of high affinity antibodies in the patient which can restrict or eliminate efficacy of the therapeutic agent.

### Ligands That Bind An Endogenous Target Compound

The invention relates to ligands that comprise a moiety (e.g., dAb) that has a binding site with binding specificity for an endogenous target compound but do not substantially inhibit the activity of said endogenous target compound. Preferably, the ligand does not bind to the active site of an endogenous target compound.

The ligands described herein do not substantially inhibit the activity of the endogenous target compounds to which they bind, and the endogenous target compound remains active when bound by the ligand. For example, a soluble receptor is bound by a ligand of the invention, and the soluble receptor in the resulting receptor-ligand complex can bind the endogenous ligand of the soluble receptor. In another example, an enzyme is bound by a ligand of the invention, and the enzyme in the resulting enzyme-ligand complex can bind substrate and catalyze the enzymatic reaction.

Generally, the ligand inhibits the activity of the endogenous target compound to which it binds by no more than about 10%, no more than about 9%, no more than about 8%, no more than about 7%, no more than about 6%, no more than about 5%, no more than about 4%, no more than about 3%, no more than about 2%, no more than about 1%, or has substantially no inhibitory effect on the activity of the endogenous target compound. In some examples, a ligand that does not substantially inhibit the activity of an endogenous target compound to which it binds, inhibits that activity of said endogenous target compound with an high inhibitory concentration 50 (IC50), such as an IC50 of at least 1 nanomolar, at least 10 nanomolar, at least 100 nanomolar, at least 1 micromolar, or at least 10 micromolar.

The ability of a ligand to inhibit of the activity of the endogenous target compound can be assessed using any suitable *in vitro* or *in vivo* assay. Many suitable assays are well-known in the art and routinely used to assess inhibitory activity of compounds. For example, the ability of ligands that comprise a moiety that has a binding site for TNFR1 to inhibit the activity or TNFR1 can be assessed using *in vivo* models of endotoxin shock or TNF-induced skin necrosis (see, Sheehan et al., J. Exp. Med., 181:607-617 (1995)), and/or the in vitro receptor binding assay, L929 cytotoxicity assay, HeLa IL-8 assay, or MRC-5 IL-8 release assay described herein. Other suitable assays for assessing whether a ligand inhibits the activity of a desired endogenous target compound, such as those described herein, are well known in the art.

The ligands *per se* generally do not have substantial therapeutic activity but are used to harness and exploit the activity of endogenous target compounds for therapeutic or diagnostic purposes. In some embodiments, the ligands have no substantial activity in a suitable *in vitro* assay for the activity of an endogenous target compound. For example, a ligand that binds an endogenous target compound and increases the half-life of the endogenous target compound will generally not alter the activity of an endogenous target compound in the assay in comparison to the activity of the endogenous target compound in the same assay but without ligand.

Ligands (e.g., dAbs) that bind a desired endogenous target compound, such as a receptor protein, a soluble agonist (e.g., a cytokine), or an enzyme, but do not bind the active site of the endogenous target compound can be identified using any suitable method. For example, individual ligands, or moieties that have a binding site for an endogenous target compound or collections of such moieties (e.g., a library or repertoire) can be screened in a suitable competition assay in which binding of an endogenous binding partner (e.g., natural ligand, substrate, cofactor) to the endogenous target compound is assessed in the presence of the ligand or moiety (or collection of ligands or moieties) being screened. No substantial reduction (e.g., inhibition) in binding of the endogenous binding partner to the endogenous target compound relative to a suitable control indicates that the ligand or moiety being screened does not bind the active site of the endogenous target compound.

Many suitable assays for screening ligands and moieties that comprise a binding site for an endogenous target compounds, such as enzymes, receptors (e.g., cytokine receptors, growth factor receptors), and soluble agonists (e.g., cytokines, chemokines, growth factors, hormones) are well-known in the art. Additionally, competition assays are routinely used to screen collections of compounds that contain 10⁶ to 10⁹ or more members. Thus, such screening activity is within the ordinary skill in the art and is not considered burdensome or undue by those in the art. For example, the variable domains disclosed in WO 03002609, WO 2004101790, WO 2005035572, WO 2004061026, WO 2004003019, WO 2004058821, WO 9404678, WO 9749805, WO 9923221, WO 9937681, WO 0024884, WO 0043507, WO 0065067, WO 0140310, WO 03035694, WO 03053531, WO 03054015, WO 0305527, WO 2004015425, WO 2004041862, WO 2004041863, WO 2004041865, WO 2004062551, WO 2005044858 and EP1134231 can be screened for the ability to bind a desired endogenous target compound without binding to the active site in a suitable competition assay. The disclosure of the variable domains, their sequences and methods of production and selection in the foregoing documents are being explicitly incorporated by reference herein to provide the skilled addressee with examples of moieties that have a binding site for an endogenous target compound for use in the invention.

The ligands can increase the half-life of the endogenous targets to which they bind, increase the amount of endogenous targets to which they bind in a subject (e.g., the systemic amount, the amount at a desire site or location), and/or increase the bioavailability of the endogenous targets to which they bind.

A ligand can increase the half-life of an endogenous target compound to which it binds by, for example, by binding the endogenous target compound and thereby increasing the hydrodynamic size of the endogenous target compound, by binding and stabilizing the endogenous target compound, or by binding and slowing the rate of clearance or metabolism of the endogenous target compound. Generally, the ligand will increase the half-life of the endogenous target compound to which it binds by a factor of at least about 1.5, at least about 2, at least about 3, at least about 4, at least about 5, at least about 6, at least about 7, at least about 8, at least about 9, or at least about 10, relative to the half-life of the endogenous target compound in the absence of ligand. For example, an endogenous target compound can have a half-life of about 1 hour, and after administration of a ligand that comprises a moiety with a binding site that binds said endogenous target compound, the half-life of endogenous target compound can be increased to about 1.5 hours, to about 10 hours, or longer.

The level or amount of endogenous target compound can increase following administration of a ligand, as described herein, due to the ligands half-life increasing effect. For example, the level or amount of endogenous target ligand (e.g., in serum) 1 hour, or 2 hours, or 3 hours, or 4 hours, or 5 hours, or 6 hours, or 7 hours, or 8 hours, or 9 hours, or 10 hours, or 11 hours, or 12 hours, or 1 day after administration of a ligand of the invention can be increased by a factor of at least about 1.5, at least about 2, at least about 3, at least about 4, at least about 5, at least about 10, at least about 50, at least about 100, at least about 500, at least about 1,000, at least about 5,000, at least about 10,000, at least about 50,000, or at least about 100,000. Increase levels or amounts of an endogenous target compound can be readily detected using any suitable method. For example, a suitable sample (e.g., serum) can be obtain prior to administration of the ligand and at one or more time points after administration, and the level or amount of endogenous target compound in the samples can be determined using any suitable method. The sample can be, for example, a sample of blood, serum, cerebrospinal fluid, synovial fluid, bronchoalveolar lavage, enema or a biopsy. In some embodiments, the amount or level of ligand in such a sample is increased, relative to the amount or level before the ligand is administered.

The ligands can increase the bioavailability of an endogenous target compound. The bioavailability can be increase systemically, or at a desired site of action (e.g., at a site where the ligand is locally administered). For example, a ligand may bind an endogenous target compound that has beneficial activity in the systemic circulation but is rapidly distributed into the tissues or cleared (e.g., a steroid or peptide hormone), and slow the rate of distribution or clearance. Such a ligand can increase bioavailability of the endogenous target compound in the systemic circulation where the activity of the endogenous target compound can have a beneficial (e.g., therapeutic) effect. A ligand can increases the bioavailability of an endogenous target compound at a desired site by recruiting the endogenous target compound to that site. For example, a ligand comprising a binding site for a desired endogenous target compound can be locally administered to a patient (e.g., as a depot formulation). The locally administered ligand can bind the desired endogenous target compound, and recruit additional endogenous target compound to the site. Thus, an increased level or amount of endogenous target compound can be present at the site to produce beneficial effects. For example, in one embodiment, the ligand comprises a moiety with a binding site for TGF beta isoform 3. Such a ligand can be locally administered to bind TGF beta isoform 3 and recruit TGF beta isoform 3 to the site of local administration, and thus promote would healing.

Similar ligands that comprise a moiety with a binding site for a desired cell surface target can be prepared using the methods described herein and used, for example, to recruit a desired cell type to a desire site (e.g., site of injury, site of inflammation).

Generally, the ligand will increase bioavailability (e.g., in the systemic circulation, at a desired site of action) of the endogenous target compound to which it binds by a factor of at least about 1.5, at least about 2, at least about 3, at least about 4, at least about 5, at least about 6, at least about 7, at least about 8, at least about 9, or at least about 10, relative to the bioavailability of the endogenous target compound in the absence of ligand. For example, when the ligand increases bioavailability in the systemic circulation, a concentration time curve for the endogenous ligand starting at the time of administration of the ligand can be prepared, and compared to normal systemic levels of the endogenous target ligand to determine the extent of increase in systemic bioavailability.

The ligands can increase the activity (*e.g*., binding activity) of the endogenous targets to which they bind. For example, a ligand may bind an endogenous target compound such as an endogenous agonist (e.g., a cytokine) or a soluble cytokine receptor and improve the binding activity of the endogenous target compound by increasing the affinity and/or avidity of the endogenous target compound for its endogenous binding partner. Ligands that comprises two or more moieties that have binding sites with binding specificity for a desired endogenous target compound can bind two or more individual endogenous target compound molecules thereby producing an endogenous target compound dimer, trimer, oligomer or multimer. Such a dimer, trimer, oligomer or multimer will have improved binding activity toward, for example, a cell that expresses the natural binding partner for the endogenous target compound due to higher avidity. Generally, such a ligand can improve the binding activity of the endogenous target compound. For example, the binding strength (e.g., affinity or avidity) of an endogenous target compound dimer, trimer, oligomer or multimer for its binding partner can be at least about 10, at least about 100, at least about 1000 or at least about 10,000 times stronger than the binding strength of the endogenous target compound as a monomer (i.e., not in a complex with a ligand of the invention).

In one example, the ligand comprises two or more moieties that have binding sites with binding specificity for a desired endogenous agonist (e.g., a cytokine, a growth factor) and binds two or more endogenous agonist molecules thereby producing an endogenous agonist dimer, trimer, oligomer or multimer. Such a dimer, trimer, oligomer or multimer will have improved binding activity toward, for example, a cell that expresses the receptor for the endogenous agonist due to higher avidity, and can have improved therapeutic efficacy. Similarly, the binding activity of a soluble receptor (e.g., soluble TNFR1) can be increased by a ligand that comprises two or more binding sites for the soluble receptor. Such a ligand can, for example, bind two soluble receptor chains forming a soluble receptor dimer that will bind the endogenous ligand of the receptor with higher avidity that a single receptor chain.

In a specific embodiment, the ligand comprises two or more moieties (dAbs) that have binding specificity for soluble TNFR1 but do not bind the active site (the active site of soluble TNFR1 is contained within Domains 2 and 3). Such a ligand can bind two or more soluble TNFR1 chains to form a soluble TNFR1 dimer, trimer, oligomer or multimer, that has improved binding activity toward its trimeric ligand, TNF, due to increased avidity. It should also be appreciated that the half-life of such a soluble TNFR1 dimer, trimer, oligomer or multimer, which has a larger hydrodynamic size that a single soluble TNFR1 chain, may also be extended relative to a single TNFR1. As a result, such a ligand can improve the TNF-inhibiting activity of soluble TNFR1 and improve its therapeutic efficacy. For example, such a ligand can improve the efficacy of soluble TNFR1 by a factor of at least about 10, at least about 100, at least about 1,000 or at least about 10,000.

In one example of this embodiment, a ligand that is a dimer of a dAb that binds Domain 1 of mouse TNFR1 is used to demonstrate that ligands that induce dimerization of soluble TNFR1 improve efficacy of soluble TNFR1. For example, such a ligand can be added to an assay containing human MRC5 cells, human TNF and soluble mouse TNFR1. Soluble mouse TNFR1 will compete with human TNFR1 on the MRC5 cells for binding to human TNF. The ligand that is a dimer of a dAb that binds Domain 1 of mouse TNFR1 binds two soluble mouse TNFR1 chains to form a mouse TNFR1 dimer that will be much more effective in inhibiting the effects of TNF in the assay. For example, the IC 50 for mouse TNFR1 can be reduced from the nanomolar range to the picomolar range (about 10 or about 100 or about 1000 fold reduction) by the addition of the ligand that induces dimerization of soluble TNFR1.

Administration of ligands that induce dimerization (or trimerization or oligomerization) of soluble receptor chains to patients may cause the clustering and activation of transmembrane forms of certain receptors that are expressed on the cell surface. Generally, any non-desired effect of such activation (*e.g*, activation of immune cells) will be minimal in comparison to the improved binding function and efficacy of the dimerized soluble receptor. Nonetheless, if desired, the interaction of a dimerizing, trimerizing or oligomerizing ligand with the transmembrane form of a receptor can be further reduced by formatting the ligand to increase its hydrodynamic size as described herein. For example, the ligand can be formatted to include a PEG moiety or other half-life extending moiety, such as a moiety that has a binding site for a polypeptide that enhances half-life in vivo (e.g., a dAb that binds serum albumin). As shown herein using dAbs that bind TNFR1, PEGylation of the dAbs dramatically reduced the ability of the dAb to inhibit TNFR1 activity in a cell based assay, but had almost no effect on the ability of the dAbs to bind TNFR1 in a receptor binding assay that approximates the conditions for binding to soluble TNFR1 in vivo. These results indicate that PEGylating dAbs and ligands that comprise two or more dAbs improves selectivity of the dAbs for soluble receptor over the transmembrane form of the receptor.

Some endogenous target compounds naturally bind together to form active dimers, trimers or multimers. Thus, the moiety that has a binding site for an endogenous target compound can be the endogenous target compound itself of a fragment or portion of the endogenous target compound. In some embodiments, such as when the moiety that has a binding site for an endogenous target compound is the endogenous target compound, the ligand can have the activity of the endogenous target compound. Accordingly, administering such a ligand can, as described herein, increase the half-life, increase the bioavailability, increase the amount and/or increase the activity of the endogenous target compound, and will also provide additional endogenous target compound activity due to the presence of a biologically active form of the endogenous target compound in the ligand.

In other embodiments, the ligand can comprise a moiety that has a binding site for an endogenous target compound that is a portion of the endogenous target compound. For example, the portion of the endogenous target compound can be a portion that binds to the endogenous target compound *in vivo,* but does not have the activity (e.g., ligand binding activity, receptor binding activity, catalytic activity) of the endogenous target compound.

Examples of endogenous target compounds that naturally form dimers, trimers or multimers include, for example, growth factors (e.g., PDGF which forms homodimers and heterodimers of A and B chains), cytokines (e.g., cytokines of the TNF superfamily (e.g., RANK-L) which form trimers), and receptors (e.g., receptor of the TNF receptor superfamily which form timers). Suitable portions of such endogenous target compounds can be easily prepared based on knowledge of the structure of the dimer, trimer or multimer forming domains of the endogenous target compounds, or by preparing peptide or polypeptide fragments of the endogenous target compound and assaying the peptides or polypeptides for the ability to bind the endogenous target compound (e.g., using surface plasmon resonance or any other suitable method).

Illustrative of portions of endogenous target compounds that bind the corresponding endogenous target compound in vivo are the so called pre-ligand assembly domains (PLAD) of members of the TNF receptor super family. (See, e.g., WO 01/58953; U.S. Patent Application Publication No. 2003/0108992 A1; Deng et al., Nature Medicine, doi: 10.1038/nm1304 (2005)). PLAD domains from a particular receptor bind to each other *in vivo.* For example, the PLAD domain of TNFR1 will bind another PLAD domain of TNFR1 *in vivo.*

The TNF receptor superfamily is an art recognized group of proteins that includes TNFR1 (p55, CD120a, p60, TNF receptor superfamily member 1A, TNFRSF1A), TNFR2 (p75, p80, CD120b, TNF receptor superfamily member 1B, TNFRSF1B), CD (TNFRSF3, LTβR, TNFR2-RP, TNFR-RP, TNFCR, TNF-R-III), OX40 (TNFRSF4, ACT35, TXGP1L), CD40 (TNFRSF5, p50, Bp50), Fas (CD95, TNFRSF6, APO-1, APTI), DcR3 (TNFRSF6B), CD27 (TNFRSF7, Tp55, S152), CD30 (TNFRSF8, Ki-1, D1S166E), CD137 (TNFRSF9, 4-1BB, ILA), TRAILR-1 (TNFRSF10A, DR4, Apo2), TRAIL-R2 (TNFRSF10B, DR5, KILLER, TRICK2A, TRICKB), TRAILR3 (TNFRSF10C, DcR1, LIT, TRID), TRAILR4 (TNFRSF10D, DcR2, TRUNDD), RANK (TNFRSF11A), OPG (TNFRSF11B, OCIF, TR1), DR3 (TNFRSF12, TRAMP, WSL-1, LARD, WSL-LR, DDR3, TR3, APO-3), DR3L (TNFRSF12L), TAC1 (TNFRSF13B), BAFFR (TNFRSF13C), HVEM (TNFRSF14, ATAR, TR2, LIGHTR, HVEA), NGFR (TNFRSF16), BCMA (TNFRSF17, BCM), AITR (TNFRSF18, GITR), TNFRSF19, FLJ14993 (TNFRSF19L, RELT), DR6 (TNFRSF21), SOBa (TNFRSF22, Tnfrh2, 2810028K06Rik), mSOB (THFRSF23, Tnfrh1).

Several PLAD domains are known in the art and other PLAD domains and variants of PLAD domains can be readily isolated and prepared using any suitable methods, such as the methods described in WO 01/58953; U.S. Patent Application Publication No. 2003/0108992 A1; Deng et al., Nature Medicine, doi: 10.1038/nm1304 (2005). Many suitable methods for preparing polypeptides, protein fragments, and peptide variants, as well as suitable binding assays, such as the TNFR1 receptor binding assay described herein are well-known and conventional in the art. Exemplary PLAD domains are presented in Table 1.

**TABLE 1.**

| Receptor | PLAD Domain |
|---|---|
| TNFR1 | Cys Pro Gln Gly Lys Tyr Ile His Pro Gln Asn Asn Ser Ile Cys Cys Thr Lys Cys His Lys Gly Thr Tyr Leu Tyr Asn Asp Cys Pro Gly Pro Gly Gln Asp Thr Asp Cys (SEQ ID NO:28) |
| TNFR2 | Cys Arg Leu Arg Glu Tyr Tyr Asp Gln Thr Ala Gln Met Cys Cys Ser Lys Cys Ser Pro Gly Gln His Ala Lys Val Phe Cys Thr Lys Thr Ser Asp Thr Val Cys (SEQ ID NO:29) |
| FAS | Arg Leu Ser Ser Lys Ser Val Asn Ala Gln Val Thr Asp Ile Asn Ser Lys Gly Leu Glu Leu Arg Lys Thr Val Thr Thr Val Glu Thr Gln Asn Leu Glu Gly Leu His His Asp Gly Gln Phe Cys (SEQ ID NO:30) |
| FAS | Arg Leu Ser Ser Lys Ser Val Asn Ala Gln Val Thr Asp Ile Asn Ser Lys Gly Leu Glu Leu Arg Lys Thr Val Thr Thr Val Glu Thr Gln Asn Leu Glu Gly Leu His His Asp Gly Gln Phe Cys His Lys Pro Cys Pro Pro Gly Glu Arg Lys Ala Arg Asp Cys Thr Val Asn Gly Asp (SEQ ID NO:31) |
| LTβR | Cys Arg Asp Gln Glu Lys Glu Tyr Tyr Glu Pro Gln His Arg Ile Cys Cys Ser Arg Cys Pro Pro Gly Thr Tyr Val Ser Ala Lys Cys Ser Arg Ile Arg Asp Thr Val Cys (SEQ ID NO:32) |
| CD40 | Cys Arg Glu Lys Gln Tyr Leu Ile Asn Ser Gln Cys Cys Ser Leu Cys Gln Pro Gly Gln Lys Leu Val Ser Asp Cys Thr Glu Phe Thr Glu Thr Glu Cys (SEQ ID NO:33) |
| CD30 | Cys His Gly Asn Pro Ser His Tyr Tyr Asp Lys Ala Val Arg Arg Cys Cys Tyr Arg Cys Pro Met Gly Leu Phe Pro Thr Gln Gln Cys Pro Gln Arg Pro Thr Asp Cys Arg Lys Gln Cys (SEQ ID NO:34) |
| CD27 | Trp Trp Leu Cys Val Leu Gly Thr Leu Val Gly Leu Ser Ala Thr Pro Ala Pro Lys Ser Cys Pro Glu Arg His Tyr Trp Ala Gln Gly Lys Leu Cys Cys Gln Met (SEQ ID NO:35) |
| HVEM | Cys Lys Glu Asp Glu Tyr Pro Val Gly Ser Glu Cys Cys Pro Lys Cys Ser Pro Gly Tyr Arg Val Lys Glu Ala Cys Gly Glu Leu Thr Gly Thr Val Cys (SEQ ID NO:36) |
| OX40 | Val Gly Ala Arg Arg Leu Gly Arg Gly Pro Cys Ala Ala Leu Leu Leu Leu Gly Leu Gly Leu Ser Thr Val Thr Gly Leu His Cys Val Gly Asp Thr Tyr |
| | (SEQ ID NO:37) |
| DR4 | Ala Thr Ile Lys Leu His Asp Gln Ser Ile Gly Thr Gln Gln Trp Glu His Ser Pro Leu Gly Glu Leu Cys Pro Pro Gly Ser His Arg (SEQ ID NO:38) |

In some embodiments, the ligand comprises an moiety that has a binding site with binding specificity for an endogenous target compound which is a PLAD, such as a PLAD of TNFR1, TNFR2, FAS, LT βR, CD40, CD30, CD27, HVEM, OX40, DR4 or other TNF receptor superfamily member, or a variant of a PLAD domain. The variant of a PLAD domain can, for example, be a PLAD domain of TNFR1, TNFR2, FAS, LT βR, CD40, CD30, CD27, HVEM, OX40, or DR4, wherein one or more amino acids has been deleted, inserted or substituted, but that retains the ability to bind to the corresponding PLAD of TNFR1, TNFR2, FAS, LT βR, CD40, CD30, CD27, HVEM, OX40, or DR4. The amino acid sequence of a variant PLAD domain can comprise a region of at least about 10 contiguous amino acids, at least about 15 contiguous amino acids, at least about 20 contiguous amino acids, at least about 25 contiguous amino acids, at least about 30 contiguous amino acids, at least about 35 contiguous amino acids, or at least about 40 contiguous amino acids that are the same as the amino acids in the amino acid sequence of the corresponding PLAD (e.g., PLAD of TNFR1, TNFR2, FAS, LT βR, CD40, CD30, CD27, HVEM, OX40, DR4). In addition, or alternatively, the amino acid sequence of a variant PLAD domain can be at least about 80%, at least about 85%, least about 90%, at least about 91%, at least about 92%, at least about 93%, at least about 94%, at least about 95%, at least about 96%, at least about 97%, at least about 98%, or at least about 99% identical to the amino acid sequence of the corresponding PLAD (e.g., PLAD of TNFR1, TNFR2, FAS, LT βR, CD40, CD30, CD27, HVEM, OX40, or DR4).

Ligands that comprise a binding moiety that has a binding site for an endogenous target compound that is a PLAD or PLAD variant, can be formatted as described herein. For example the ligand can be a dimer, trimer or multimer of a PLAD or PLAD variant, or a fusion protein comprising a PLAD or PLAD variant and a half-life extending moiety (e.g., a moiety that has a binding site for serum albumin or neonatal Fc receptor).

Ligands that comprise two or more PLAD or PLAD variants (e.g., PLAD dimers, trimers, multimers) can bind two or more soluble receptor chains to form soluble receptor dimers, trimers or multimers which, as described herein, can have improved binding function in comparison to soluble receptor monomers. In some circumstances, such a ligand may bind soluble receptor and also bind the transmembrane form of the receptor expressed on the cell surface. If desired, selectivity for binding soluble receptor can be improved by formatting the ligand to have a larger hydrodynamic size, for example using a PEG moiety, or other half-life extending moiety as described herein.

In particular embodiments, the ligand comprises a PLAD (e.g., PLAD of TNFR1, TNFR2, FAS, LT βR, CD40, CD30, CD27, HVEM, OX40, or DR4) or PLAD variant and a half-life extending moiety, such as a PEG moiety or a dAb that binds serum albumin or neonatal Fc receptor.

Preferably, a ligand that induce dimerization (or trimerization or oligomerization) of soluble receptor chains does not substantially agonize cell surface or transmembrane forms of the receptor in a standard cell assay (i. e., when present at a concentration of 1 nM, 10 nM, 100 nM, 1 µM, 10 µM, 100 µM, 1000 µM or 5,000 µM, results in no more than about 5% of the receptor-mediated activity induced by natural ligand of the receptor in the assay).

It is preferred that the moiety that has a binding site with binding specificity for an endogenous target compound binds the endogenous target compound with high affinity, such as a K_{d} of 300 nM to 5 pM (i.e., 3 x 10⁻⁷ to 5 x 10⁻¹²M), preferably 50 nM to 20 pM, more preferably 5 nM to 200 pM and most preferably 1 nM to 100 pM, for example 1 x 10⁻⁷ M or less, preferably 1 x 10⁻⁸ M or less, more preferably 1 x 10⁻⁹ M or less, advantageously 1 x 10^{- 10} M or less and most preferably 1 x 10⁻¹¹ M or less; and/or a K_{off} rate constant of 5 x 10⁻¹ s⁻¹ to 1 x 10⁻⁷ s⁻¹ preferably 1 x 10⁻² s⁻¹ to 1 x 10⁻⁶ s⁻¹, more preferably 5 x 10⁻³ s⁻¹ to 1 x 10⁻⁵ s⁻¹, for example 5 x 10⁻¹ s⁻¹ or less, preferably 1 x 10⁻² s⁻¹ or less, advantageously 1 x 10⁻³ s⁻¹ or less, more preferably 1 x 10⁻⁴ s⁻¹ or less, still more preferably 1 x 10⁻⁵ s⁻¹ or less, and most preferably 1 x 10⁻⁶ s⁻¹ or less as determined by surface plasmon resonance. A moiety that has a binding site with binding specificity for an endogenous target compound and binds the endogenous target compound with high affinity can be formatted into any suitable ligand format as described herein.

In addition to the features of the ligands described herein, it is preferred that the ligand bind the endogenous target compound with high affinity, such as a K_{d} of 300 nM to 5 pM (i.e., 3 x 10⁻⁷ to 5 x 10⁻¹²M), preferably 50 nM to 20 pM, more preferably 5 nM to 200 pM and most preferably 1 nM to 100 pM, for example 1 x 10⁻⁷ M or less, preferably 1 x 10⁻⁸ M or less, more preferably 1 x 10⁻⁹ M or less, advantageously 1 x 10⁻¹⁰ M or less and most preferably 1 x 10⁻¹¹ M or less; and/or a K_{off} rate constant of 5 x 10⁻¹ s⁻¹ to 1 x 10⁻⁷ s⁻¹, preferably 1 x 10⁻² s⁻¹ to 1 x 10⁻⁶ s⁻¹, more preferably 5 x 10⁻³ s⁻¹ to 1 x 10⁻⁵ s⁻¹, for example 5 x 10⁻¹ s⁻¹ or less, preferably 1 x 10⁻² s⁻¹ or less, advantageously 1 x 10-³ s⁻¹ or less, more preferably 1 x 10⁻⁴ s⁻¹ or less, still more preferably 1 x 10⁻⁵ s⁻¹ or less, and most preferably 1 x 10⁻⁶ s⁻¹ or less as determined by surface plasmon resonance.

In certain embodiments, the ligand comprises a dAb that binds an endogenous target compound. The endogenous target compound can be, for example, a soluble receptor (e.g., soluble cytokine receptor, soluble growth factor receptor, soluble hormone receptor), an endogenous receptor antagonist (e.g., IL-1ra), an enzyme, an endogenous agonist (e.g., a cytokine, a growth factor, a hormone). In some embodiments, the ligand is a dAb dimer, trimer, oligomer or multimer.

The ligands can comprise a moiety that has a binding site for an endogenous target compound, wherein said moiety that has a binding site for an endogenous target compound is selected from the group consisting of an affibody, an SpA domain, an LDL receptor class A domain, an EGF domain, and an avimer. The ligand can also comprise a moiety that has a binding site for an endogenous target compound wherein the moiety is an antibody or antibody fragment, such as a Fv fragment, a single chain Fv fragment, a disulfide bonded Fv fragment, a Fab fragment, a Fab' fragment, a F(ab')₂ fragment, a diabody, an immunoglobulin single variable domain (e.g., human V_{H}, human V_{L},V_{HH}).

If desired, the ligand can further comprise a half-life extending moiety as described herein. For example, the ligand can comprise a half-life extending moiety selected from the group consisting of a polyalkylene glycol moiety, serum albumin or a fragment thereof, transferrin receptor or a transferrin-binding portion thereof, or a moiety comprising a binding site for a polypeptide that enhances half-life *in vivo.* Suitable a moieties comprising a binding site for a polypeptide that enhances half-life *in vivo* include an affibody, an SpA domain, an LDL receptor class A domain, an EGF domain, an avimer, an antibody or antibody fragment, such as a Fv fragment, a single chain Fv fragment, a disulfide bonded Fv fragment, a Fab fragment, a Fab' fragment, a F(ab')₂ fragment, a diabody, and an immunoglobulin single variable domain (e.g., human V_{H}, human V_{L}, V_{HH}).

As described herein, the ligands of the invention do not substantially inhibit the activity of the endogenous target compound to which they bind. However, where a target has more than one activity (e.g., binding activity and signaling activity), the ligand may inhibit the activity that is not associated with the intended therapeutic activity or benefit. For example, a ligand or dAb monomer may bind a soluble receptor and also bind the transmembrane form of the receptor. In such a situation, the transmembrane form of the receptor may have ligand-binding activity and also signaling activity. Preferably, the ligand of the invention will not inhibit the ligand binding activity (of the soluble and transmembrane forms of the receptor) which acts to inhibit ligand-induced processes, but may inhibit the signaling activity of the transmembrane form of the receptor.

To further illustrate the invention, exemplary embodiments of ligands that bind soluble TNFR1 are described below. Embodiments of ligands that bind soluble TNFR1 are illustrative of ligands of the invention that bind other receptors.

TNFR1 is a transmembrane receptor containing an extracellular region that binds ligand and an intracellular domain that lacks intrinsic signal transduction activity but can associate with signal transduction molecules. The complex of TNFR1 with bound TNF contains three TNFR1 chains and three TNF chains. (Banner et al., Cell, 73(3) 431-445 (1993).) The TNF ligand is present as a trimer, which is bound by three TNFR1 chains. (Id.) The three TNFR1 chains are clustered closely together in the receptor-ligand complex, and this clustering is a prerequisite to TNFR1-mediated signal transduction. In fact, multivalent agents that bind TNFR1, such as anti-TNFR1 antibodies, can induce TNFR1 clustering and signal transduction in the absence of TNF and are commonly used as TNFR1 agonists. (See, *e.g.,* Belka et al., EMBO, 14(6):1156-1165 (1995); Mandik-Nayak et al., J. Immunol, 167:1920-1928 (2001).) Accordingly, multivalent agents that bind TNFR1, are generally not effective antagonists of TNFR1 even if they block the binding of TNFα to TNFR1.

The extracellular region of TNFR1 comprises a thirteen amino acid amino-terminal segment (amino acids 1-13 of SEQ ID NO:2 (human); amino acids 1-13 of SEQ ID NO:4 (mouse)), Domain 1 (amino acids 14-53 of SEQ ID NO: 2 (human); amino acids 14-53 of SEQ ID NO: 4 (mouse)), Domain 2 (amino acids 54-97 of SEQ ID NO: 2 (human); amino acids 54-97 of SEQ ID NO: 4 (mouse)), Domain 3 (amino acids 98-138 of SEQ ID NO: 2 (human); amino acid 98-138 of SEQ ID NO: 4 (mouse)), and Domain 4 (amino acids 139-167 of SEQ ID NO: 2 (human); amino acids 139-167 of SEQ ID NO: 4 (mouse)) which is followed by a membrane-proximal region (amino acids 168-182 of SEQ ID NO: 2 (human); amino acids 168-183 SEQ ID NO: 4 (mouse)). (See, Banner et al., Cell 73(3) 431-445 (1993) and Loetscher et al., Cell 61 (2) 351-359 (1990).) Domains 2 and 3 make contact with bound ligand (TNFβ, TNFα). (Banner et al., Cell, 73(3) 431-445 (1993).) The extracellular region of TNFR1 also contains a region referred to as the pre-ligand binding assembly domain or PLAD domain (amino acids 1-53 of SEQ ID NO: 2 (human); amino acids 1-53 of SEQ ID NO: 4 (mouse)) (The Government of the USA, WO 01/58953; Deng et al., Nature Medicine, doi: 10.1038/nm1304 (2005)).

TNFR1 is shed from the surface of cells *in vivo* through a process that includes proteolysis of TNFR1 in Domain 4 or in the membrane-proximal region (amino acids 168-182 of SEQ ID NO: 2; amino acids 168-183 of SEQ ID NO: 4), to produce a soluble form of TNFR1. Soluble TNFR1 retains the capacity to bind TNFα, and thereby functions as an endogenous inhibitor of the activity of TNFα.

In some embodiments, a ligand or dAb monomer that binds soluble TNFR1 can also bind transmembrane TNFR1 and inhibit TNFα-induced clustering of TNFR1 on the cell surface, which precedes signal transduction through the receptor, but not inhibit binding of TNFa to transmembrane TNFR1 and soluble TNFR1.

In particular embodiments, the ligand or dAb monomer does not inhibit the TNF-binding activity of soluble and transmembrane forms of TNFR1 but can inhibit the signaling activity of TNFR1. A ligand or dAb monomer of this type can bind Domain 1 or Domain 4 of TNFR1. Such a ligand or dAb monomer can bind soluble TNFR1 and transmembrane TNFR1, but does not inhibit the TNF-binding activity of either form of TNFR1. Accordingly, such a ligand or dAb can be administered, for example, to extend the half-life of soluble TNFR1 or to enhance the binding activity of soluble TNFR1. Accordingly, administering such ligand or dAb monomer to a mammal in need thereof can harness and exploit the endogenous regulatory pathways that inhibit the activity of cell surface TNFR1 for therapeutic purposes.

In other embodiments, the invention provides similar ligands and dAb monomers that bind other soluble receptors but do not inhibit binding of endogenous ligands to soluble receptors.

In more particular embodiments, the ligand comprises a dAb that binds Domain 1 of TNFR1 and competes with TAR2m-21-23 for binding to mouse TNFR1 or competes with TAR2h-205 for binding to human TNFR1. In more particular embodiments, the ligand is a dimer of such a dAb and, if desired, further comprises a PEG moiety.

In certain embodiments, the ligand or dAb monomer is substantially resistant to aggregation. For example, in some embodiments, less than about 10%, less than about 9%, less than about 8%, less than about 7%, less than about 6%, less than about 5%, less than about 4%, less than about 3%, less than about 2% or less than about 1% of the ligand or dAb monomer aggregates when a 1-5 mg/ml, 5-10 mg/ml, 10-20 mg/ml, 20-50 mg/ml, 50-100 mg/ml, 100-200 mg/ml or 200 -500 mg/ml solution of ligand or dAb in a solvent that is routinely used for drug formulation such as saline, buffered saline, citrate buffer saline, water, an emulsion, and, any of these solvents with an acceptable excipient such as those approved by the FDA, is maintained at about 22°C, 22-25°C, 25-30°C, 30-37°C, 37-40°C, 40-50°C, 50-60°C, 60-70°C, 70-80°C, 15-20°C, 10-15°C, 5-10°C, 2-5°C, 0-2°C, -10°C to 0°C, - 20°C to -10°C, -40°C to -20°C, -60°C to -40°C, or -80°C to -60°C, for a period of about time, for example, 10 minutes, 1 hour , 8 hours, 24 hours, 2 days, 3 days, 4 days, 1 week, 2 weeks, 3 weeks, 1 month, 2 months, 3 months, 4 months, 6 months, 1 year, or 2 years.

Aggregation can be assessed using any suitable method, such as, by microscopy, assessing turbidity of a solution by visual inspection or spectroscopy or any other suitable method. Preferably, aggregation is assessed by dynamic light scattering. Ligands or dAb monomers that are resistant to aggregation provide several advantages. For example, such ligands or dAb monomers can readily be produced in high yield as soluble proteins by expression using a suitable biological production system, such as *E*. *coli,* and can be formulated and/or stored at higher concentrations than conventional polypeptides, and with less aggregation and loss of activity.

In addition, ligands or dAb monomers that are resistant to aggregation can be produced more economically than other antigen- or epitope-binding polypeptides (e.g., conventional antibodies). For example, generally, preparation of antigen- or epitope-binding polypeptides intended for *in vivo* applications includes processes (e.g., gel filtration) that remove aggregated polypeptides. Failure to remove such aggregates can result in a preparation that is not suitable for *in vivo* applications because, for example, aggregates of an antigen-binding polypeptide that is intended to act as an antagonist can function as an agonist by inducing cross-linking or clustering of the target antigen. Protein aggregates can also reduce the efficacy of therapeutic polypeptide by inducing an immune response in the subject to which they are administered.

In contrast, the aggregation resistant ligands or dAb monomers of the invention can be prepared for *in vivo* applications without the need to include process steps that remove aggregates, and can be used in *in vivo* applications without the aforementioned disadvantages caused by polypeptide aggregates.

In some embodiments, the ligand or dAb monomer unfolds reversibly when heated to a temperature (Ts) and cooled to a temperature (Tc), wherein Ts is greater than the melting temperature (Tm) of the dAb, and Tc is lower than the melting temperature of the dAb. For example, the dAb monomer can unfold reversibly when heated to 80°C and cooled to about room temperature. A polypeptide that unfolds reversibly loses function when unfolded but regains function upon refolding. Such polypeptides are distinguished from polypeptides that aggregate when unfolded or that improperly refold (misfolded polypeptides), *i.e*., do not regain function.

Polypeptide unfolding and refolding can be assessed, for example, by directly or indirectly detecting polypeptide structure using any suitable method. For example, polypeptide structure can be detected by circular dichroism (CD) (*e.g*., far-UV CD, near-UV CD), fluorescence (*e.g*., fluorescence of tryptophan side chains), susceptibility to proteolysis, nuclear magnetic resonance (NMR), or by detecting or measuring a polypeptide function that is dependent upon proper folding (e.g., binding to target ligand, binding to generic ligand). In one example, polypeptide unfolding is assessed using a functional assay in which loss of binding function (*e.g*., binding a generic and/or target ligand, binding a substrate) indicates that the polypeptide is unfolded.

The extent of unfolding and refolding of a ligand or dAb monomer can be determined using an unfolding or denaturation curve. An unfolding curve can be produced by plotting temperature as the ordinate and the relative concentration of folded polypeptide as the abscissa. The relative concentration of folded ligand or dAb monomer can be determined directly or indirectly using any suitable method (*e.g*., CD, fluorescence, binding assay). For example, a ligand or dAb monomer solution can be prepared and ellipticity of the solution determined by CD. The ellipticity value obtained represents a relative concentration of folded ligand or dAb monomer of 100%. The ligand or dAb monomer in the solution is then unfolded by incrementally raising the temperature of the solution and ellipticity is determined at suitable increments (*e.g*., after each increase of one degree in temperature). The ligand or dAb monomer in solution is then refolded by incrementally reducing the temperature of the solution and ellipticity is determined at suitable increments. The data can be plotted to produce an unfolding curve and a refolding curve. The unfolding and refolding curves have a characteristic sigmoidal shape that includes a portion in which the ligand or dAb monomer molecules are folded, an unfolding/refolding transition in which ligand or dAb monomer molecules are unfolded to various degrees, and a portion in which the ligand or dAb monomer molecules are unfolded. The y-axis intercept of the refolding curve is the relative amount of refolded ligand or dAb monomer recovered. A recovery of at least about 50%, or at least about 60%, or at least about 70%, or at least about 75%, or at least about 80%, or at least about 85%, or at least about 90%, or at least about 95% is indicative that the ligand or dAb monomer unfolds reversibly.

In a preferred embodiment, reversibility of unfolding of the ligand or dAb monomer is determined by preparing a ligand or dAb monomer solution and plotting heat unfolding and refolding curves. The ligand or dAb monomer solution can be prepared in any suitable solvent, such as an aqueous buffer that has a pH suitable to allow the ligand or dAb monomer to dissolve (*e.g*., pH that is about 3 units above or below the isoelectric point (pI)). The ligand or dAb monomer solution is concentrated enough to allow unfolding/folding to be detected. For example, the ligand or dAb monomer solution can be about 0.1 1µM to about 100 µM, or preferably about 1 µM to about 10 µM.

If the melting temperature (Tm) of the ligand or dAb monomer is known, the solution can be heated to about ten degrees below the Tm (Tm-10) and folding assessed by ellipticity or fluorescence (*e.g*., far-UV CD scan from 200 nm to 250 nm, fixed wavelength CD at 235 nm or 225 nm; tryptophan fluorescent emission spectra at 300 to 450 nm with excitation at 298 nm) to provide 100% relative folded ligand or dAb monomer. The solution is then heated to at least ten degrees above Tm (Tm+10) in predetermined increments (*e.g*., increases of about 0.1 to about 1 degree), and ellipticity or fluorescence is determined at each increment. Then, the ligand or dAb monomer is refolded by cooling to at least Tm-10 in predetermined increments and ellipticity or fluorescence determined at each increment. If the melting temperature of the ligand or dAb monomer is not known, the solution can be unfolded by incrementally heating from about 25°C to about 100°C and then refolded by incrementally cooling to at least about 25°C, and ellipticity or fluorescence at each heating and cooling increment is determined. The data obtained can be plotted to produce an unfolding curve and a refolding curve, in which the y-axis intercept of the refolding curve is the relative amount of refolded protein recovered.

The ligand or dAb monomer can comprise any suitable immunoglobulin variable domain, and preferably comprises a human variable domain or a variable domain that comprises human framework regions. In certain embodiments, the dAb monomer comprises a universal framework, as described herein.

The universal framework can be a V_{L} framework (Vλ or Vκ), such as a framework that comprises the framework amino acid sequences encoded by the human germline DPK1, DPK2, DPK3, DPK4, DPK5, DPK6, DPK7, DPK8, DPK9, DPK10, DPK12, DPK13, DPK15, DPK16, DPK18, DPK19, DPK20, DPK21, DPK22, DPK23, DPK24, DPK25, DPK26 or DPK 28 immunoglobulin gene segment. If desired, the V_{L} framework can further comprises the framework amino acid sequence encoded by the human germline J_{κ}1, J_{κ}2, J_{κ}3, J_{κ}4, or J_{κ}5 immunoglobulin gene segment.

In other embodiments the universal framework can be a V_{H} framework, such as a framework that comprises the framework amino acid sequences encoded by the human germline DP4, DP7, DP8, DP9, DP10, DP31, DP33, DP38, DP45, DP46, DP47, DP49, DP50, DP51, DP53, DP54, DP65, DP66, DP67, DP68 or DP69 immunoglobulin gene segment. If desired, the V_{H} framework can further comprises the framework amino acid sequence encoded by the human germline J_{H}1, J_{H}2, J_{H}3, J_{H}4, J_{H}4b, J_{H}5 and J_{H}6 immunoglobulin gene segment.

In particular embodiments, the dAb monomer ligand comprises the DPK9 V_{L} framework, or a V_{H} framework selected from the group consisting of DP47, DP45 and DP38.

The dAb monomer can comprises a binding site for a generic ligand, such as protein A, protein L and protein G.

In certain embodiments, the dAb monomer comprises one or more framework regions comprising an amino acid sequence that is the same as the amino acid sequence of a corresponding framework region encoded by a human germline antibody gene segment, or the amino acid sequences of one or more of said framework regions collectively comprise up to 5 amino acid differences relative to the amino acid sequence of said corresponding framework region encoded by a human germline antibody gene segment.

In other embodiments, the amino acid sequences of FW1, FW2, FW3 and FW4 of the dAb monomer are the same as the amino acid sequences of corresponding framework regions encoded by a human germline antibody gene segment, or the amino acid sequences of FW1, FW2, FW3 and FW4 collectively contain up to 10 amino acid differences relative to the amino acid sequences of corresponding framework regions encoded by said human germline antibody gene segment.

In other embodiments, the dAb monomer comprises FW1, FW2 and FW3 regions, and the amino acid sequence of said FW1, FW2 and FW3 regions are the same as the amino acid sequences of corresponding framework regions encoded by human germline antibody gene segments.

In some embodiments, the dAb monomer does not comprise a *Camelid* immunoglobulin variable domain, or one or more framework amino acids that are unique to immunoglobulin variable domains encoded by *Camelid* germline antibody gene segments.

In certain embodiments, the ligands (e.g., dAb monomers) of the invention are efficacious in models of diseases when an effective amount is administered. Generally an effective amount is about 1 mg/kg to about 10 mg/kg (e.g., about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, or about 10 mg/kg). Many suitable models of diseases exist. The models of chronic inflammatory disease described herein are recognized by those skilled in the art as being predictive of therapeutic efficacy in humans. The prior art does not suggest using ligands that comprise a moiety that binds an endogenous target compound, such as a soluble cytokine receptor (e.g., soluble TNFR1) in these models, or that they would be efficacious.

In particular embodiments, the ligand or dAb monomer is efficacious in the standard mouse collagen-induced arthritis model. For example, administering an effective amount of the ligand can reduce the average arthritic score of the summation of the four limbs in the standard mouse collagen-induced arthritis model, for example, by about 1 to about 16, about 3 to about 16, about 6 to about 16, about 9 to about 16, or about 12 to about 16, as compared to a suitable control. In another example, administering an effective amount of the ligand can delay the onset of symptoms of arthritis in the standard mouse collagen-induced arthritis model, for example, by about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average arthritic score of the summation of the four limbs in the standard mouse collagen-induced arthritis model of 0 to about 3, about 3 to about 5, about 5 to about 7, about 7 to about 15, about 9 to about 15, about 10 to about 15, about 12 to about 15, or about 14 to about 15.

In other embodiments, the ligand or dAb monomer is efficacious in the mouse ΔARE model of arthritis. (Kontoyiannis et al., J Exp Med 196:1563-74 (2002).) For example, administering an effective amount of the ligand can reduce the average arthritic score in the mouse ΔARE model of arthritis, for example, by about 0.1 to about 2.5, about 0.5 to about 2.5, about 1 to about 2.5, about 1.5 to about 2.5, or about 2 to about 2.5, as compared to a suitable control. In another example, administering an effective amount of the ligand can delay the onset of symptoms of arthritis in the mouse ΔARE model of arthritis by, for example, about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average arthritic score in the mouse ΔARE model of arthritis of 0 to about 0.5, about 0.5 to about 1, about 1 to about 1.5, about 1.5 to about 2, or about 2 to about 2.5.

In other embodiments, the ligand or dAb monomer is efficacious in the mouse ΔARE model of inflammatory bowel disease (IBD). (Kontoyiannis et al., J Exp Med 196:1563-74 (2002).) For example, administering an effective amount of the ligand can reduce the average acute and/or chronic inflammation score in the mouse ΔARE model of IBD, for example, by about 0.1 to about 2.5, about 0.5 to about 2.5, about 1 to about 2.5, about 1.5 to about 2.5, or about 2 to about 2.5, as compared to a suitable control. In another example, administering an effective amount of the ligand can delay the onset of symptoms of IBD in the mouse ΔARE model of IBD by, for example, about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average acute and/or chronic inflammation score in the mouse ΔARE model of IBD of 0 to about 0.5, about 0.5 to about 1, about 1 to about 1.5, about 1.5 to about 2, or about 2 to about 2.5.

In other embodiments, the ligand or dAb monomer is efficacious in the mouse dextran sulfate sodium (DSS) induced model of IBD. See, e.g., Okayasu I. et al., Gastroenterology 98:694-702 (1990); Podolsky K., J Gasteroenterol. 38 suppl XV:63-66 (2003).) For example, administering an effective amount of the ligand can reduce the average severity score in the mouse DSS model of IBD, for example, by about 0.1 to about 2.5, about 0.5 to about 2.5, about 1 to about 2.5, about 1.5 to about 2.5, or about 2 to about 2.5, as compared to a suitable control. In another example, administering an effective amount of the ligand can delay the onset of symptoms of IBD in the mouse DSS model of IBD by, for example, about 1 day, about 2 days, about 3 days, about 4 days, about 5 days, about 6 days, about 7 days, about 10 days, about 14 days, about 21 days or about 28 days, as compared to a suitable control. In another example, administering an effective amount of the ligand can result in an average severity score in the mouse DSS model of IBD of 0 to about 0.5, about 0.5 to about 1, about 1 to about 1.5, about 1.5 to about 2, or about 2 to about 2.5.

In particular embodiments, the ligand or dAb monomer is efficacious in the mouse tobacco smoke model of chronic obstructive pulmonary disease (COPD). (See, Wright and Churg, Chest, 122:301-306 (2002), Groneberg, DA et al., Respiratory Research 5:18 (2004), Coffman RL et al., J. Exp. Med. 201(12):1875-1879 (2001), Van Scott, MR et al., J. App. Physiol. 96:1433-1444 (2004)).

In further embodiments, the ligand of dAb monomer are efficacious in an animal model of asthma (see, Coffman RL et al., J. Exp. Med. 201(12):1875-1879 (2001); Van Scott, MR et al., J. App. Physiol. 96:1433-1444 (2004)), pulmonary fibrosis (e.g., Venkatesan, N et al., Lung 287:1342-1347 (2004), systemic lupus erythematosus (SLE) (Knight et al. (1978) J. Exp. Med., 147: 1653; Reinersten et al. (1978) New Eng. J. Med., 299: 515), myasthenia gravis (Lindstrom et al. (1988) Adv. Immunol., 42: 233), arthritis (Stuart et al. (1984) Ann. Rev. Immunol., 42: 233; Van Eden et al. (1988) Nature, 331: 171), thyroiditis (Maron et al. (1980) J. Exp. Med., 152: 1115), insulin dependent diabetes mellitus (IDDM) (Kanasawa et al. (1984) Diabetologia, 27: 113), and the EAE model of multiple sclerosis (see Paterson (1986) Textbook of Immunopathology, Mischer et al., eds., Grune and Stratton, New York, pp. 179-213; McFarlin et al. (1973) Science, 179: 478: and Satoh et al. (1987) J. Immunol., 138: 179).

Preferably, the ligand or dAb monomer is secreted in a quantity of at least about 0.5 mg/L when expressed in *E. coli* or in *Pichia* species (e.g., *P. pastoris).* In other preferred embodiments, the dAb monomer is secreted in a quantity of at least about 0.75 mg/L, at least about 1 mg/L, at least about 4 mg/L, at least about 5 mg/L, at least about 10 mg/L, at least about 15 mg/L, at least about 20 mg/L, at least about 25 mg/L, at least about 30 mg/L, at least about 35 mg/L, at least about 40 mg/L, at least about 45 mg/L, or at least about 50 mg/L, or at least about 100 mg/L, or at least about 200 mg/L, or at least about 300 mg/L, or at least about 400 mg/L, or at least about 500 mg/L, or at least about 600 mg/L, or at least about 700 mg/L, or at least about 800 mg/L, at least about 900 mg/L, or at least about 1g/L when expressed in *E. coli* or in *Pichia* species (e.g., *P. pastoris).* In other preferred embodiments, the dAb monomer is secreted in a quantity of at least about 1 mg/L to at least about 1g/L, at least about 1 mg/L to at least about 750 mg/L, at least about 100 mg/L to at least about 1 g/L, at least about 200 mg/L to at least about 1 g/L, at least about 300 mg/L to at least about 1 g/L, at least about 400 mg/L to at least about 1 g/L, at least about 500 mg/L to at least about 1g/L, at least about 600 mg/L to at least about 1 g/L, at least about 700 mg/L to at least about 1 g/L, at least about 800 mg/L to at least about 1g/L, or at least about 900 mg/L to at least about 1g/L when expressed in *E. coli* or in *Pichia* species (e.g., *P. pastoris).* Although, the ligands and dAb monomers described herein can be secretable when expressed in *E. coli* or in *Pichia* species (e.g., *P. pastoris),* they can be produced using any suitable method, such as synthetic chemical methods or biological production methods that do not employ *E. coli* or *Pichia* species.

In some embodiments, the ligand is an antibody that has binding specificity for an endogenous target compound or an antigen-binding fragment thereof, such as an Fab fragment, Fab' fragment, F(ab')₂ fragment or Fv fragment (e.g., scFV). In other embodiments, the antagonist or ligand is monovalent, such as a dAb or a monovalent antigen-binding fragment of an antibody, such as an Fab fragment, Fab' fragment, or Fv fragment.

In other embodiments of the invention described throughout this disclosure, instead of the use of a "dAb" in a ligand of the invention, it is contemplated that the skilled addressee can use a domain that comprises the CDRs of a dAb that binds an endogenous target compound (e.g., CDRs grafted onto a suitable protein scaffold or skeleton, eg an affibody, an SpA scaffold, an LDL receptor class A domain or an EGF domain) or can be a protein domain comprising a binding site for TNFR1, e.g., wherein the domain is selected from an affibody, an SpA domain, an LDL receptor class A domain or an EGF domain. The disclosure as a whole is to be construed accordingly to provide disclosure of antagonists, ligands and methods using such domains in place of a dAb. In addition, the ligands of the invention can be formatted (e.g., extended half-life formats) as described herein.

### Uses of Ligands That Bind An Endogenous Target Compound and Therapeutic Methods

The invention relates to ligands that comprise a moiety (e.g., dAb) that has a binding site with binding specificity for an endogenous target compound but do not substantially inhibit the activity of said endogenous target compound. Preferably, the ligand does not bind to the active site of an endogenous target compound. Further features of the ligands of the invention are described herein. For conciseness and to avoid repetition, not all disclosed features of the ligands are specifically described with respect to the uses and methods of the invention. It is intended that the uses and methods of the invention include the use, and methods that comprise administering, any of the ligands described herein.

The invention relates to compositions comprising a ligand that comprise a moiety (e.g., dAb) that has a binding site with binding specificity for an endogenous target compound, but does not substantially inhibit the activity of said endogenous target compound, and a pharmaceutically acceptable carrier, diluent or excipient. The invention also relates to therapeutic and diagnostic methods that employ the ligands or compositions of the invention. The ligands described herein may be used for *in vivo* therapeutic and prophylactic applications, *in vivo* diagnostic applications and the like. For example, the ligands (e.g., dAb monomers), of the present invention will typically find use in preventing, suppressing or treating diseases (e.g., acute and/or chronic inflammatory diseases). For example, the antagonists and/or ligands can be administered to treat, suppress or prevent a inflammatory diseases (e.g., acute and/or chronic inflammation), cancers and neoplasms (e.g., lymphomas (e.g., B cell lymphomas, T cell lymphomas, acute myeloid lymphoma), multiple myeloma, lung cancer, carcinomas), metabolic diseases (e.g., diabetes, obesity), allergic hypersensitivity, bacterial or viral infection, autoimmune disorders (which include, but are not limited to, Type I diabetes, asthma, multiple sclerosis, arthritis (e.g., rheumatoid arthritis, juvenile rheumatoid arthritis, psoriatic arthritis, spondylarthropathy (e.g., ankylosing spondylitis)), systemic lupus erythematosus, inflammatory bowel disease (e.g., Crohn's disease, ulcerative colitis), myasthenia gravis and Behcet's syndrome), psoriasis, endometriosis, abdominal adhesions (e.g., post abdominal surgery), osteoarthritis, chronic obstructive pulmonary disease, or other disease that is amenable to therapy with an endogenous target compound.

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

The invention relates to the use of a ligand, as described herein, for the manufacture of a medicament that can be administered to a patient to harness and exploit the beneficial activities of endogenous compounds to produce beneficial effects (e.g., therapeutically beneficial effects, diagnostically beneficial effects). Accordingly, the invention relates to use of a ligand for the manufacture of a medicament for increasing the amount of said endogenous target compound in a subject (e.g., the amount of the endogenous target compound in the systemic circulation, the amount at a particular site, such as a particular organ, tissue or region (e.g., in a joint, in the lung, in a muscle, in site of the skin, in the CNS)), for increasing the bioavailability said endogenous target compound in a subject (e.g., systemically or at a desired site of action (e.g., in a joint, in the lung, in a muscle, in site of the skin, in the CNS)), for increasing the *in vivo* half-life of said endogenous target compound, or for treating a disease that is amenable to treatment with an endogenous target compound (e.g., such as a disease as described herein), for example. The medicament can be for systemic or local administration.

The invention relates to the use of a ligand comprising a moiety that has a binding site for an endogenous target compound for the manufacture of a medicament for increasing the half-life of said endogenous target compound in a subject.

The medicament can be administered to a patient to increase the half-life of an endogenous target compound to which the ligand binds by, for example, by binding the endogenous target compound and thereby increasing the hydrodynamic size of the endogenous target compound, by binding and stabilizing the endogenous target compound, or by binding and slowing the rate of clearance or metabolism of the endogenous target compound. Generally, the ligand will increase the half-life of the endogenous target compound to which it binds by a factor of at least about 1.5, at least about 2, at least about 3, at least about 4, at least about 5, at least about 6, at least about 7, at least about 8, at least about 9, or at least about 10, relative to the half-life of the endogenous target compound in the absence of ligand. For example, an endogenous target compound can have a half-life of about 1 hour, and after administration of a ligand that comprises a moiety with a binding site that binds said endogenous target compound, the half-life of endogenous target compound can be increased to about 1.5 hours, to about 10 hours, or longer.

The invention relates to use of a ligand comprising a moiety that has a binding site for an endogenous target compound for the manufacture of a medicament for increasing the amount of said endogenous target compound in a subject.

The level or amount of endogenous target compound can increase following administration of a ligand, as described herein, for example, due to the ligands half-life increasing effect. For example, the level or amount of endogenous target ligand (e.g., in serum) 1 hour, or 2 hours, or 3 hours, or 4 hours, or 5 hours, or 6 hours, or 7 hours, or 8 hours, or 9 hours, or 10 hours, or 11 hours, or 12 hours, or 1 day after administration of a ligand of the invention can be increased by a factor of at least about 1.5, at least about 2, at least about 3, at least about 4, at least about 5, at least about 10, at least about 50, at least about 100, at least about 500, at least about 1,000, at least about 5,000, at least about 10,000, at least about 50,000, or at least about 100,000. Increase levels or amounts of an endogenous target compound can be readily detected using any suitable method. For example, a suitable sample (e.g., serum) can be obtain prior to administration of the ligand and at one or more time points after administration, and the level or amount of endogenous target compound in the samples can be determined using any suitable method. The sample can be, for example, a sample of blood, serum, cerebrospinal fluid, synovial fluid, bronchoalveolar lavage, enema or a biopsy. In some embodiments, the amount or level of ligand in such a sample is increased, relative to the amount or level before the ligand is administered.

The invention relates to use of a ligand comprising a moiety that has a binding site for an endogenous target compound for the manufacture of a medicament for increasing the bioavailability said endogenous target compound in a subject.

The medicament can be administered to a subject to increase bioavailability of an endogenous target compound systemically, or at a desired site of action (e.g., at a site where the ligand is locally administered). For example, a ligand may bind an endogenous target compound that has beneficial activity in the systemic circulation but is rapidly distributed into the tissues or cleared (e.g., a steroid or peptide hormone), and slow the rate of distribution or clearance. Such a ligand can increase bioavailability of the endogenous target compound in the systemic circulation where the beneficial activity of the endogenous target compound can have a beneficial (e.g., therapeutic) effect. A ligand can increases the bioavailability of an endogenous target compound at a desired site by recruiting the endogenous target compound to that site. For example, a ligand comprising a binding site for a desired endogenous target compound can be locally administered to a patient (e.g., as a depot formulation). The locally administered ligand can bind the desired endogenous target compound, and recruit additional endogenous target compound to the site. Thus, an increased level or amount of endogenous target compound can be present at the site to produce beneficial effects. For example, in one embodiment, the ligand comprises a moiety with a binding site for TGF beta isoform 3. Such a ligand can be locally administered to bind TGF beta isoform 3 and recruit TGF beta isoform 3 to the site of local administration, and thus promote would healing.

Generally, the ligand will increase bioavailability (e.g., in the systemic circulation, at a desired site of action) of the endogenous target compound to which it binds by a factor of at least about 1.5, at least about 2, at least about 3, at least about 4, at least about 5, at least about 6, at least about 7, at least about 8, at least about 9, or at least about 10, relative to the bioavailability of the endogenous target compound in the absence of ligand. For example, when the ligand increases bioavailability in the systemic circulation, a concentration time curve for the endogenous ligand starting at the time of administration of the ligand can be prepared, and the curve and area under the curve (AUC) can be compared to normal systemic levels of the endogenous target ligand to determine the extent of increase in systemic bioavailability.

The invention relates to use of a ligand comprising a moiety that has a binding site for an endogenous target compound for the manufacture of a medicament for increasing the activity of said endogenous target.

The ligands can increase the activity (*e.g*., binding activity) of the endogenous targets to which they bind. For example, a ligand may bind an endogenous target compound such as an endogenous agonist (e.g., a cytokine) or a soluble cytokine receptor and improve the binding activity of the endogenous target compound by increasing the affinity and/or avidity of the endogenous target compound for its endogenous binding partner. Ligands that comprises two or more moieties that have binding sites with binding specificity for a desired endogenous target compound can bind two or more individual endogenous target compound molecules thereby producing an endogenous target compound dimer, trimer, oligomer or multimer. Such a dimer, trimer, oligomer or multimer will have improved binding activity toward, for example, a cell that expresses the natural binding partner for the endogenous target compound due to higher avidity. Generally, such a ligand can improve the binding activity of the endogenous target compound. For example, the binding strength (e.g., affinity or avidity) of an endogenous target compound dimer for its binding partner can be at least about 10, at least about 100, at least about 1,000 or at least about 10,000 times stronger than the binding strength of the endogenous target compound as a monomer (i.e., not in a complex with a ligand of the invention).

In a specific embodiment, the ligand comprises two or more moieties (dAbs) that have binding specificity for soluble TNFR1 but do not bind the active site (the active sire of soluble TNFR1 is contained within Domains 2 and 3). Such a ligand can bind two or more soluble TNFR1 chains to form a soluble TNFR1 dimer, trimer, oligomer or multimer, that has improved binding activity toward its trimeric ligand, TNF, due to increased avidity. It should also be appreciated that the half-life of such a soluble TNFR1 dimer, trimer, oligomer or multimer, which has a larger hydrodynamic size that a single soluble TNFR1 chain, may also be extended relative to a single TNFR1. As a result, such a ligand can improve the TNF-inhibiting activity of soluble TNFR1 and improve its therapeutic efficacy. For example, such a ligand can improve the efficacy of soluble TNFR1 by a factor of at least about 10, at least about 100, at least about 1,000 or at least about 10,000.

In one example of this embodiment, a ligand that is a dimer of a dAb that binds Domain 1 of mouse TNFR1 is used to demonstrate that ligands that induce dimerization of soluble TNFR1 improve efficacy of soluble TNFR1. For example, such a ligand can be added to an assay containing human MRC5 cells, human TNF and soluble mouse TNFR1. Soluble mouse TNFR1 will compete with human TNFR1 on the MRC5 cells for binding to human TNF. The ligand that is a dimer of a dAb that binds Domain 1 of mouse TNFR1 binds two soluble mouse TNFR1 chains to form a mouse TNFR1 dimer that will be much more effective in inhibiting the effects of TNF in the assay. For example, the IC 50 for mouse TNFR1 can be reduced from the nanomolar range to the picomolar range (about 10 or about 100 or about 1000 fold reduction) by the addition of the ligand that induces dimerization of soluble TNFR1.

Preferably, ligand that induce dimerization (or trimerization or oligomerization) of soluble receptor chains does not substantially agonize cell surface or transmembrane forms of the receptor in a standard cell assay (*i.e*., when present at a concentration of 1 nM, 10 nM, 100 nM, 1 µM, 10 µM, 100 µM, 1000 µM or 5,000 µM, results in no more than about 5% of the receptor-mediated activity induced by natural ligand of the receptor in the assay).

In a particular embodiment, the invention relates to the use of a ligand comprising two or more moieties that have a binding site for an endogenous target compound for the manufacture of a medicament for increasing the binding activity of said endogenous target compound, wherein said ligand binds said endogenous target, does not bind the active site of said endogenous target, and does not substantially inhibit the binding activity of said endogenous target compound.

The invention also relates to a ligand that binds an endogenous target compound having activity suitable for treating a disease in a subject, wherein said ligand does not bind the active site of said endogenous target compound or substantially inhibit the activity of said endogenous target compound, for use in therapy of a disease amenable to treatment with said endogenous target compound. For example, the ligand can increase the in vivo half-life of said endogenous target compound, increase the amount of said endogenous target compound in a subject, increase the bioavailability of said endogenous target compound, and/or increase the binding activity of said endogenous compound.

The invention relates to use of a ligand comprising a binding moiety that has a binding site for an endogenous target compound for increasing the half-life, bioavailability or activity of said endogenous compound, wherein said binding moiety that has a binding site for an endogenous compound is said endogenous compound or a portion or variant thereof, and wherein said ligand binds said endogenous target compound and does not substantially inhibit the activity of said endogenous target compound.

The invention relates to use of a ligand comprising a binding moiety that has a binding site for a member of the TNF receptor superfamily for increasing the half-life, bioavailability or activity of said member of the TNF receptor superfamily, wherein said ligand binds said member of the TNF receptor superfamily and does not substantially inhibit the activity of said member of the TNF receptor superfamily, and wherein said binding moiety that has a binding site for a member of the TNF receptor superfamily is a pre-ligand assembly domain (PLAD) or a variant thereof.

The invention relates to a method for increasing the half-life of an endogenous target compound in a subject, comprising administering to a subject in need thereof an effective amount of a ligand comprising a moiety that has a binding site with binding specificity for said endogenous target compound.

The invention relates to a method for increasing the amount of an endogenous target compound in a subject, comprising administering to a subject in need thereof an effective amount of a ligand comprising a moiety that has a binding site with binding specificity for said endogenous target compound.

The invention relates to a method for increasing the bioavailability of an endogenous target compound in a subject, comprising administering to a subject in need thereof an effective amount of a ligand comprising a moiety that has a binding site with binding specificity for said endogenous target compound.

The invention relates to a method for increasing the activity (e.g., binding activity) of an endogenous target compound in a subject, comprising administering to a subject in need thereof an effective amount of a ligand comprising a moiety that has a binding site with binding specificity for said endogenous target compound.

The invention relates to a method for treating a subject having a disease that is amenable to treatment with an endogenous target compound in a subject, comprising administering to a subject in need thereof an effective amount of a ligand comprising a moiety that has a binding site with binding specificity for said endogenous target compound.

The invention relates to a method for increasing the half-life, bioavailability or activity of an endogenous target compound, comprising administering to a subject in need thereof an effective amount of a ligand comprising a moiety that has a binding site with binding specificity for said endogenous target compound, wherein said binding moiety that has a binding site for an endogenous compound is said endogenous compound or a portion or variant thereof, and wherein said ligand binds said endogenous target compound and does not substantially inhibit the activity of said endogenous target compound.

The invention relates to a method for increasing the half-life, bioavailability or activity of for a member of the TNF receptor superfamily, comprising administering to a subject in need thereof an effective amount of a ligand comprising a binding moiety that has a binding site for a member of the TNF receptor superfamily, wherein said ligand binds said member of the TNF receptor superfamily and does not substantially inhibit the activity of said member of the TNF receptor superfamily, and wherein said binding moiety that has a binding site for a member of the TNF receptor superfamily is a pre-ligand assembly domain (PLAD) or a variant thereof.

Therapeutic and prophylactic uses of ligands of the invention involve the administration of ligands according to the invention to a recipient mammal, such as a human. Dual-specific and Multi-specific ligands (*e.g*., dual-specific antibody formats) bind to multimeric antigen with great avidity. Dual- or Multispecific ligands can allow the cross-linking of two antigens, for example to produce high avidity dimers, trimers or multimers of soluble receptors.

Substantially pure ligands of at least 90 to 95% homogeneity are preferred for administration to a mammal, and 98 to 99% or more homogeneity is most preferred for pharmaceutical uses, especially when the mammal is a human. Once purified, partially or to homogeneity as desired, the ligands may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY).

Generally, the ligands will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition). A variety of suitable formulations can be used, including extended release formulations.

The route of administration of pharmaceutical compositions according to the invention may be any of those commonly known to those of ordinary skill in the art. For therapy, including without limitation immunotherapy, the selected ligands thereof of the invention can be administered to any patient in accordance with standard techniques. The administration can be by any appropriate mode, including parenterally, intravenously, intramuscularly, intraperitoneally, transdermally, *via* the pulmonary route, or also, appropriately, by direct infusion with a catheter. Parenteral administration can also be by intraarterial, intrathecal, intraarticular, subcutaneous, or other suitable injection or infusion. Additional suitable modes of administration include pulmonary administration, intranasal administration, intravaginal, intrarectal administration (e.g., by suppository or enema). The dosage and frequency of administration will depend on the age, sex and condition of the patient, concurrent administration of other drugs, counterindications and other parameters to be taken into account by the clinician. Administration can be local (e.g., local delivery to the lung by pulmonary administration, e.g., intranasal administration) or systemic as indicated.

The ligands of the present invention may be used as separately administered compositions or in conjunction with other agents. These can include various immunotherapeutic drugs, such as cyclosporine, methotrexate, adriamycin or cisplatinum, and immunotoxins. Pharmaceutical compositions can include "cocktails" of various cytotoxic or other agents in conjunction with the antagonists (e.g., ligands) of the present invention, or even combinations of ligands according to the present invention having different specificities, such as ligands selected using different target antigens or epitopes, whether or not they are pooled prior to administration. Generally, the ligand and any additional agent are administered in a manner that provides an overlap of therapeutic effect.

In certain embodiments, the ligand comprising a moiety that has a binding site for an endogenous target compound is administered together with a low dose of the endogenous target compound (e.g., a recombinant form of the endogenous target compound). As described herein, the ligand can, for example, increase the half-life or bioavailability of the endogenous target compound, or increase the activity of the endogenous target compound, rendering the low does therapeutically effective. This therapeutic approach is advantageous because side effects associated with higher doses of agents for treating disease (e.g., recombinant forms of endogenous target proteins) can be avoided.

In other embodiments, a ligand that has been preloaded with an endogenous target compound (e.g., incubated with and allowed to bind a recombinant form of an endogenous target compound) is administered. Preloaded ligands are well suited for local administration to provide high bioavailability of an endogenous target ligand that is normally present at subtherapeutic levels or that is difficult to recruit to a site where the activity of the endogenous target ligand is desired.

Ligands according to the invention which able to bind to extracellular targets involved in endocytosis (e.g. Clathrin) can be endocytosed, enabling access to intracellular targets. In addition, dual or multispecific ligands, provide a means by which a binding domain (e.g., a dAb monomer) that is specificity able to bind to an intracellular target can be delivered to an intracellular environment. This strategy requires, for example, a dual-specific ligand with physical properties that enable it to remain functional inside the cell. Alternatively, if the final destination intracellular compartment is oxidising, a well folding ligand may not need to be disulphide free.

Advantageously, dual- or multi-specific ligands may be used to target cytokines and other molecules which cooperate synergistically in therapeutic situations in the body of an organism. The invention therefore provides a method for synergising the activity of two or more binding domains (e.g., dAbs) that bind cytokines or other molecules, comprising administering a dual- or multi-specific ligand capable of binding to said two or more molecules (e.g., cytokines). In this aspect of the invention, the dual- or multi-specific ligand may be any dual- or multi-specific ligand, including a ligand composed of complementary and/or non-complementary domains, a ligand in an open conformation, and a ligand in a closed conformation. For example, this aspect of the invention relates to combinations of V_{H} domains and V_{L} domains, V_{H} domains only and V_{L} domains only.

Synergy in a therapeutic context may be achieved in a number of ways. For example, target combinations may be therapeutically active only if both targets are targeted by the ligand, whereas targeting one target alone is not therapeutically effective. In another embodiment, one target alone may provide some low or minimal therapeutic effect, but together with a second target the combination provides a synergistic increase in therapeutic effect.

Animal model systems which can be used to screen the effectiveness of the ligands in protecting against or treating the disease are available. Suitable animal model can be used, such as the models disclosed herein.

The ligands of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present ligands or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of ligand *per* kilogram of body weight, with doses of 0.05 to 2.0 mg/kg/dose being more commonly used. For prophylactic applications, compositions containing the present ligands or cocktails thereof may also be administered in similar or slightly lower dosages, to prevent, inhibit or delay onset of disease (e.g., to sustain remission or quiescence, or to prevent acute phase). The skilled clinician will be able to determine the appropriate dosing interval to treat, suppress or prevent disease. When an antagonist of TNFR1 (e.g., ligand) is administered to treat, suppress or prevent a chronic inflammatory disease, it can be administered up to four times per day, twice weekly, once weekly, once every two weeks, once a month, or once every two months, at a dose off, for example, about 10 µg/kg to about 80 mg/kg, about 100 µg/kg to about 80 mg/kg, about 1 mg/kg to about 80 mg/kg, about 1 mg/kg to about 70 mg/kg, about 1 mg/kg to about 60 mg/kg, about 1 mg/kg to about 50 mg/kg, about 1 mg/kg to about 40 mg/kg, about 1 mg/kg to about 30 mg/kg, about 1 mg/kg to about 20 mg/kg , about 1 mg/kg to about 10 mg/kg, about 10 µg/kg to about 10 mg/kg, about 10 µg/kg to about 5 mg/kg, about 10 µg/kg to about 2.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg or about 10 mg/kg. In particular embodiments, the antagonist of TNFR1 (e.g., ligand) is administered to treat, suppress or prevent a chronic inflammatory disease once every two weeks or once a month at a dose of about 10 µg/kg to about 10 mg/kg (e.g., about 10 µg/kg, about 100 µg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg or about 10 mg/kg.). The ligands can also be administered (e.g., systemically or locally), for example, at a dose of about 1 mg/day to about 10 mg/day (e.g., 10 mg/day, 9 mg/day, 8 mg/day, 7 mg/day, 6 mg/day, 5 mg/day, 4 mg/day, 3 mg/day, 2 mg/day, or 1 mg/day). Accordingly, the agent can be locally administered to pulmonary tissue at a dose of about 1 µg/kg/day to about 200 µg/kg/day (e.g., about 10 µg/kg/day, about 20 µg/kg/day, about 30 µg/kg/day, about 40 µg/kg/day, about 50 µg/kg/day, about 60 µg/kg/day, about 70 µg/kg/day, about 80 µg/kg/day, about 90 µg/kg/day, about 100 µg/kg/day, about 110 µg/kg/day, about 120 µg/kg/day, about 130 µg/kg/day, about 140 µg/kg/day, about 150 µg/kg/day, about 160 µg/kg/day, about 170 µg/kg/day, about 180 µg/kg/day, or about 190 µg/kg/day).

Treatment or therapy performed using the compositions described herein is considered "effective" if one or more symptoms are reduced (e.g., by at least 10% or at least one point on a clinical assessment scale), relative to such symptoms present before treatment, or relative to such symptoms in an individual (human or model animal) not treated with such composition or other suitable control. Symptoms will obviously vary depending upon the disease or disorder targeted, but can be measured by an ordinarily skilled clinician or technician. Such symptoms can be measured, for example, by monitoring the level of one or more biochemical indicators of the disease or disorder (e.g., levels of an enzyme or metabolite correlated with the disease, affected cell numbers, etc.), by monitoring physical manifestations (e.g., inflammation, tumor size, etc.), or by an accepted clinical assessment scale, for example, the Expanded Disability Status Scale (for multiple sclerosis), the Irvine Inflammatory Bowel Disease Questionnaire (32 point assessment evaluates quality of life with respect to bowel function, systemic symptoms, social function and emotional status - score ranges from 32 to 224, with higher scores indicating a better quality of life), the Quality of Life Rheumatoid Arthritis Scale, the St. George's Respiratory Questionnaire, or other accepted clinical assessment scale as known in the art. A sustained (e.g., one day or more, preferably longer) reduction in disease or disorder symptoms by at least 10% or by one or more points on a given clinical scale is indicative of "effective" treatment. Similarly, prophylaxis performed using a composition as described herein is "effective" if the onset or severity of one or more symptoms is delayed, reduced or abolished relative to such symptoms in a similar individual (human or animal model) not treated with the composition.

A composition containing a ligand or cocktail thereof according to the present invention may be utilised in prophylactic and therapeutic settings to aid in the alteration, inactivation, killing or removal of a select target cell population in a mammal. In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the ligands, e.g. antibodies, cell-surface receptors or binding proteins thereof whereby the undesired cells are killed or otherwise removed from the blood for return to the mammal in accordance with standard techniques.

A composition containing an ligand according to the present invention may be utilised in prophylactic and therapeutic settings to aid in the alteration, inactivation, killing or removal of a select target cell population in a mammal.

In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the ligands, e.g. antibodies, cell- surface receptors or binding proteins thereof whereby the undesired cells are killed or otherwise removed from the blood for return to the mammal in accordance with standard techniques.

### Endogenous Target Compounds

Suitable endogenous target compounds include, for example, soluble cytokine receptors (e.g., soluble TNF1, soluble TNFR2, soluble IL-1 receptor, soluble IL-4 receptor, soluble IL-13 receptor), endogenous receptor antagonists (IL-1ra, IL-6ra), enzymes (e.g., beta- glucocerebrosidase (E.C. 3.2.1.45)), blood factors (e.g., Factor II, Factor VIII), erythropoietin, human growth hormone, TPO, interferon-alpha, interferon-beta, interferon-gamma, GLP-1, OXM, sex hormones (e.g., testosterone, estrogen), bone morphogenic proteins (e.g., BMP-1, BMP-2, BMP-3, BMP-4, BMP-5, BMP-6, BMP-7), transforming growth factor beta (TGF-beta) isoform 1, TGF-beta isoform 2, TGF-beta isoform 3, IL-10, IL-2, granulocyte macrophage colony stimulating factor, granulocyte colony stimulating factor, insulin, glucagon, GIP.

Suitable endogenous target compounds include cytokines, such as interleukins, interferons, colony stimulating factors and chemokines. Examples of such cytokines include, interleukins, IL1, IL2, IL3, IL4, IL5, IL6, IL7, IL8, IL9, IL10, IL11, IL12, IL13, IL14, IL15 (IL-T), IL16, IL17, IL17B, IL17C, IL17E, IL17F, IL18, IL19, IL20, IL21, IL22, IL23, IL24, IL25, IL26, IL27, IL28A, IL28B, IL29, IL30, IL-31 and IL-32, and interferons IFN-alpha, IFN-beta, IFN-delta, IFN-gamma, IFN-kappa, IFN-lambda-1, IFN-lambda-2, IFN-lambda-3, IFN-omega, and IFN-tau.

Suitable endogenous target compounds include chemokines such as, CCF18, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, ECIP-1, EDNAP, ENA-78, ENAP, ENAP-alpha, ENAP-beta, Endothelial cell growth inhibitor, Endothelial IL8, FIC, FDNCF, FINAP, GDCF-2, GCF, GCP-2, GRO-alpha, GRO-beta, GRO-gamma, Heparin neutralizing protein, Humig, 1-309, ILC, ILINCK, IMAC, I-TAC, IL8, IP-9, IP-10, Lymphotactin, LAG-1, LARC, LCC-1, LD78-alpha, LD78-beta, LD78-gamma, LDCF, LEC, Lkn-1, LMC, LAI, LCF, LA-PF4, LDGF, LDNAP, LIF, LIX, MARC, MCAF, MCIF, Mexikine, MCP-1, MCP-2, MCP-3, MCP-4, MCP-5, MDC, MEC, MIP-1-alpha, MIP-1-beta, MIP-1-delta, MIP-1-gamma, MIP-3, MIP-3-alpha, MIP-3-beta, MIP-4, MIP-4-alpha, MIP-5, Monotactin-1, MPIF-1, MPIF-2, MRP-1, MRP-2, MDGF, MDNAP, MDNCF, Megakaryocyte-stimulatory-factor, MGSA, MGSA-alpha, MGSA-beta, Mig, MIP-2, MIP-2-alpha, MIP-2-beta, MIP-2-gamma, NAF, NAP-1, NAP-2, NAP-3, NAP-4, NCP, Oncostatin A, PARC, PBP, PBP-like, PBSF, PF4, PLF, PPBP, RANTES, Regakine-1, SCM-1-alpha, SCYC1, SCYC2, SCI, SCYA1, SCYA2, SCYA3, SCYA4, SCYA5, SCYA6, SCYA7, SCYA8, SCYA9, SCYA10, SCYA11, SCYA12, SCYA13, SCYA14, SCYA15, SCYA16, SCYA17, SCYA19, SCYA20, SCYA21, SCYA22, SCYA23, SCYA24, SCYA25, SCYA26, SCYA27, SCYA28, SLC, SMC-CF, Stop-1, SCYB1, SCYB2, SCYB3, SCYB4, SCYB5, SCYB6, SCYB7, SCYB8, SCYB9, SCYB9B, SCYB10, SCYB11, SCYB12, SCYB13, SCYB14, SCYB15, SCYB16, SDF-1-alpha, SDF-1-beta, SR-PSOX, SCUD1, SR-PSOX, TARC, TCA-3, TCA-4, TDCF, TECK, TSC-1, TSG-8, TCF, TCK-1, TLSF-alpha, TLSF-beta, TPAR-1, TSG-1, WECHE.

Suitable endogenous target compounds include colony stimulating factors (CSFs). CSFs are cytokines that stimulate the proliferation of specific pluripotent stem cells of the bone marrow in adults. Granulocyte-CSF (G-CSF) is specific for proliferative effects on cells of the granulocyte lineage. Macrophage-CSF (M-CSF) is specific for cells of the macrophage lineage. Granulocyte-macrophage-CSF (GM-CSF) has proliferative effects on both classes of lymphoid cells. Epo is also considered a CSF as well as a growth factor, since it stimulates the proliferation of erythrocyte colony-forming units. IL-3 (secreted primarily from T-cells) is also known as multi-CSF, since it stimulates stem cells to produce all forms of hematopoietic cells.

Suitable endogenous target compounds include Erythropoietin (Epo). Epo is synthesized by the kidney and is the primary regulator of erythropoiesis. Epo stimulates the proliferation and differentiation of immature erythrocytes; it also stimulates the growth of erythroid progenitor cells (e.g. erythrocyte burst-forming and colony-forming units) and induces the differentiation of erythrocyte colony-forming units into proerythroblasts. When patients suffering from anemia due to kidney failure are given Epo, the result is a rapid and significant increase in red blood cell count.

Suitable endogenous target compounds include Insulin-Like Growth Factor-I (IGF-I). IGF-I (originally called somatomedin C) is a growth factor structurally related to insulin. IGF-I is the primary protein involved in responses of cells to growth hormone (GH): that is, IGF-I is produced in response to GH and then induces subsequent cellular activities, particularly on bone growth. It is the activity of IGF-I in response to GH that gave rise to the term somatomedin. Subsequent studies have demonstrated, however, that IGF-I has autocrine and paracrine activities in addition to the initially observed endocrine activities on bone. The IGF-I receptor, like the insulin receptor, has intrinsic tyrosine kinase activity. Owing to their structural similarities IGF-I can bind to the insulin receptor but does so at a much lower affinity than does insulin itself.

Suitable endogenous target compounds include Insulin-Like Growth Factor-I (IGF-II). IGF-II is almost exclusively expressed in embryonic and neonatal tissues. Following birth, the level of detectable IGF-II protein falls significantly. For this reason IGF-II is thought to be a fetal growth factor. The IGF-II receptor is identical to the mannose-6-phosphate receptor that is responsible for the integration of lysosomal enzymes (which contain mannose-6-phosphate residues) to the lysosomes.

Suitable endogenous target compounds include tumor necrosis factor beta. TNF-beta (also called lymphotoxin) is characterized by its ability to kill a number of different cell types, as well as the ability to induce terminal differentiation in others. One significant non-proliferative response to TNF-beta is an inhibition of lipoprotein lipase present on the surface of vascular endothelial cells. The predominant site of TNF-beta synthesis is T-lymphocytes, in particular the special class of T-cells called cytotoxic T-lymphocytes (CTL cells). The induction of TNF-beta expression results from elevations in IL-2 as well as the interaction of antigen with T-cell receptors.

Suitable endogenous target compounds include tumor necrosis factor alpha (TNF-alpha). TNF-alpha (also called lymphotoxin B, or cachectin) is a pleiotropic inflammatory cytokine and it can cause necrosis of some types of tumors. TNF-alpha shares only 36% amino acid sequence homology with TNF-beta. Yet, the tertiary structures of the two proteins are remarkably similar and both bind to TNF receptors TNFR-1 and TNFR-2. TNF-alpha is a trimeric protein encoded within the major histocompatibility complex. It was first identified in its 17 kd secreted form, but further research then showed that a noncleaved 27kd precursor form also existed in transmembrane form. Stimulated macrophages produce 27kd TNF-alpha, which can either bind directly to TNFR-1 and TNFR-2 receptors through cell-to-cell contact or undergo cleavage and bind in its soluble form. The cytokine is produced by several types of cells, but especially by macrophages. Low levels of TNF-alpha promote the remodeling or replacement of injured and senescent tissue by stimulating fibroblast growth. Additional beneficial functions of TNF-alpha include its role in the immune response to bacterial, and certain fungal, viral, and parasitic invasions as well as its role in the necrosis of specific tumors. Lastly it acts as a key mediary in the local inflammatory immune response. TNF-alpha is an acute phase protein which initiates a cascade of cytokines and increases vascular permeability, thereby recruiting macrophage and neutrophils to a site of infection. TNF-alpha secreted by the macrophage causes blood clotting which serves to contain the infection. TNF-alpha also exhibits chronic effects (eg in inflammation) and prolonged overproduction of TNF-alpha also results in a condition known as cachexia, which is characterized by anorexia, net catabolism, weight loss and anemia and which occurs in illnesses such as cancer and AIDS.

Suitable endogenous target compounds include cytokines and growth factors in hematopoiesis, such as, CD34, CIL (colony-inhibiting lymphokine), Daniplestim, GADS (GRB2 related adapter downstream of shc), Hematopoietic cell growth factor, Hematopoietic cell kinase, Hematopoietic cell phosphatase, Hematopoietic consensus tyrosine-lacking kinase, Hematopoietic colony stimulating factor-309, Hematopoietin-1, Hematopoietin-2, Hemoregulatory peptide, HIM, Hiwi, HnudC, progenipoietin, progenipoietin-1, progenipoietin-2, progenipoietin-4, Promegapoietin, Promegapoietin-1, Promegapoietin-la, and Synthokine.

Other hematopoietic growth factors in a wide sense include the various colony stimulating factors (see: CSF, including G-CSF, GM-CSF, M-CSF), Epo, SCF(stem cell factor), SCPF(stem cell proliferation factor), various Interleukins(IL1, IL3, IL4, IL5, IL6, IL11, IL12), LIF, TGF-beta, MIP-1-alpha, TNF-alpha, also many other low molecular weight factors, and several other cytokines. Many of these proteins are multifunctional. They act on very early differentiation stages or at later stages; they can also act in a lineage-specific manner or may influence more than one lineage. Proliferation and maturation of committed progenitors is controlled by late-acting lineage-specific factors such as Epo, M-CSF, G-CSF, and IL5. Multipotential progenitors beginning active cell proliferation are regulated by several overlapping cytokines, including IL3, GM-CSF, and IL4. Triggering of cycling by dormant primitive progenitors and maintenance of B-cell potential of the primitive progenitors appears to require interactions of early acting cytokines including IL6, G-CSF, IL11, IL12, LIF, and SCF. Depending upon their biological activities hematopoietins have been classified into several subgroups.

The term type-1 hematopoietins is occasionally used to describe those factors involved in the regulation of hematopoiesis that act directly on some cell types. This group includes IL3 (multi-CSF) and GM-CSF. Factors that synergise with colony stimulating factors but that by themselves do not possess intrinsic colony stimulating activity have been referred to occasionally as type-2 hematopoietins. They include IL1, IL4, IL5 and IL6. Type-3 hematopoietins are those modulating the hematopoietic growth by stimulating the release of colony stimulating factors by their respective producer cells. They include IL1, IL2, TNF-beta and IFN-gamma.

Some factors negatively regulate processes of hematopoiesis. They may selectively inhibit the proliferation of some types of hematopoietic cells and may even induce cell death. The transforming growth factor TGF-beta, for example, predominantly acts on primitive hematopoietic cells and lymphoid cells. The factor called MIP-1-alpha (macrophage inflammatory protein-1-alpha) shows similar activity on primitive myelopoietic cells

Suitable endogenous target compounds include endogenous compounds involved in angiogenesis. Examples of endogenous compounds involved in angiogenesis include, 16K Prolactin, ADAPTS-1, ADAMTS-8, Adrenomedullin, Angio-associated migratory cell protein, Angiogenin, angiogenin-related protein, Angiomodulin, Angiomotin, Angiopoietin-1, Angiopoietin-2, Angiopoietin-3, Angiopoietin-4, Angiopoietin-5, Angiopoietin-like 1, angiopoietin-like 2, angiopoietin-like 3, angiopoietin-like 4, Angiopoietin-related protein-2, Angiostatin, Angiotensin-2, Angiotropin, ARP-2, B16-F1 melanoma autocrine motility factor, brain-specific angiogenesis inhibitor-1, brain-specific angiogenesis inhibitor-2, brain-specific angiogenesis inhibitor-3, C49a, CAM assay, Cartilage-derived anti-tumor factor, Cartilage-derived inhibitor, CATF, cCAF, CD55, CDI, CDT6, chondrocyte-derived inhibitor, Chondrocyte-derived inhibitor of angiogenesis and metalloproteinase activity, Chondromodulin-1, Chondrosarcoma-derived growth factor, chorioallantoic membrane assay CLAF, collagen type 18, Connective tissue growth factor, corpus luteum angiogenic factor, degranulation inhibitory protein, EGF, EG-VEGF, embryonic kidney-derived angiogenesis factor-1, embryonic kidney-derived angiogenesis factor-2, Endocrine gland-derived vascular endothelial growth factor, Endorepellin, endostatin, Endothelial cell stimulating angiogenic factor, endothelial cell-derived growth factor, Endothelial-monocyte activating polypeptide-2, ESAF, f-ECGF, FGF, FGF-4, Fibrin fragment E, Fibroblast derived endothelial cell growth factor, fibroblast inducible secreted protein-12, FrzB2, GFB-111, glioma-derived angiogenesis inhibitory factor, growth hormone, GSM, HAF, Haptoglobin, heparin-binding neurite-promoting factor, hepatocyte growth factor, HGF, HUAF, human angiogenic factor, human inhibitor angiogenesis factor-1, human uterine angiogenesis factor, IFN-alpha, IFN-gamma, IGF, IGF,-1, IGF-BP-7, jagged, KAF, kallistatin, K-FGF, kidney angiogenic factor, kininostatin, migration stimulating factor, mitogen-regulated protein/proliferin, Mitogen-regulated protein-4, Monocyto-Angiotropin, MRP/PLF, MRP-4, Neuroleukin, Neuropilin-1, NKG5, NLK, Notch-1, Notch-4, ORF-74, ovarian growth factor, PAF, parathyroid hormone-like related protein, PD-ECGF, PDGF, PDGF-BB, PEDF, PF4, PGI, pigment epithelium-derived factor placenta growth factor, placental angiogenic factor, platelet factor-4, platelet-derived endothelial cell growth factor, P1GF, PrGF, Prokineticin-1, proliferin-related protein, Prostacyclin stimulating factor, prostatic growth factor, Prothrombin kringle-2 domain PRP, PTHrP, RNASE5, Scatter factor, semaphorin-3, Sprouty, TAF, TAMF, TGF-alpha, TGF-beta, thrombin, Thrombospondin, TIE-1, TIE-2, tissue factor, TNF-alpha, TNF-beta, TNF-like weak inducer of apoptosis, Transferrin, TSP, tumor angiogenesis factor, tumor autocrine motility factor, Tumstatin, TWEAK, Uterine angiogenesis factor, vascular permeability factor, Vasculotropin, VEGF, VEGF-162, VEGF-C, VEGF-D, VEGF-E, VEGI, VPF.

Many different growth factors and cytokines have been shown to exert chemotactic, mitogenic, modulatory or inhibitory activities on endothelial cells, smooth muscle cell and fibroblasts and can, therefore, be expected to participate in angiogenic processes in one way or another. The process involves the concerted action of proteolytic enzymes, extracellular matrix components, cell adhesion molecules, and vasoactive factors. Factors modulating growth, chemotactic behavior and/or functional activities of smooth muscle cells include Activin A , Adrenomedullin, aFGF, ANF, Angiogenin, Angiotensin-2, Betacellulin , bFGF, CLAF , ECDGF (endothelial cell-derived growth factor), ET(Endothelins), Factor X , Factor Xa, HB-EGF, Heart derived inhibitor of vascular cell proliferation, IFN-gamma, IL1, LDGF(Leiomyoma-derived growth factor), MCP-1, MDGF(macrophage-derived growth factor, monocyte-derived growth factor), NPY, Oncostatin M, PD-ECGF, PDGF, Prolactin, Protein S, SDGF(smooth muscle cell-derived growth factor), SDMF(Smooth muscle cell-derived migration factor), Tachykinins, TGF-beta, Thrombospondin.

Factors modulating growth, chemotactic behavior and/or functional activities of vascular endothelial cells include AcSDKP, aFGF, ANF, Angiogenin, angiomodulin, Angiotropin, AtT20-ECGF, B61, bFGF, bFGF inducing activity, CAM-RF, ChDI, CLAF, ECGF, ECI, EDMF, EGF, EMAP-2, Neurothelin(see: EMMPRIN), Endostatin, Endothelial cell growth inhibitor, Endothelial cell-viability maintaining factor, Epo, FGF-5, IGF-2(see: Growth-promoting activity for vascular endothelial cells), HBNF, HGF, HUAF, IFN-gamma, IL1, K-FGF, LIF, MD-ECI, MECIF, NPY, Oncostatin M, PD-ECGF, PDGF, PF4, P1GF, Prolactin, TNF-alpha, TNF-beta, Transferrin, VEGF. Some of these factors are protein factors detected initially due to some other biological activities and later shown to promote angiogenesis. The list of protein factors angiogenically active in vivo includes fibroblast growth factors(see: FGF), Angiogenin, Angiopoietin-1, EGF, HGF, NPY, VEGF, TNF-alpha, TGF-beta, PD-ECGF, PDGF, IGF, IL8, Growth hormone. Fibrin fragment E has been shown also to have angiogenic activity. In addition there are factors such as Angiopoietin-1 which do not behave as classical growth factors for endothelial cells but play a prominent role in vasculogenic and angiogenic processes. PF4 and a 16 kDa fragment of Prolactin are inhibitory in vivo.

Suitable endogenous target compounds include endogenous compounds involved in formation and maintenance of the central and/or peripheral nervous systems, such as neurotrophic factors which enhance neuronal differentiation, induce proliferation, influence synaptic functions, and promote the survival of neurons that are normally destined to die during different phases of the development of the central and peripheral nervous system. Examples of such proteins include neurotrophins, such as BDNF(brain-derived neurotrophic factor), NGF, NT-3 (neurotrophin-3), NT-4, NT-5, NT-6, NT-7, CNTF (ciliary neuronotrophic factor), GDNF (Glial cell line-derived neurotrophic factor), and Purpurin. Growth factors such as bFGF or LIF are frequently also counted among the neurotrophins due to their trophic activities on a number of neurons.

BDNF, NGF and NT-3 are sometimes referred to also collectively as the NGF protein family as NGF is the founder member of this family of proteins. It has been possible, by combination of structural elements from NGF, BDNF and NT-3, to engineer the multifunctional Pan-Neurotrophin-1(PNT-1) that efficiently activates all trk receptors and displays multiple neurotrophic specificities.

Another neuronal survival factor is NSE (neuron-specific enolase). Other factors with neurotrophic activities normally not classified as neurotrophins and often possessing a broader spectrum of functions are EGF, HBNF(heparin-binding neurite-promoting factor), IGF-2, aFGF and bFGF, PDGF, NSE (neuron-specific enolase), and Activin A. For antiproliferative growth factors affecting neural cells see also: Neural antiproliferative protein, Astrostatine, GGIF (glial growth inhibitory factor).

Depending upon their bioactivities a distinction is made sometimes between Neurite promoting factors (NPFs) and Neuronal differentiation factors. Neurite promoting factors do not promote neuronal survival or general growth by themselves but are required in addition to one or more neurotrophic factors to induce outgrowth of axonal or dendritic processes. Factors with NPF activity include NGF, S100, GMF-beta (glia maturation factor), proteoglycans, merosin, collagens, cell adhesion molecules, and laminin.

Suitable endogenous target compounds include endogenous compounds involved in wound healing. Platelets are a rich source of locally active growth factors and cytokines . Among other things platelet-derived factors include adenosine dinucleotide (which induces platelet aggregation and also stimulates cell proliferation and migration), Beta-Thromboglobulin, bFGF, CTAP-3 (Connective tissue activating protein-3), EGF, Eosinophil chemotactic polypeptide-1 (i.e. RANTES ), f-ECGF (Fibroblast derived endothelial cell growth factor ), fibronectin (which serves as an early matrix ligand for platelet aggregation), HCI (human collagenase inhibitor), HGF (hepatocyte growth factor), HRF (histamine-releasing factors), IGF-BP-3 , NAP-2 (neutrophil-activating protein-2), NAP-4 (neutrophil-activating protein-4), PBP (platelet basic protein), PD-ECGF (platelet-derived endothelial cell growth factor), PDGF, PF4, platelet activating factor (PAF, involved also in platelet aggregation), serotonin (which induces vascular permeability and is a chemoattractant for neutrophils), Somatostatin, TGF-alpha, TGF-beta, thromboxane A2 (which is involved in vasoconstriction, platelet aggregation, and chemotaxis), Vitronectin.

Circulating peripheral blood leukocytes migrate into the wound space. The first cells to appear in the wound area are neutrophils. Neutrophil numbers reach peak levels approximately 24 hours after injury. Their migration is stimulated by various chemotactic factors and cytokines, including complement factors, IL1, TNF-alpha, TGF-beta, and chemokines such as IL8, GRO-alpha, PF4, MCP-1, IP-10, mig, and also by bacterial polysaccharides. Neutrophils adhere to the endothelium by means of selectins, which function as neutrophil receptors on the endothelial cell surface. Integrin receptors on the neutrophil cell surfaces facilitate binding of neutrophils to the extracellular matrix. Neutrophils do not appear to play a critical role in wound healing in the absence of infections as wounds can heal in animals in which neutrophils are depleted. Neutrophils are removed by tissue macrophages when they are no longer needed.

Monocytes appear approximately 24 hours after injury and peak at 48 hours post-injury. Since monocytes mature into macrophages they can be considered an essential source of cytokines driving repair processes. Macrophages and monocytes are also attracted by a variety of chemokines. These chemokines contribute to the spatially and temporally different infiltration of leukocyte subsets and thus integrate inflammatory and reparative processes during wound repair.

Tissue macrophages are the cells that essentially control and regulate the wound healing process and wounds cannot heal without the participation of these cells as shown by experiments involving depletion of wound macrophages. The differentiation of macrophages is initiated by several specific cytokines. Many cytokines produced and secreted by activated macrophages favor further migration of inflammatory cells into the wound area. Macrophages also control the degradation of the extracellular matrix and regulate remodeling of the wound matrix. Macrophages secrete cytokines and growth factors including TGF-beta, FGF, VEGF, and chemokines such as JE.

TGF-beta appears to be the major factor responsible for the formation of granulation tissue and the synthesis of proteins of the extracellular matrix and thus has been referred to as a "wound hormone". TGF-beta is a member of one of the most complex groups of cytokine superfamilies, consisting of various TGF-beta isoforms and other family members, for example, Activin A and BMP. The complexity of the wound healing process is illustrated by the observation that manipulation of the ratios of TGF-beta superfamily members, particularly the ratio of TGF-beta-lrelative to TGF-beta-3, reduces scarring and fibrosis.

Re-epithelialization is mediated by chemotactic and mitogenic growth factors of the EGF family of growth factors. Leptin has been shown to be a potent growth factor for keratinocytes during wound healing.

The final phase of wound healing is characterized by the gradual replacement of granulation tissues by connective tissue. This process also requires locally acting cytokines. However, little is known about the factors and mechanisms that eventually restrain tissue growth once the repair process has been completed.

The synthesis of collagen and proteinase inhibitors is stimulated, among other things, by TGF-beta and related factors. Closing of the wound and the evolution of a scar is associated with a striking decrease in cellularity, including disappearance of typical myofibroblasts. It has been suggested that cell death by apoptosis is the mechanism responsible for the evolution of granulation tissue into a scar.

Fetal wound healing is conspicuous for its absence of scarring. There is some evidence that fetal and adult fibroblasts display phenotypic differences in terms of migratory activity, motogenic response to cytokines and the synthesis of motogenic cytokines, growth factors , and matrix macromolecules.

By manipulating the actions of growth factors and cytokines, it is possible to accelerate or modify wound healing. Animal experiments and also clinical experience have demonstrated that the topical administration of various cytokines, including bFGF, EGF, KGF, PDGF, TGF-beta, either alone or in combination, considerably accelerates wound healing by stimulating granulation tissue formation and enhancing epithelialization

Suitable endogenous target compounds include acute phase proteins of inflammation. Suitable examples of such proteins and their functions are provided in table 2.

**Table 2.**

| **Protein** | **Function** |
|---|---|
| alpha-1 acid glycoprotein | interaction with collagen promotion of fibroblast growth binding of certain steroids |
| lalpha-1 antichymotrypsinogen | protease inhibitor |
| alpha- 1 antitrypsin alpha-1 | protease inhibitor resolution of emphysema |
| alpha-2 antiplasmin | modulation of coagulation cascade |
| Alpha-2-Macroglobulin | inhibitor of several serum proteases and other functions |
| Antithrombin-3 | modulation of coagulation cascade |
| C1 inhibitor | negative control of complement cascade |
| C2 | complement component |
| C4 | complement component |
| | complement component |
| C4 binding protein | complement component |
| C5 | complement component |
| C9 | complement component |
| C-reactive protein | binding to membrane phosphorylcholine complement activation and opsonization interaction with T-cells and B-cells |
| Ceruloplasmin | copper transport protein reactive oxygen scavenger |
| Factor VIII | clotting formation of fibrin matrix for repair |
| Factor-B | complement component |
| Ferritin | iron transport protein |
| Fibrinogen | clotting formation of fibrin matrix for repair |
| Fibronectin | fibrin clot formation |
| Haptoglobin | hemoglobin scavenger |
| Heme oxygenase | heme degradation |
| Hemopexin | heme binding and transport protein |
| Heparin cofactor-2 | (proteinase inhibitor |
| Kallikreins | vascular permeability and dilatation |
| LPS binding protein | macrophage cell activation |
| Manganese superoxide dismutase | copper zinc binding protein formation of reactive oxygen species |
| Mannose-binding protein | serum lectin |
| Plasminogen | proteolytic activation of complement, clotting, fibrinolysis |
| Plasminogen activator inhibitor-1 | protease inhibitor |
| Prothrombin | clotting formation of fibrin matrix for repair |
| Serum amyloid A | cholesterol and HDL scavenger |
| Serum amyloid-P | formation of IgG immune complexes |
| von Willebrand factor | coagulation protein |
| IL1ra (IL1 receptor antagonist) | IL 1ra |

The major inducers of acute phase proteins are IL1, IL6, and TNF. The two mediators IL1 and IL6 have been used to classify acute phase proteins into two subgroups. Type-1 acute phase proteins are those that require the synergistic action of IL6 and IL1 for maximum synthesis. Examples of Type-1 proteins are C-reactive protein, serum amyloid A and alpha-1 acid glycoprotein. Type-2 acute phase proteins are those that require IL6 only for maximal induction. Examples of Type-2 proteins are fibrinogen chains, haptoglobin, and alpha-2-Macroglobulin. Expression of genes encoding Type-2 acute phase proteins is suppressed rather than being enhanced frequently by IL1 (Ramadori et al; Fey et al). Additive, synergistic, co-operative, and antagonistic effects between cytokines and other mediator substances influencing the expression of acute phase proteins do occur and have been observed in almost all combinations. Many cytokines also show differential effects, inducing the synthesis of one or two acute phase proteins but not others. For example, Activin A induces a subset of acute phase proteins in HepG2 cells. Bacterial lipopolysaccharides and several cytokines (mainly IL1, IL6 and TNF but also LIF, CNTF, oncostatin M, IL11, and cardiotrophin-1) are involved in the induction of SAA synthesis and some of these cytokines act synergistically (Benigni et al).

IL1 and also IFN-gamma reduce some of the effects of IL6. Some of the effects of IL2 and IL6 are antagonized by TGF-beta. The combined action of two or even more cytokines may produce effects that no factor on its own would be able to achieve. In cultured HepG2 hepatoma cells IL1, IL6, TNF-alpha and TGF-beta induce the synthesis of antichymotrypsin and at the same time repress the synthesis of albumin and AFP (alpha-Fetoprotein). The synthesis of fibrinogen is induced by IL6 and this effect is, in turn, suppressed by IL1-alpha, TNF-alpha or TGF-beta-1.

Suitable endogenous target compounds include endogenous compounds involved in haemostasis and coagulation cascades. Apart from being involved in the regulation of capillary permeability and vessel tonus the endothelium of blood vessels plays a decisive role in maintaining a non-thrombogenic surface by providing activators and inhibitors of coagulation and fibrinolysis. The endothelium is also involved in modulation of various immunological processes.

In endothelial cells IL1 induces, among other things, the synthesis of various colony-stimulating factors, IL6, TNF-alpha, prostaglandins, platelet activating factor (PAF), plasminogen activator inhibitor (PAI). An important endogenous regulator of IL1 is PGE2 which inhibits secretion of IL1 and TNF-alpha. TNF-alpha, among other things, induces the synthesis of tissue thromboplastin (TPL) which participates in the formation of the factor X activator complex that catalyses generation of thrombin. The complex also activates factor IX. TNF-alpha also induces the synthesis of IL1 by endothelial cells and this IL1 can also promote production of TPL by endothelial cells. The synthesis of TNF-alpha can also be induced by IL1. TNF-alpha and IL1 also induce the synthesis of cell surface antigens. The expression of adhesion molecules increases adhesion of lymphocytes and leukocytes at the vessel wall and thus facilitates transendothelial migration of these cells. TNF-alpha and IL1 down-regulate the Protein C inactivation system by inhibiting the synthesis of thrombomodulin. Thrombomodulin complexed with thrombin activates protein C which can then form complexes with membrane-bound protein S. These complexes inhibit factor Va. Activated protein C also neutralizes plasminogen activator inhibitor (PAI) by complexation. TNF-alpha and also IL1 thus reduce inactivation of factor Va.

Other suitable endogenous target compounds involved in coagulation include, Kallikrein, Factor XII, Factor XIb, Factor XI, Factor XIa, Factor IX, Factor IXa, Factor VIII, Factor VIIIa, Factor X, Factor Xa, Factor Va, Factor XIIIa, Prothrombin, Thrombin, Fibrinogen, Fibrin, Plasminigen, Plasmin.

Suitable endogenous target compounds also include hormones, such as stress hormones (e.g., Adrenaline, Adrenocorticotropic hormone, Corticosterone, Epinephrine, Growth Hormone, Hydrocortisone), fluid/electrolyte and vascular hormones (e.g., Aldosterone, Androstenedione, Vasopressin, Bradykinin, Calcitonin, Neurotensin), liver and digestive hormones (e.g., Cholecystokinin, Cholesterol, VIP), reproductive hormones (e.g., Chorionic Gonadotrophin, Epogen, Estradiol, Estriol, Estrone, Etiocholanolone, FSH, Lactogenic Hormone, Lutenizing Hormone, Testosterone, Oxytocin, Progesterone, Prolactin, Gonadotrophin), sugar handling hormones (e.g., Glucagon, Insulin), thyroid hormones (e.g., T2, T3, T4, Thyrotropic Hormone, Thyrotropic releasing factor, TSH, Parathyroid Hormone), bone hormones (e.g., CSF-1, RANKL, Bone Morphogenic Protein family members), and other hormones, such as, Thyroid-stimulating hormone (TSH), Follicle-stimulating hormone (FSH), Luteinizing hormone (LH), Prolactin (PRL), Growth hormone (GH), Adrenocorticotropic hormone (ACTH), Antidiuretic hormone (ADH)(vasopressin), Oxytocin, Thyrotropin-releasing hormone (TRH), Gonadotropin-releasing hormone (GnRH), Growth hormone-releasing hormone (GHRH), Corticotropin-releasing hormone (CRH), Somatostatin, Dopamine, Melatonin, Thyroxine (T4), Calcitonin, Parathyroid hormone (PTH), Glucocorticoids (e.g., cortisol), Mineralocorticoids (e.g., aldosterone), Androgens (e.g., testosterone), Adrenaline (epinephrine), Noradrenaline (norepinephrine), Estrogens (e.g., estradiol), Progesterone, Human chorionic gonadotropin (HCG), Insulin, Glucagon, Somatostatin, Amylin, Erythropoietin (EPO), Calcitriol, Calciferol (vitamin D3), Atrial-natriuretic peptide (ANP), Gastrin, Secretin, Cholecystokinin (CCK), Somatostatin, Neuropeptide Y, Ghrelin, PYY3-36, Insulin-like growth factor-1 (IGF-1), Angiotensinogen, Thrombopoietin, Leptin, Retinol Binding Protein 4, and Adiponectin. Further suitable hormones are listed in Table 3.

**TABLE 3.**

| **Endocrine gland** | **Hormone** | **Abbr.** | **Main function(s)** |
|---|---|---|---|
| Pineal | Melatonin | | Biological clock |
| Hypothalamus | Antidiuretic hormone | ADH | Acts on the kidney to preserve fluid and electrolyte balance |
| | Proopiomelanocortin | POMC | Precursor hormone for ACTH and MSH |
| Pituitary | Luteinizing hormone | LH | In females this LH acts on the ovary to stimulate the production of estrogens and induce ovulation. In males this LH acts on the testis to stimulate the production of testosterone. |
| | Follicle Stimulating hormone | FSH | In females FSH stimulates the maturation of ovarian follicles. In males FSH acts on Sertoli cells and participates in the regulation of sperm production. |
| | Adrenocorticotrophic hormone | ACTH | ACTH acts on the adrenal gland to stimulate the production of cortisol. |
| | Growth Hormone | GH | GH acts of various tissues to stimulate the growth. |
| | Prolactin | Prl | Milk let down during lactation |
| | Melanocyte stimulating hormone | MSH | Stimulates skin tone |
| | Thyroid stimulating hormone | TSH | TSH acts on the thyroid gland to signal the production of thyroxin |
| Pancreas | Insulin | | Regulates blood sugar levels. |
| Thyroid | Triiodothyronine and thyroxine | T3 and T4 | Development of the brain and reproductive tract, and regulation of metabolism |
| Adrenal | Cortisol | | Immune suppression and stress response |
| | Dehydroepiand-rostendione | DHEA | |
| Ovary | Estrogens (estradiol, estrone, estriol) | E2, E1, E3 | Growth promotion, maintain elasticity of connective tissues, preserve bone mass and, vascular compliance, |
| | Progesterone | P4 | Maintain endometrium in preparation for pregnancy |
| | Testosterone | T | Precursor for estrogen and acts on libido. |
| | Inhibin | | Feedback regulation on pituitary FSH secretion |
| Testis | Testosterone | T | Growth of male secondary sexual characteristics, sperm production and libido |
| | Dihydrotestosterone | DHT | Some male secondary sexual characteristics. |
| | Inhibin | | Feedback regulation on pituitary FSH secretion |
| Placenta | Progesterone | P4 | Maintenance of pregnancy |
| | Estriol | E3 | |

Table 4 provides a non-exhaustive list of suitable endogenous target compounds and preferred therapeutics for targeting the endogenous compound with a ligand as described herein. The "Exemplary Identifier" column provides Chemical Abstracts Services (CAS) Registry Numbers (published by the American Chemical Society) and/or Genbank Accession Numbers ((e.g., Locus ID, NP_XXXXX (Reference Sequence Protein), and XP_XXXXX (Model Protein) identifiers available through the national Center for Biotechnology Information (NCBI) webpage at www.ncbi.nlm.nih.gov) that correspond to entries in the CAS Registry or Genbank database which contain an amino acid sequence of the endogenous target compound or of a fragment or variant of the endogenous target compound. The summary pages associated with each of these CAS and Genbank and GenSeq Accession Numbers as well as the cited journal publications (e.g., PubMed ID number (PMID)) are each incorporated by reference in their entireties, particularly with respect to the amino acid sequences described therein. The "Biological activity" column describes biological activities associated with the endogenous target molecule. The "Preferred Indication" column describes disease, disorders, and/or conditions that may be treated, prevented, diagnosed, or ameliorated by a ligand that comprises a moiety that has a binding site with binding specificity for the indicated endogenous target compound.

The invention also relates to a ligand that comprises a moiety having a binding site with binding specificity for any of the endogenous target compounds listed in Table 4 for use in the manufacture of a medicament for treating any of the corresponding preferred indications disclosed in Table 4.

The invention also relates to a method for treating any of the preferred indication listed in Table 4, comprising administering to a subject in need thereof an effective amount of a ligand that comprises a moiety having a binding site with binding specificity for the corresponding endogenous target compound listed in Table 4.

**TABLE 4.**

| Endogenous target compound | Identifier | Biological Activity | Preferred Indication |
|---|---|---|---|
| Acidic FGF | LocusID: 2246 | Binds to cells expressing the Acidic fibroblast growth factor 1 receptor. | Vascular Disease |
| Activin (Activin A; EDF, inhibin, beta A) | CAS-114949-22-3 LocusID: 3624 NP_002183 XP_004832 | The Activin A complex, composed of a homodimer of inhibin beta A subunits, is a pituitary FSH secretion inhibitor, which has been shown to regulate gonadal stromal cell proliferation negatively and to have tumour-suppressor activity. In addition, serum levels of this complex have been shown to reflect the size of granulosa-cell tumors and can therefore be used as a marker for primary as well as recurrent disease. | Fracture; Postmenopausal Osteoporosis; Cancer; Male Infertility; Female Infertility; Sickle Cell Anemia |
| Activin beta c (inhibin, beta c) | LocusID: 3626 NP_005529 XP_006661 | Inhibin beta C subunit is similar to the inhibin/activin beta A and beta B subunits and may be part of a transforming growth factor-beta superfamily. | Neurodegenerative Disorders; Wound Healing; Liver Regeneration |
| Adenosine deaminase | LocusID: 100 NP_000013 XP_009517 | Adenosine deaminase catalyzes the hydrolysis of adenosine to inosine. | Adenosine Deaminase Deficiency In Patients With Severe Combined Immunodeficiency (SCID) |
| Adiposin | CAS-9035-55-6 Genbank: 223277 Genbank: 223281 | Adiposin is produced by the sequential cleavage of the precursor protein pre-pro-opiomelanocortin (POMC). Adiposin plays a key role in the regulation of food intake via activation of the brain melanocortin-4-receptor. | Obesity; Cancer |
| Agouti signal protein | LocusID: 434 NP_001663 XP_009476 | Agouti Signaling Protein (ASP), encodes a 132 amino acid protein, the mRNA for which is expressed in testis, ovary, and heart, and at lower levels in liver, kidney, and foreskin. ASP interacts with the extension gene (which encodes the melanocyte receptor for alpha-melanocyte stimulating hormone [alpha-MSH]), and expression of ASP in cell culture blocks the alpha-MSH-stimulated accumulation of cAMP in mouse melanoma cells. | Pigmentation Disorders; Obesity |
| Alpha glucosidase (Acid alpha-glucosidase; Pompase) | LocusID: 2548 NP_000143 XP_012708 | Hydrolysis of alphal-6 linkages in glycogen; debranching enzyme; degradation of starch in muscle, liver and other tissues | Pompe's Disease |
| Alpha-galactosidase | LocusID: 2717 | Hydrolytic cleavage of galactose residues | Fabry's Disease |
| (Arginosuccinate lyase; Beta-glucosidase; x-galactosidase A; agalsidase alpha; CC-galactosidase) | NP_000160 XP_010108 | from the glycosphingolipid galactosylgalactosylglucosylceramide | |
| Alpha-L iduronidase (Alronidase; Laronidase; Iduronato-2-sulfatase) | LocusID: 3425 NP_000194 | Iduronidase is a lysosomal enzyme that participates in the degradation of dermatan sulfate and heparan sulfate. | Mucopolysaccharidosis I; Mucopolysaccharidosis Ii; Hurler Syndrome; Hurler-Scheie Syndrome; Scheie Syndrome; Hunter Syndrome |
| Angiopoietin 1 | NP_001137 | Angiopoeitin 1 is a growth factor involved in the control of angiogenesis through the Tie receptors. | Inflammatory Disorders; Cardiovascular Disorders |
| Angiopoietin 2 | Genbank: BAA95590 | Angiopoeitin 2 is a growth factor involved in the control of angiogenesis through the Tie receptors. | Cancer |
| Angiostatin (Angiostatin) | CAS-86090-08-6 LocusID: 5340 NP_000292 XP_004371 | Angiostatin represents an internal fragment of plasminogen (containing the first four kringle structures). Individual or combined kringle structures of angiostatin have been shown to inhibit effects of on capillary endothelial cell proliferation. | Solid Tumors |
| Anti-dorsalizing morphogenetic protein-1 (ADMP) | Genbank: AAC59736 Genbank: AAD52011 | ADMP is related to human Bone Morphogenetic Protein-3. Its expression peaks during gastrulation and results in dose-dependent suppression of dorsal and anterior structures. | Cancer |
| AP02 Ligand (TRAIL) | LocusID: 8743 NP_003801 XP_003200 | Type II glycoprotein of the tumor necrosis factor ligand superfamily; mediates cell death. TRAIL can induce apoptosis in a wide variety of transformed cell lines of diverse lineage, but does not appear to kill normal cells. | Cancer |
| Arresten | Genbank: AF72630 | Arresten functions as an anti-angiogenic molecule by inhibiting endothelial cell proliferation, migration, tube formation, and Matrigel neovascularization. | Cancer; Solid Tumors |
| Arylsulfatase B (BM 102; recombinant human N-acetylgalactosamine-4-sulfatase; rhASB) | LocusID: 411 NP_000037 Genbank: AAB19988 Genbank: CAA51272 Genbank: AAA51784 Genbank: AAA51779 | The arylsulfatase B homodimer hydrolyzes sulfate groups ofN-Acetyl-D-galactosamine, chondriotin sulfate, and dermatan sulfate. The protein is targeted to the lysozyme. Defects in this gene cause Maroteaux-Lamy syndrome. | Mucopolysaccharidosis VI |
| Asparaginase | CAS-130167-69-0 Genbank: CAA01168 | Asparaginase is the enzyme responsible for hydrolysis of L-Asparagine. | Acute Lymphoblastic Leukemia |
| Bactericidal permeability increasing protein 21 | LocusID: 671 NP_001716 AAA51841 | A membrane-associated protein, similar to LBP, CETP, and PLTP, with bactericidal activity. | Ocular Inflammation; Cystic Fibrosis; Haemorrhagic Trauma; Intra-Abdominal Infections; Meningococcal Infection; Meningococcemia; Otitis Media; Partial Hepatectomy; Toxoplasmosis |
| BDNF (Brain-derived neurotrophic factor) | LocusID 627 NP_001700 XP_006027 the | Brain-derived neurotrophic factor promotes the survival of neuronal populations that are all located either in the central nervous system or directly connected to it. | Amyotrophic Lateral Sclerosis; Neurodegeneration |
| B-glucocerebrosidase | CAS-143003-46-7 CAS-154248-97-2 LocusID: 2629 NP_000148 XP_002191 | glucocerebrosidase (beta-glucosidase) catalyzes the removal of glucose from glucosylceramide to form ceramide | Gaucher's Disease |
| BMP-2 (Bone Morphogenetic Protein 2; Bone-related protein) | CAS-192509-82-3 LocusID: 650 NP_001191 XP_009629 | BMP2 belongs to the transforming growth factor-beta (TGFB) superfamily. Bone morphogenic protein induces bone formation. BMP2 is a candidate gene for the autosomal dominant disease of fibrodysplasia (myositis) ossificans progressiva. | Bone Regeneration; Bone And Tissue Repair; Cancer |
| BRCA1 (BRCA1 tumor suppressor protein) | Locus ID: 672 NP_006759 XP_007013 | BRCA1, a tumor suppressor in human breast cancer cells, is a nuclear phosphoprotein which associates with RNA polymerase II holoenzyme. BRCA1 functions as a transcriptional regulator and is also a granin-like protein that functions as a secreted growth inhibitory protein. | Cancer; Breast Cancer; Prostate Cancer; Ovarian Cancer |
| BRCA2 | Locus ID: 675 NP_000050 XP_007138 | BRCA2, a tumor suppressor in human breast cancer cells, is a nuclear phosphoprotein which associates with RNA polymerase II holoenzyme. BRCA2 exists in a dimeric complex with BRCA1, functions as a transcriptional regulator, and is also a granin-like protein that functions as a secreted growth inhibitory protein. | Cancer; Breast Cancer; Ovarian Cancer |
| Calcitonin gene-related peptide (CGRP) | CAS-83652-28-2 CAS-83652-28-2 Locus ID: 796 Locus ID: 797 Locus ID: 27297 NP_001732 NP_000719 NP_055293 XP_006209 XP_006016 XP_004758 | CGRP is a potent vasodilator, and a regulator of endothelial and osteoblast cell proliferation. Additional effects of CGRP include reduced gastric secretion, increased body temperature, anorexic effects, and positive inotropic and chronotropic effects on the heart. | Angina Pectoris; Arrythmias; Heart Failure; Hypertension; Postmenopausal Osteoporosis; Raynaud's Disease; Subarachnoid Haemorrhage |
| Calreticulin | Locus ID: 811 NP_004334 XP_009055 Genbank: AH02500 | Calreticulin is a multifunctional protein that acts as a major Ca(2+)-binding (storage) protein in the lumen of the endoplasmic reticulum. Calreticulin can act as an important modulator of the regulation of gene transcription by nuclear hormone receptors. | Cancer; Wound Healing |
| CD4 | Locus ID: 920 NP_000607 XP_006966 | CD4 is a T-cell surface glycoprotein which plays roles in cell-cell interactions in signal transduction. CD4 binds gp120 of HIV and is a cell surface receptor for HIV entry. | Hiv Infection |
| CD40 ligand | LocusID: 959 NP_000065 XP_010367 | CD40-L is expressed on the surface of T cells. It regulates B cell function by engaging CD40 on the B cell surface. A defect in this gene results in inability of immunoglobulin class switch and is associated with hyper IgM syndrome. | Epithelial Solid Tumors; Head And Neck Cancer; Immunodeficiency Disorders; Non-Hodgkin's Lymphoma; Renal Cancer; Renal Cell Carcinoma; Solid Tumors; Viral Infections |
| Chemokine Binding Proteins (CBP 1; CPB 2) | LocusID: 1238 NP_001287 XP_003126 LocusID: 1241 NP_000743 XP_007314 | Chemokine binding proteins are involved in chemokine-mediated signaling. | Transplant Rejection; Cardiovascular Disease; Rheumatoid Arthritis; Inflammatory Disorders; Immune System Disorders |
| Ciliary neurotrophic factor (CNTF) | LocusID: 1270 NP_000605 XP_006012 | CNTF promotes the differentiation and survival of a wide range of cell types in the nervous system | Amyotrophic Lateral Sclerosis; Diabetic Neuropathies; Huntington's Disease; Obesity; Retinal Disorders; Type 2 Diabetes Mellitus |
| Contortrostatin | CAS-153858-68-5 | Contortrostatin is a unique dimeric disintegrin which antagonizes integrins | Cancer Metasteses; Thrombosis; Stroke |
| Corticotropin releasing factor binding protein | LocusID: 1393 NP_001873 XP_003672 Genbank: P24387 Genbank: CAA41086 | Corticotropin-releasing factor (CRF) is a peripheral and a central mediator of inflammation and stress-related responses. CRF-binding protein inactivates CRF. | Rheumatoid Arthritis; Inflammation |
| CTLA4 | LocusID: 1493 NP_005205 XP_002490 | CTLA4 is a B7 ligand receptor normally expressed on activated T cells. CTLA4 Ig is a soluble chimeric receptor with anti-inflammatory activity. | Organ Transplant Rejection; Allergies; Rheumatoid Arthritis; Graft-Versus-Host Disorders; Psoriasis; Type 1 Diabetes Mellitus; Xenotransplant Rejection |
| Decorin | LocusID: 1634 XP_012239 NP_001911 | This member of the specialized collagens and SLRP family is a small proteoglycan that interacts with collagen and growth factors, and is involved in epithelial/mesenchymal interactions during organ development and shaping. | Cancer; Diabetic Nephropathies; Inflammatory Disorders; Post-Surgery Adhesions; Pulmonary Fibrosis |
| Del-1 (Developmentally-regulated endothelial locus-1; EDIL3; EGF-like repeats and discoidin I-like domains 3) | LocusID: 10085 Genbank: U70312 NP_005702 XP_003954 | a new ligand for the alphavbeta3 integrin receptor and may function to regulate vascular morphogenesis or remodeling in embryonic development | Ischemia; Cancer; Restenosis |
| DNASE | CAS-143831-71-4 CAS-9003-98-9 CAS-132053-08-0 LocusID: 1773 NP_005214 XP_008097 | Dnase is an endonuclease that selectively cleaves DNA and removes DNA from nuclear antigens at sites of high cell turnover | Chronic Obstructive Pulmonary Disease; Cystic Fibrosis; Lupus Nephritis |
| Ectoapyrases (CD39 family; human ectoapyrase (ecto-ADPases); CD39-L2; CD39-L4) | LocusID: 953 LocusID: 954 LocusID: 955 LocusID: 956 LocusID: 957 NP_001767 NP_001237 NP_001238 NP_001239 NP_001240 XP_005712 XP_011771 XP_009435 XP_003296 XP_007435 | Mediates catabolism of extracellular nucleotides | Myocardial Infarction; Stroke |
| EGF (Epidermal growth factor; | CAS-62229- | EGF has bifunctional regulatory properties: it inhibited the growth of several epithelial tumor cells and stimulated the growth of fibroblast and other cell types | Gastric Ulcers; Wound Healing; Coronary Restenosis; Vascular Restenosis; Non-Small Cell Lung Cancer; Psoriasis |
| EMAP II (Endothelial monocyte activating polypeptide II) | LocusID: 9255 LocusID: 13722 NP_004748 XP_003390 | EMAP-II is a tumor-derived cytokine which inhibits angiogenesis, exhibits properties of a proinflammatory mediator, and exerts potent effects on endothelial cells in vitro and in vivo. | Cancer |
| FGF-1 | LocusID: 2246 NP_000791 | FGF-1 stimulates proliferation and angiogenesis | Peripheral Vascular Disease; Peripheral Arterial Occlusive Disease; Critical Limb Ischemia |
| FGF-2 (Fibroblast Growth Factor-2; Fiblast) | CAS-131094-16-1 LocusID: 2247 NP_001997 XP_003306 | Fibroblast growth factor. Angiogenic growth factor | Coronary Disorders; Fracture; Periodontal Disease; Peripheral Vascular Disease; Postmenopausal Osteoporosis; Skin Ulcer; Stroke; Peripheral Artery Disease; Coronary Artery Disease |
| FLT3 ligand | LocusID: 2323 NP_001450 XP_008921 | Stimulates growth and survival of hematopoietic progenitor cells. Stimulates dendritic cell development. | Chemoprotection; Haematological Disorders; Malignant Melanoma; Myelosuppression; Non-Hodgkin's Lymphoma; Prostate Cancer; Stem Cell Mobilization |
| Follitropin | CAS-146479-72-3 CAS-56832-30-5 CAS-110909-60-9 CAS-150490-84-9 CAS-97048-13-0 LocusID: 1081 LocusID: 2488 NP_000726 NP_000501 XP_011444 XP_006316 | Follitropin is a pituitary glycoprotein hormone which stimulates steroidogenesis via a cAMP pathway. | Female Infertility; Male Infertility |
| GDNF (Glial-derived neurotrophic factor; Neurturin) | CAS-185857-51-6 LocusID: 2668 NP_000505 XP_003703 | Glial-derived neurotrophic factor (GDNF) is a distant member of the transforming growth factor-beta (TGF-beta) superfamily which acts intracellularly via the receptor tyrosine kinase Ret. GDNF is a potent survival factor for midbrain dopamine neurons, motoneurons, noradrenergic neurons, as well as for sympathetic, parasympathetic and sensory neurons. However, for most neuronal populations, except for motoneurons, TGF-beta is required as a cofactor for GDNF. GDNF also has distinct functions outside the nervous system, promoting ureteric branching in kidney development and regulating spermatogenesis. | Amyotrophic Lateral Sclerosis; Parkinson's Disease; Huntingdon's Disease |
| Gelsolin | LocusID: 2934 NP_000168 Genbank: CAA28000 | Gelsolin is a calcium-dependent protein which severs and caps actin filaments, and has been shown to decrease the viscosity of cystic fibrosis sputum. | Bronchitis; Cystic Fibrosis |
| Glial growth factor-2 (GGF-2; Neuregulin 1) | LocusID: 3084 NP_039256 Genbank: AAB59622 | GGF2 is an isoform of neuregulin 1, containing a kringle-like sequence plus Ig and EGF-like domains. The GGF2 receptor is a member of the ERBB family of tyrosine kinase transmembrane receptors. Through interaction with ERBB receptors, GGF2 plays a central role in neuronal and glial development in the central nervous system. | Multiple Sclerosis; Chemotherapy-Induced Neuropathy; AIDS Neuropathy; Diabetic Neuropathy; Peripheral Neuropathies |
| Glucagon | CAS-16941-32-5 CAS-118549-37-4 LocusID: 2641 NP_002045 XP_002210 | Glucagon is a 29 amino acid peptide hormone liberated in the A cells of the islets of Langerhans. Glucagon is produced in response to a drop in blood sugar concentration. Its effect is to raise serum glucose. In the treatment of severe hypoglycaemia in insulin-dependent diabetics, glucagon causes the liver to release glucose by stimulating the conversion of glycogen to glucose. Glucagon also relaxes gastrointestinal smooth muscle, allowing for improved radiological exams. | Hypoglycemia; Gastrointestinal Tract Diagnostic; Diabetes Mellitus; Obesity; Type 2 Diabetes Mellitus |
| HBNF (Heparin-binding neurotrophic factor; PTN; pleiotrophin; heparin binding growth factor 8; neurite growth-promoting factor 1; NEGF1) | LocusID: 5764 NP_002816 Genbank: AAA41311 Genbank: M68916 | HBNF stimulates neurite outgrowth in neurons and is expressed in a developmentally regulated manner in the rat brain. | Neuropathies; Neurological Disorders |
| HCG (Human chorionic | LocusID: 1081 | Human chorionic gonadotropin is a | Breast Cancer; Kaposi's |
| gonadotropin; Pregnancy Urine Hormone; Ovidrel; Ovidrelle; APL) | LocusID: 1082 NP_000726 NP_000728 XP_011444 XP_012903 | heterodimer of a common alpha chain and a unique beta chain (chorionic gonadotropin beta), which confers biological specificity to a number of glycopeptide hormones including thyrotropin, lutropin, follitropin and gonadotropin. Only the heterodimer is functional. Its normal function is to stimulate the ovaries to synthesize the steroids that are essential for the maintenance of pregnancy. | Sarcoma; Female Infertility; Hypogonadism; Male Infertility |
| Heat shock protein (HSP; HSP96; gp96) | LocusID: 3316 Genbank: M11717 Genbank: M15432 Genbank: X15183 Genbank: M33716 | Heat shock proteins chaperone antigenic peptides in the display pathway. Gp96 elicits a tumor-specific killing response when complexed with antigenic tumor antigen. | Solid Tumors; Colorectal Cancer; Gastric Cancer; Malignant Melanoma; Non-Hodgkin's Lymphoma; Pancreatic Cancer; Renal Cancer; Sarcoma; Epilepsy; Neuroprotection; Stroke |
| IL-1 (Interleukin-1; interleukin-1 alpha; interleukin-1 beta) | LocusID: 3552 LocusID: 3553 NP_000566 NP_000567 XP_010760 | The cytokine interleukin-1 (IL-1)1 elicits a wide array of biological activities that initiate and promote the host response to injury or infection, including fever, sleep, loss of appetite, acute phase protein synthesis, chemokine production, adhesion molecule up-regulation, vasodilatation, the pro-coagulant state, increased hematopoiesis, and production and release of matrix metalloproteinases and growth factors. | Bacterial Infections; Peptic Ulcer; Transplant Rejection; Cancer; Malignant Melanoma; Non-Small Cell Lung Cancer; Preleukemia; Chemoprotection; Radioprotection |
| IL-1 receptor antagonist | CAS-143090-92-0 LocusID: 3557 NP_000568 XP_010756 | The IL1 receptor antagonist is a protein that binds avidly to IL1 receptors without activating the target cells and inhibits the binding of IL1-alpha and IL1-beta. As a consequence, the biologic activity of these 2 cytokines is neutralized in physiologic and pathophysiologic immune and inflammatory responses. | Rheumatoid Arthritis; Asthma; Diabetes Mellitus; GVHD; Inflammatory Bowel Disorders; Ocular Inflammation; Psoriasis; Septic Shock; Transplant Rejection; Inflammatory Disorders; Rheumatic Disorders; Postmenopausal Osteoporosis; Stroke |
| IL-10 | LocusID: 3586 NP_000563 XP_001409 | IL-10 inhibits the synthesis of a number of cytokines, including ifn-gamma, il-2, il-3, tnf and gm-csf produced by activated macrophages and by helper t cells. | Inflammatory Bowel Diseases; Acute Lung Injury; Autoimmune Disorders; Cancer; Crohn's Disease; Graft-Versus-Host Disorders; Growth Disorders; Hepatic Fibrosis; Hepatitis C; Herpes Simplex Virus Infections; HIV Infections Treatment; Ischaemia/Reperfusion; Multiple Sclerosis; Myocarditis; Psoriasis; Rheumatoid Arthritis; Sepsis; Transplant Rejection; Type 1 Diabetes Mellitus; Ulcerative Colitis; Rheumatoid Arthritis; Inflammatory Bowel Diseases |
| IL-11 | CAS-145941-26-0 LocusID: 3589 NP_000632 XP_008906 | stromal cell-derived cytokine, initially characterized as a hematopoietic cytokine with thrombopoietic activity, IL-11 has now been shown to be expressed and exhibits pleiotropic action on hematopoietic cells and multiple other tissues, including brain, spinal cord neurons, gut, and testis. | Thrombocytopenia; Bone Marrow Transplant Rejection; Crohn's Disease; Graft Versus Host Disorders; Inflammatory Disorders; Mucositis; Rheumatoid Arthritis; Sepsis-Induced Systemic Inflammatory Response Syndrome |
| IL-12 | LocusID: 3592 | IL-12 exhibits antitumor, antiviral, and | Cancer; Hepatitis C; |
| IL18 binding protein | LocusID: 10068 NP_005690 XP_006006 | IL18 binding protein inhibits the early Th1 cytokine response, it is a member of the immunoglobulin superfamily. | Autoimmune Disease; Inflammation; Rheumatoid Arthritis |
| IL2 | LocusID: 3558 | Binds to and activates cells expressing the IL2 receptor. | cancer Infection; Rheumatoid Arthritis; Cancer |
| IL-4 | LocusID: 3565 | Binds to and activates cells expressing the IL4 receptor. | Cancer; Acute Immunodeficiency |
| IL-4 Receptor | LocusID: 3566 NP_000409 XP_007989 | Binds to and can block activity of IL4 which has a wide range of effects including inducing B-cells to proliferate and induce cytokine production; inducing maturation of thymocytes; activation of mature T-cells, NK cells, modulates myelopoesis, enhancement of eosinophil and mast cell generation; proliferation of endothelial cells. | Asthma; Graft-Versus-Host Disorders; Hypersensitivity |
| IL-8 (Interleukin-8) | LocusID: 3576 NP_000575 XP_003501 | IL-8 Interleukin 8 is a cytokine that plays a role in chemoattraction and activation of neutrophils. It has similarity to several platelet-derived factors. | Labor Induction |
| Interferon gamma | CAS-98059-61-1 LocusID: 3458 NP_000610 XP_006883 | Anti-vial, anti-viral and immunomodulatory activities. | Serious Infections Associated With Chronic Granulomatous Disease; Severe; Malignant Osteopetrosis; Tuberculosis; Atopic Dermatitis; Chronic Granulomatous Disease; Cryptococcoses; Cystic Fibrosis; Keloids; Malignant Melanoma; Mycobacterial Infections; Mycoses; Ovarian Cancer; Pulmonary Fibrosis; Renal Cancer; Small Cell Lung Cancer; Systemic Scleroderma; Mycosis Fungoides; Skin Cancer |
| Interferon omega | LocusID: 3467 NP_002168 XP_005411 | Regulates antiviral defence, cell growth, and immune activation; member of the type I interferon family of proteins. | Cancer; Hepatits C |
| Interferon-inducible protein 10 | LocusID: 3627 NP_001556 CAA26370 | IFN-gamma-inducible protein is a member of the C-X-C chemokine family, and has chemotactic and mitogenic activity. | Leukaemia |
| Interleukin-3 | CAS-148641-02-5 CAS-161753-30-6 LocusID: 3562 NP_000579 XP_003752 | Interleukin-3 (colony-stimulating factor) is a member of a family of growth factors which plays a role in hematopoeisis. It is capable of supporting the of a broad range of hematopoietic cell types. | Cancer; Hematological Disorders; Hematological Malignancies; Preleukemia; Hodgkin's Disease; Myeloid Leukemia; Non-Hodgkin's Lymphoma; Thrombocytopenia; Chemoprotection; Radioprotection |
| KGF-1 (Keratinocyte growth factor-1) | LocusID: 2252 NP_002000 | Keratinocyte growth factor 1 (KGF-1) is a human mitogen that is specific for epithelial cells. It has the properties of a stromal mediator of epithelial cell proliferation. | Chemoprotection; Graft-Versus-Host Disorders; Mucositis |
| Kunitz protease inhibitor 1 (KPI 1) | LocusID: 6692 NP_003701 XP_007555 | Kunitz protease inhibitor 1 is a potent inhibitor specific for HGF activator and is thought to be involved in regulation of proteolytic activation of HGF in injured tissues. | Thrombosis; Anticoagulant |
| Lactoferrin (Apolactoferrin) | Locus ID: 4057 NP_002334 XP_010963 | Lactoferrin, a member of the transferrin family, transports iron in extracellular fluid, and has antibacterial and antiviral properties. | Allergic Contact Dermatitis; Bacterial Infections; Cardiovascular Disorders; Coagulation Disorders; Dry Eye; Gastritis; Hepatitis C; Psoriasis |
| Leptin | LocusID: 3952 NP_000221 XP_004625 | Secreted by fat cells. Obesity control. Modulation of food intake, reduction of body weight, and lowering of insulin and glucose level. | Obesity; Type 2 Diabetes Mellitus; Vascular Disorders; Immunological Disorders; Immunosuppression |
| Leukemia inhibitory factor | LocusID: 3976 NP_002300 XP_009915 | Leukaemia inhibitory factor is a cytokine that induces macrophage differentiation. LIF acts in conjunction with BMP2 on primary fetal neural progenitor cells to induce astrocytes. | Female Infertility; Neuromuscular Disorders |
| Lys plasminogen (Recombinant truncated plasminogen) | Locus ID: 5340 NP_000292 XP_004371 | A more reactive, truncated form of plasminogen. Plasminogen is the precursor of plasmin, a serine protease that digests fibrin in clots. | Thrombosis; Arterial Occlusive Disorders |
| Maspin | CAS-157857-21-1 Locus ID: 5268 NP_002630 XP_008742 | A serine protease inhibitor that suppresses tumor metastasis by inhibiting angiogenesis. | Cancer; Breast Cancer; Prostate Cancer |
| Methioninase | CAS-42616-25-1 Genbank: AAB03240 | Methioninase catalyzes alpha, gamma-elimination reactions of homocysteine and its S-substituted derivatives as well as alpha, beta-elimination reactions of cysteine and its derivatives. It also catalyzes exchange reactions between the substituent at the gamma-carbon of homocysteine substrates and exogenously added alkanethiols, forming the corresponding S-alkylhomocysteines. It also catalyzes similar beta-exchange reactions between cysteine and alkanethiols. | Cancer; Gastrointestinal Cancer; Non-Small Cell Lung Cancer; Pancreatic Cancer |
| Microsomal transfer protein | Locus ID: 4547 NP_000244 XP_003363 | MTP encodes the large subunit of the heterodimeric microsomal triglyceride transfer protein. Protein disulfide isomerase (PDI) completes the heterodimeric microsomal triglyceride transfer protein, which has been shown to play a central role in lipoprotein assembly. | Lipid Disorders |
| MSH-diphtheria toxin chimera | Locus ID: 5443 NP_000930 XP_002485 K01722 AAA32182 | Binds to and kills cells expressing the MSH receptor. | Cancer |
| Nerve growth factor (NGF) | LocusID: 4803 NP_002497 XP_002122 | A growth factor with roles in neuronal differentiation and survival. | HIV-Associated Sensory Neuropathy; Diabetic Neuropathy; Neurodegenerative Disorders |
| Neutral endopeptidase (NEP) | CAS-82707-54-8 Locus ID: 4311 NP_000893 NP_009218 NP_009219 NP_009220 XP_003136 XP_003137 XP_003138 XP_003139 | Neutral endopeptidase is a 100-kD type II transmembrane glycoprotein that is particularly abundant in kidney, where it is present on the brush border of proximal tubules and on glomerular epithelium. NEP cleaves peptides at the amino side of hydrophobic residues and inactivates several peptide hormones including glucagon, enkephalins, substance P, neurotensin, oxytocin, and bradykinin. | Cancer; Migraine; Inflammatory Bowel Disease; Inflammation; Asthma; Respiratory Disease; Lung Cancer; Small Cell Lung Cancer |
| NIF (Neutrophil inhibitory factor) | Genbank: AAA27789 | NIF is a glycoprotein inhibitor of a number of neutrophil functions, such as adhesion to endothelial cells and adhesion-dependent release of hydrogen peroxide. These functional effects resulted from its specific binding to alphaMbeta2 but not to other beta2 integrins. NIF has been shown effective in attenuating the deleterious effects of excessive neutrophil activation, such as tissue damage and ischemia-reperfusion injury in animal models | Stroke |
| Noggin | Locus ID: 9241 NP_005441 XP_008151 | Noggin binds and inactivates members of the transforming growth factor-beta (TGF-beta) superfamily signaling proteins, such as bone morphogenetic protein-4 (BMP4). | Fibrodysplasia Ossificans Progressiva; Abnormal Bone Growth; Pathological Bone Growth |
| NT-3 (Neurotrophin 3) | LocusID: 4908 NP_002518 | NT-3 is a member of a family of neurotrophic factors, neurotrophins, that control survival and differentiation of mammalian neurons. This gene is closely related to both nerve growth factor and brain-derived neurotrophic factor. The protein encoded by this gene may be involved in the maintenance of the adult nervous system, and affect development of neurons in the embryo when it is expressed in human placenta. | Enteric Neuropathies; Constipation; Diabetic Neuropathies; Peripheral Nerve Disorders |
| Osteogenic protein-1 (OP-1) | LocusID: 655 NP_001710 XP_012943 | OP-1 is a member of the transforming growth factor-beta superfamily of regulatory molecules which signals through receptor serine/threonine kinases to stimulate cellular responses. Its effects include increased VEGF expression by fetal calvaria cells and increased dendritic outgrowth in cerebral cortical neurons. | Acute Fractures; Cartilage Repair; Neurological Disorders; Orthopaedic Reconstruction; Parkinson's Disease; Periodontal Tissue Repair; Renal Failure; Spinal Fusion; Stroke; Tooth Dentin Regeneration; Long Bone Nonunions |
| Osteoprotegrin (Osteoclastogenesis Inhibiting Factor) | Locus ID: 4982 NP_002537 Locus ID: 7941 NP_005075 XP_004492 | Osteoprotegrin inhibits osteoclastogenesis and bone resorption, and induces fibroblast proliferation. Platelet-activating factor acetylhydrolase inactivates platelet-activating factor and related phospholipids. | Bone Disorders; Cancer Pain; Postmenopausal Osteoporosis; Rheumatoid Arthritis Asthma; Necrotizing Enterocolitis; Acute Enterocolitis; Adult Respiratory Distress Syndrome; Allergic Asthma; inflammatory Bowel Disorders; Ischaemia/Reperfusion; Pancreatitis; Sepsis-Induced Systemic Inflammatory Response Syndrome; Solid Organ Preservation; Type 1 Diabetes Mellitus; |
| Patched (hedgehog receptor) | Locus ID: 5727 NP_000255 XP_005574 | Patched, the receptor for Sonic hedgehog, is a tumour-suppressor which controls developmental patterning and growth. | Basal Cell Cancer; Brain Cancer |
| PDGF (PGDF-B) | CAS-165101-51-9 LocusID: 5154 LocusID: 5155 NP_002598 NP_002599 XP_009997 | PDGF-B is a potent mitogen and transforming agent. It acts through the PDGF receptor tyrosine kinase to exert its mitogenic and transforming effects. | Lower-Extremity Diabetic Neuropathic Ulcers; Decubitus Ulcer; Diabetic Foot Ulcer; Venous Stasis Ulcers; Periodontal Disease; Skin Ulcer; |
| PEDF (Pigment epithelium-derived factor) | Locus ID: 5176 NP_002606 | Pigment epithelium-derived factor is a noninhibitory member of the serpin family of serine protease inhibitors. It acts as a neurite-promoting factor by a mechanism other than serine protease inhibition. | Amyotrophic Lateral Sclerosis; Inflammatory Eye Diseases; Vascular Eye Diseases; Degenerative Eye Diseases; Dystrophic Eye Disease; Neuropathies |
| Plasminogen activator inhibitor (Plasminogen activator inhibitor-1) | Locus ID: 5054 NP_000593 XP_004828 | Plasminogen activator inhibitor I regulates fibrinolysis by inhibiting the conversion of inactive plasminogen to plasmin. Plasminogen activator inhibitor I is a member of the serpin family of serine protease inhibitors. | Hemorrhage |
| Prosaptide | Genbank: AAG31635 Pubmed: PMID: 9114068 | Prosaptide is a neurotrophic peptide (TXLIDNNATEEILY; where X equals D-alanine) derived from prosaposin. | Diabetic Nephropathy; Pain; Central Nervous System Disorders |
| Relaxin (Human relaxin) | LocusID: 6013 LocusID: 6019 NP_008842 NP_005050 XP_011804 XP_005512 | Relaxin is a peptide hormone synthesized in the corpora lutea of ovaries during pregnancy and is released into the blood stream prior to parturition. Its major biological effect is to remodel the mammalian reproductive tract to facilitate the birth process. | Scleroderma; Infertility; Congestive Heart Failure; Labour Disorders; Peripheral Arterial Disorders; Pulmonary Fibrosis; Pulmonary Hypertension |
| Retinal S-antigen (Retinal Arrestin) | Genbank: AAA30378 | Retinal S-Antigen is a photoreceptor protein which binds to phosphorylated rhodopsins and plays a role in deactivating the phototransduction cascade. | Uveitis |
| Human luteinizing hormone | LocusID: 1081 LocusID: 3972 NP_000726 NP_000885 XP_011444 XP_009418 | hLH is a pituitary hormone dimer consisting alpha and beta subunits that are associated noncovalently. Its main effects are in the maturation of oocytes and the secretion of estrogen and progesterone by ovarian follicles. | Infertility |
| Saruplase | CAS-99149-95-8 Genbank: CAA01515 | Saruplase is an unglucosylated single-chain recombinant urokinase-type plasminogen activator. | Myocardial Infarction |
| Soluble complement receptor type 1 | LocusID: 1378 NP_000564 NP_000642 | Complement receptor type 1 (C3b/C4b receptor) is the receptor for C3b-and C4b-coated ligands. It lacks transmembrane and cytoplasmic domains and inhibits C3 and C5 convertase activity by preferentially binding C4b and C3b. | Myocardial Infarction; Acute Respiratory Distress Syndrome; Adult Respiratory Distress Syndrome; Allotransplant Rejection; Disseminated Intravascular Coagulation; Lung Transplant Rejection; Multiple Sclerosis; Reperfusion Injury; Rheumatoid Arthritis; Systemic Lupus Erythematosus; Xenotransplant Rejection |
| Sonic hedgehog (hedgehog) | LocusID: 6469 NP_000184 XP_004942 | Sonic hedgehog plays a central role in patterning of the embryo. It occurs as an inactive precursor which must be proteolytically cleaved and modified to become active as a signalling molecule. The receptor for Sonic hedgehog is Patched, a multi-pass transmembrane protein, and downstream targets of Sonic hedgehog signalling are transcription factors like Gli3. | Parkinson's Disease; Neurological Disorders; Alopecia |
| Staphylokinase | Genbank: CAA29822 Genbank: CAA01335 | Staphylokinase is a single domain protein that proteolytically activates plasminogen. | Myocardial Infarction; Arterial Occlusive Disorders; Stroke |
| Stem cell factor (Kit ligand) | CAS-163545-26-4 NP_003985 | SCF is substantially necessary for mast cell survival and induces marginal mast cell proliferation in vitro. In the presence of SCF, mast cells predominantly produce pro-inflammatory cytokines including tumor necrosis factor (TNF)-alpha, IL-1beta, IL-6, IL-8, IL-16, and IL-18. | Mobilization Of Progenitor Cells Before PBPCT In Breast Cancer; In Conjunction With Neupogen; Stem Cell Mobilization; Anemia; Xenotransplant Rejection |
| Streptokinase | CAS-9002-01-1 Genbank: E03308 | Streptokinase is a 3 domain (alpha, beta, gamma) molecule, which non-proteolytically activates human (h)-plasminogen and protects plasmin from inactivation by alpha2-antiplasmin. | Myocardial Infarction; Blood Clots; Pulmonary Embolism |
| Superoxide dismutase | CAS-9016-01-7 LocusID: 6647 LocusID: 6648 LocusID: 6649 NP_000445 NP_000627 NP_003093 XP_009723 XP_004242 XP_003578 | Copper zinc superoxide dismutase is an intracellular protein which catalyzes dismutation of superoxide to oxygen and hydrogen peroxide. | Asthma; Bronchopulmonary Dysplasia In Prematurity; Burns; Eye Disorders; Head Injuries; Infant Respiratory Distress Syndrome; Inflammatory Disorders; Kidney Disorders; Myocardial Infarction; Ischemic Heart Disorders; Reperfusion Injury; Respiratory Disorders; Stroke; Autoimmune Disorders; Acute Lung Injury; HIV Infections Treatment; Amyotrophic Lateral Sclerosis; Respiratory Syncytial Virus; Cystitis; Radioprotection; Rheumatic Disorders |
| T1/ST2 receptor (T1/ST2 receptor) | Genbank: P14719 | T1/ST2 is a member of the IL-1R family, preferentially expressed on the surface of Th2 cells, and it plays an important role in the activation of Th2 cells. | Asthma; Allergies |
| TGF Beta 1 (Transforming growth factor-beta) | LocusID: 7040 NP_000651 XP_008912 | Regulates cell proliferation, differentiation, and apoptosis in numerous cell types. | Alzheimer's Disease; Atherosclerosis; Cancer; Skin Disorders; Systemic Lupus Erythematosus; Transplant Rejection |
| TGF Beta 2 (Transforming Growth Factor Beta 2) | LocusID: 7042 NP_003229 XP_001754 | Transforming growth factor-beta 2 (TGF-beta 2) has been found to inhibit inducible nitric oxide synthase (iNOS) gene transcription, esp. in interleukin-1-beta (IL1-beta) stimulated rat smooth muscle cells, and at a dose which does not inhibit constitutive NOS. | Chronic Wounds; Mucositis; Age-Related Macular Degeneration; Autoimmune Disorders; Cancer; Diabetic Foot Ulcer; Eye Disorders; Multiple Sclerosis; Postmenopausal Osteoporosis; Rheumatoid Arthritis; Skin Disorders; Transplant Rejection; |
| TGF Beta 3 (Transforming Growth Factor Beta 3) | LocusID: 7043 NP_003230 XP_007417 | Transforming growth factor-beta 3 is a cytokine which transmits mitogenic signals through transmembrane serine/threonine kinases. TGF beta 3 is required for normal development of the lung and palate. | Chronic Wounds; Oral Mucositis; Radioprotection |
| Thrombopoietin | LocusID: 7066 NP_000451 XP_002815 | Thrombopoietin binds to the c-Mpl receptor and regulates megakaryocyte development. | Thromocytopenia; Chemoprotection |
| Tie-2 (Tek; tunica interna endothelial-cell kinase) | LocusID: 7010 NP_000450 XP_005480 | Tie-2/Tek, the angiopoietin receptor tyrosine kinase, mediates intracellular signalling and angiogenic responses following stimulation by angiopoietin. | Cancer |
| Tissue Factor Pathway Inhibitor | LocusID: 7035 NP_006278 XP_002672 | Tissue factor pathway inhibitor is a lipoprotein-associated coagulation inhibitor. It is a Kunitz-type protease inhibitor that inhibits fibrin clot formation. | Sepsis; Coagulation Disorders; Reperfusion Injury; Atherosclerosis; |
| TNFAlpha | LocusID: 7124 NP_000585 XP_011402 | TNF plays a central role in the pathophysiology of sepsis. High levels of TNF alpha correlates with increased disease severity in severe bacterial infection and malaria TNF alpha signaling may lead to activation of NF kappa B and induction of apoptosis. | Cancer; CNS Cancer; Lymphoma; Skin Cancer; Urogenital Cancer; |
| TNF binding protein | NP_001056 | TNF binding protein inhibits activation of the TNF-receptor by competing for TNF binding. | Rheumatoid Arthritis; Cardiac Reperfusion Injury; Autoimmune Disorders; Crohn's Disease; Malaria; Reperfusion Injury; Septic Shock; |
| Soluble TNF Receptor | CAS-185243-69-0 LocusID: 7132 LocusID: 7133 NP_001056 NP_001057 XP_006950 XP_001743 | Tumor Necrosis Factor receptor mediates proinflammatory cellular responses in response to TNF stimulation. | Rheumatoid Arthritis; Cachexia; Heart Failure; HIV 1 Infections; Juvenile Rheumatoid Arthritis; Psoriasis; Psoriatic Arthritis; Septic Shock; Transplant Rejection; Allergic Asthma |
| t-PA | CAS-105857-23-6 CAS-171870-23-8 CAS-156616-23-8 CAS-151912-42-4 CAS-133652-38-7 CAS-191588-94-0 LocusID: 5327 NP_000921 NP_000922 XP_005024 | Tissue-type plasminogen activator; serine protease that converts inactive plasminogen to plasmin | Embolism; Myocardial Infarction; Stroke; Thrombosis; Congestive Heart Failure; Ischemic Heart Disorders; Coronary Restenosis |
| Trophoblast interferon (interferon tau; IFN-tau; Trophoblastin) | Genbank: A53746 | Involved in placental cell growth and differentiation, as well protecting the fetus in viral environments | HIV Infections Treatment; Multiple Sclerosis |
| Troponin 1 | LocusID: 7135 NP_003272 XP_001918 LocusID: 7136 | Troponin I is a regulatory protein which prevents actin and myosin interaction in resting muscle tissue. Troponin I an inhibitory subunit of troponin. | Cancer Metasteses; Diabetic Retinopathy; Eye Disorders; Macular Degeneration; Solid Tumors |
| Urate oxidase | LocusID: 7377 Genbank: S94095 | Urate oxidase is an enzyme which catalyzes the oxidation of uric acid. | Chemoprotection; Prevention And Treatment Of Chemotherapy-Related Uricemia; Gout |
| Urokinase (Pro-urokinase) | CAS-9039-53-6 LocusID: 5328 NP_002649 XP_011861 | Urokinase acts as a plasminogen activator involved in blood clotting regulation. It is a serine protease that cleaves plasminogen to form plasmin. | Catheter Clearance; Coronary Restenosis; Diabetic Retinopathy; Myocardial Infarction; Thrombosis; Vitreous Haemorrhage; Peripheral Vascular Disorders; Stroke |
| VEGF-1 (VEGF-121) | LocusID: 7422 LocusID: 7423 LocusID: 7424 NP_003367 NP_003368 NP_005420 XP_004512 XP_006539 XP_003456 | VEGF-1 is a growth factor which induces endothelial cell proliferation and vascular permeability. | Cardiovascular Disease; Vascular Disorders; Fracture Treatment; |

The presented lists of endogenous target compounds is by no means exhaustive. Where a ligand binds to two epitopes (on the same or different antigens), the antigen(s) may be selected from this list. In particular embodiments, the ligand comprises a dAb that binds TNFR1 and a second dAb or epitope binding domain that binds any one of the these antigens. In such embodiments, the multispecific ligand can comprise any combination of immunoglobulin variable domains (e.g., V_{H}V_{H}, V_{H}V_{L}, V_{L}V_{L}).

### Ligands and dAb Monomers that Bind Serum Albumin

The invention provides a ligand or dAb monomer (*e.g*., dual specific ligand comprising such a dAb) that binds to serum albumin (SA) with a K_{d} of 1nM to 500 µM (i.e., x 10⁻⁹ to 5 x 10⁻⁴), preferably 100 nM to 10 µM. Preferably, for a dual specific ligand comprising a first anti-SA dAb and a second dAb to another target, the affinity (eg K_{d} and/or K_{off} as measured by surface plasmon resonance, eg using BiaCore) of the second dAb for its target is from 1 to 100000 times (preferably 100 to 100000, more preferably 1000 to 100000, or 10000 to 100000 times) the affinity of the first dAb for SA. For example, the first dAb binds SA with an affinity of approximately 10 µM, while the second dAb binds its target with an affinity of 100 pM. Preferably, the serum albumin is human serum albumin (HSA). In one embodiment, the first dAb (or a dAb monomer) binds SA (eg, HSA) with a K_{d} of approximately 50, preferably 70, and more preferably 100, 150 or 200 nM.

In certain embodiments, the dAb monomer that binds SA resists aggregation, unfolds reversibly and/or comprises a framework region as described above for dAb monomers that bind TNFR1.

### Ligand Formats

Ligands and dAb monomers can be formatted as mono or multispecific antibodies or antibody fragments or into mono or multispecific non-antibody structures. Suitable formats include, any suitable polypeptide structure in which an antibody variable domain or one or more of the CDRs thereof can be incorporated so as to confer binding specificity for antigen on the structure. A variety of suitable antibody formats are known in the art, such as, IgG-like formats, chimeric antibodies, humanized antibodies, human antibodies, single chain antibodies, bispecific antibodies, antibody heavy chains, antibody light chains, homodimers and heterodimers of antibody heavy chains and/or light chains, antigen-binding fragments of any of the foregoing (*e.g.,* a Fv fragment (*e.g*., single chain Fv (scFv), a disulfide bonded Fv), a Fab fragment, a Fab' fragment, a F(ab')₂ fragment), a single variable domain (*e.g*., V_{H}, V_{L}, V_{HH}), a dAb, and modified versions of any of the foregoing (*e.g*., modified by the covalent attachment of polyalkylene glycol (e.g., polyethylene glycol, polypropylene glycol, polybutylene glycol) or other suitable polymer). See, PCT/GB03/002804, filed June 30, 2003, which designated the United States, (WO 2004/081026) regarding PEGylated of single variable domains and dAbs, suitable methods for preparing same, increased *in vivo* half life of the PEGylated single variable domains and dAb monomers and multimers, suitable PEGS, preferred hydrodynamic sizes of PEGs, and preferred hydrodynamic sizes of PEGylated single variable domains and dAb monomers and multimers. The entire teaching of PCT/GB03/002804 (WO 2004/081026), including the portions referred to above, are incorporated herein by reference.

The ligand can be formatted as a dimer, trimer or polymer of the a desired dAb monomers, for example using a suitable linker such as (Gly₄Ser)ₙ, where n = from 1 to 8, e.g., 2, 3, 4, 5,6 or 7. If desired, ligands, including dAb monomers, dimers and trimers, can be linked to an antibody Fc region, comprising one or both of C_{H}2 and C_{H}3 domains, and optionally a hinge region. For example, vectors encoding ligands linked as a single nucleotide sequence to an Fc region may be used to prepare such polypeptides.

Ligands and dAb monomers can also be combined and/or formatted into non-antibody multi-ligand structures to form multivalent complexes, which bind target molecules with the same antigen, thereby providing superior avidity. For example natural bacterial receptors such as SpA can been used as scaffolds for the grafting of CDRs to generate ligands which bind specifically to one or more epitopes. Details of this procedure are described in US 5,831,012. Other suitable scaffolds include those based on fibronectin and affibodies. Details of suitable procedures are described in WO 98/58965. Other suitable scaffolds include lipocallin and CTLA4, as described in van den Beuken et al., J. Mol. Biol. 310:591-601 (2001), and scaffolds such as those described in WO 00/69907 (Medical Research Council), which are based for example on the ring structure of bacterial GroEL or other chaperone polypeptides. Protein scaffolds may be combined; for example, CDRs may be grafted on to a CTLA4 scaffold and used together with immunoglobulin V_{H} or V_{L} domains to form a ligand. Likewise, fibronectin, lipocallin and other scaffolds may be combined

A variety of suitable methods for preparing any desired format are known in the art. For example, antibody chains and formats (e.g., IgG-like formats, chimeric antibodies, humanized antibodies, human antibodies, single chain antibodies, bispecific antibodies, antibody heavy chains, antibody light chains, homodimers and heterodimers of antibody heavy chains and/or light chains) can be prepared by expression of suitable expression constructs and/or culture of suitable cells (e.g., hybridomas, heterohybridomas, recombinant host cells containing recombinant constructs encoding the format). Further, formats such as antigen-binding fragments of antibodies or antibody chains (*e.g.,* a Fv fragment (*e.g*., single chain Fv (scFv), a disulfide bonded Fv), a Fab fragment, a Fab' fragment, a F(ab')₂ fragment), can be prepared by expression of suitable expression constructs or by enzymatic digestion of antibodies, for example using papain or pepsin.

The ligand can be formatted as a dual specific ligand or a multispecific ligand, for example as described in WO 03/002609, the entire teachings of which are incorporated herein by reference. The dual specific ligands comprise immunoglobulin single variable domains that have different binding specificities. Such dual specific ligands can comprise combinations of heavy and light chain domains. For example, the dual specific ligand may comprise a V_{H} domain and a V_{L} domain, which may be linked together in the form of an scFv (e.g., using a suitable linker such as Gly₄Ser), or formatted into a bispecific antibody or antigen-binding fragment thereof (e.g. F(ab')₂ fragment). The dual specific ligands do not comprise complementary V_{H}/V_{L} pairs which form a conventional two chain antibody antigen-binding site that binds antigen or epitope co-operatively. Instead, the dual format ligands comprise a V_{H}/V_{L} complementary pair, wherein the V domains have different binding specificities.

In addition, the dual specific ligands may comprise one or more C_{H} or C_{L} domains if desired. A hinge region domain may also be included if desired. Such combinations of domains may, for example, mimic natural antibodies, such as IgG or IgM, or fragments thereof, such as Fv, scFv, Fab or F(ab')₂ molecules. Other structures, such as a single arm of an IgG molecule comprising V_{H}, V_{L}, C_{H}1 and C_{L} domains, are envisaged. Preferably, the dual specific ligand of the invention comprises only two variable domains although several such ligands may be incorporated together into the same protein, for example two such ligands can be incorporated into an IgG or a multimeric immunoglobulin, such as IgM. Alternatively, in another embodiment a plurality of dual specific ligands are combined to form a multimer. For example, two different dual specific ligands are combined to create a tetra-specific molecule. It will be appreciated by one skilled in the art that the light and heavy variable regions of a dual-specific ligand produced according to the method of the present invention may be on the same polypeptide chain, or alternatively, on different polypeptide chains. In the case that the variable regions are on different polypeptide chains, then they may be linked via a linker, generally a flexible linker (such as a polypeptide chain), a chemical linking group, or any other method known in the art.

The multispecific ligand possesses more than one epitope binding specificity. Generally, the multi-specific ligand comprises two or more epitope binding domains, such dAbs or non-antibody protein domain comprising a binding site for an epitope, e.g., an affibody, an SpA domain, an LDL receptor class A domain, an EGF domain, an avimer. Multispecific ligands can be formatted further as described herein.

In some embodiments, the ligand is an IgG-like format. Such formats have the conventional four chain structure of an IgG molecule (2 heavy chains and two light chains), in which one or more of the variable regions (V_{H} and or V_{L}) have been replaced with a dAb or single variable domain of a desired specificity. Preferably, each of the variable regions (2 V_{H} regions and 2 V_{L} regions) is replaced with a dAb or single variable domain. The dAb(s) or single variable domain(s) that are included in an IgG-like format can have the same specificity or different specificities. In some embodiments, the IgG-like format is tetravalent and can have one, two, three or four specificities. For example, the IgG-like format can be monospecific and comprises 4 dAbs that have the same specificity; bispecific and comprises 3 dAbs that have the same specificity and another dAb that has a different specificity; bispecific and comprise two dAbs that have the same specificity and two dAbs that have a common but different specificity; trispecific and comprises first and second dAbs that have the same specificity, a third dAbs with a different specificity and a fourth dAb with a different specificity from the first, second and third dAbs; or tetraspecific and comprise four dAbs that each have a different specificity. Antigen-binding fragments of IgG-like formats (e.g., Fab, F(ab')₂, Fab', Fv, scfᵥ) can be prepared. Preferably, the IgG-like formats or antigen-binding fragments thereof do not crosslink TNF1.

### Half-life Extended Formats

Ligands can be formatted to extend *in vivo* serum half life. Increased *in vivo* half-life is useful in *in vivo* applications of immunoglobulins, especially antibodies and most especially antibody fragments of small size such as dAbs. Such fragments (Fvs, disulphide bonded Fvs, Fabs, scFvs, dAbs) are rapidly cleared from the body, which can severely limit clinical applications.

An ligand (e.g., a dAb monomer) can be formatted to have a larger hydrodynamic size, for example, by attachment of a polyalkyleneglycol group (e.g. polyethyleneglycol (PEG) group, polypropyleneglycol group), serum albumin, transferrin, transferrin receptor or at least the transferrin-binding portion thereof, an antibody Fc region, or by conjugation to an antibody domain. In some embodiments, the ligand is PEGylated. Preferably the PEGylated ligand binds an endogenous target compound with substantially the same affinity as the same ligand that is not PEGylated. For example, the ligand can be a PEGylated dAb monomer that binds an endogenous target compound, wherein the PEGylated dAb monomer binds said endogenous target compound with an affinity that differs from the affinity of dAb in unPEGylated form by no more than a factor of about 1000, preferably no more than a factor of about 100, more preferably no more than a factor of about 10, or with affinity substantially unchanged affinity relative to the unPEGylated form.

Small ligands, such as a dAb monomer, can be formatted as a larger antigen-binding fragment of an antibody or as an antibody (e.g., formatted as a Fab, Fab', F(ab)₂, F(ab')₂, IgG, scFv). The hydrodynamic size of an antagonist (e.g., ligand, dAb monomer) and its serum half-life can also be increased by conjugating or linking the antagonist to a binding domain (e.g., antibody or antibody fragment) that binds an antigen or epitope that increases half-live *in vivo,* as described herein. For example, the ligand (e.g., dAb monomer) can be conjugated or linked to an anti-serum albumin or anti-neonatal Fc receptor antibody or antibody fragment, eg an anti-SA or anti-neonatal Fc receptor dAb, Fab, Fab' or scFv, or to an anti-SA affibody or anti-neonatal Fc receptor affibody.

Hydrodynamic size of the ligands (e.g., dAb monomers and multimers) of the invention may be determined using methods which are well known in the art. For example, gel filtration chromatography may be used to determine the hydrodynamic size of a ligand. Suitable gel filtration matrices for determining the hydrodynamic sizes of ligands, such as cross-linked agarose matrices, are well known and readily available.

The size of a ligand format (e.g., the size of a PEG moiety attached to a binding moiety that has a binding site for an endogenous target compound, can be varied depending on the desired application. For example, where ligand is intended to leave the circulation and enter into peripheral tissues, it is desirable to keep the hydrodynamic size of the ligand low to facilitate extravazation from the blood stream. Alternatively, where it is desired to have the ligand remain in the systemic circulation for a longer period of time the size of the ligand can be increased, for example by formatting an and Ig like protein or by addition of a 30 to 60 kDa PEG moiety.

Examples of suitable albumin, albumin fragments or albumin variants for use in ligand according to the invention are described in WO 2005/077042A2, which is incorporated herein by reference in its entirety. In particular, the following albumin, albumin fragments or albumin variants can be used in the present invention:
- SEQ ID NO: (as disclosed in WO 2005/077042A2, this sequence being explicitly incorporated into the present disclosure by reference);
- Albumin fragment or variant comprising or consisting of amino acids 1-387 of SEQ ID NO: in WO 2005/077042A2;
- Albumin, or fragment or variant thereof, comprising an amino acid sequence selected from the group consisting of: (a) amino acids 54 to 61 of SEQ ID NO:1 in WO 2005/077042A2; (b) amino acids 76 to 89 of SEQ ID NO: in WO 2005/077042A2; (c) amino acids 92 to 100 of SEQ ID NO: in WO 2005/077042A2; (d) amino acids 170 to 176 of SEQ ID NO:1 in WO 2005/077042A2; (e) amino acids 247 to 252 of SEQ ID NO:1 in WO 2005/077042A2; (f) amino acids 266 to 277 of SEQ ID NO: 1 in WO 2005/077042A2; (g) amino acids 280 to 288 of SEQ ID NO: 1 in WO 2005/077042A2; (h) amino acids 362 to 368 of SEQ ID NO: in WO 2005/077042A2; (i) amino acids 439 to 447 of SEQ ID NO:1 in WO 2005/077042A2 (j) amino acids 462 to 475 of SEQ ID NO:1 in WO 2005/077042A2; (k) amino acids 478 to 486 of SEQ ID NO:1 in WO 2005/077042A2; and (1) amino acids 560 to 566 of SEQ ID NO:1 in WO 2005/077042A2.

Further examples of suitable albumin, fragments and analogs for use in a ligand according to the invention are described in WO 03/076567A2, which is incorporated herein by reference in its entirety. In particular, the following albumin, fragments or variants can be used in the present invention:
- Human serum albumin as described in WO 03/076567A2, eg, in figure 3 (this sequence information being explicitly incorporated into the present disclosure by reference);
- Human serum albumin (HA) consisting of a single non-glycosylated polypeptide chain of 585 amino acids with a formula molecular weight of 66,500 (See, Meloun, et al., FEBS Letters 58:136 (1975); Behrens, et al., Fed. Proc. 34:591 (1975); Lawn, et al., Nucleic Acids Research 9:6102-6114 (1981); Minghetti, et al., J. Biol. Chem. 261:6747 (1986));
- A polymorphic variant or analog or fragment of albumin as described in Weitkamp, et al., Ann. Hum. Genet. 37:219 (1973);
- An albumin fragment or variant as described in EP 322094, eg, HA(1-373., HA(1-388), HA(1-389), HA(1-369), and HA(1-419) and fragments between 1-369 and 1-419;
- An albumin fragment or variant as described in EP 399666, eg, HA(1-177) and HA(1-200) and fragments between HA(1-X), where X is any number from 178 to 199.

Where a (one or more) half-life extending moiety (eg, albumin, transferrin and fragments and analogues thereof) is used in the ligand of the invention, it can be conjugated using any suitable method, such as, by direct fusion to the binding moiety that has a binding site for an endogenous target compound, for example by using a single nucleotide construct that encodes a fusion protein, wherein the fusion protein is encoded as a single polypeptide chain with the half-life extending moiety located N- or C-terminally to the TNFR1 binding moiety. Alternatively, conjugation can be achieved by using a peptide linker between moieties, eg, a peptide linker as described in WO 03/076567A2 or WO 2004/003019 (these linker disclosures being incorporated by reference in the present disclosure to provide examples for use in the present invention).

Typically, a polypeptide that enhances serum half-life *in vivo* is a polypeptide which occurs naturally *in vivo* and which resists degradation or removal by endogenous mechanisms which remove unwanted material from the organism (*e.g*., human). For example, a polypeptide that enhances serum half-life *in vivo* can be selected from proteins from the extracellular matrix, proteins found in blood, proteins found at the blood brain barrier or in neural tissue, proteins localized to the kidney, liver, lung, heart, skin or bone, stress proteins, disease-specific proteins, or proteins involved in Fc transport.

Suitable polypeptides that enhance serum half-life *in vivo* include, for example, transferrin receptor specific ligand-neuropharmaceutical agent fusion proteins (see U.S. Patent No. 5,977,307, the teachings of which are incorporated herein by reference), brain capillary endothelial cell receptor, transferrin, transferrin receptor (*e.g*., soluble transferrin receptor), insulin, insulin-like growth factor 1 (IGF 1) receptor, insulin-like growth factor 2 (IGF 2) receptor, insulin receptor, blood coagulation factor X, α1-antitrypsin and HNF 1α. Suitable polypeptides that enhance serum half-life also include alpha-1 glycoprotein (orosomucoid; AAG), alpha-1 antichymotrypsin (ACT), alpha-1 microglobulin (protein HC; AIM), antithrombin III (AT III), apolipoprotein A-1 (Apo A-1), apolipoprotein B (Apo B), ceruloplasmin (Cp), complement component C3 (C3), complement component C4 (C4), C1 esterase inhibitor (C1 INH), C-reactive protein (CRP), ferritin (FER), hemopexin (HPX), lipoprotein(a) (Lp(a)), mannose-binding protein (MBP), myoglobin (Myo), prealbumin (transthyretin; PAL), retinol-binding protein (RBP), and rheumatoid factor (RF).

Suitable proteins from the extracellular matrix include, for example, collagens, laminins, integrins and fibronectin. Collagens are the major proteins of the extracellular matrix. About 15 types of collagen molecules are currently known, found in different parts of the body, e.g. type I collagen (accounting for 90% of body collagen) found in bone, skin, tendon, ligaments, cornea, internal organs or type II collagen found in cartilage, vertebral disc, notochord, and vitreous humor of the eye.

Suitable proteins from the blood include, for example, plasma proteins (*e.g*., fibrin, α-2 macroglobulin, serum albumin, fibrinogen (*e.g*., fibrinogen A, fibrinogen B), serum amyloid protein A, haptoglobin, profilin, ubiquitin, uteroglobulin and β-2-microglobulin), enzymes and enzyme inhibitors (*e.g*., plasminogen, lysozyme, cystatin C, alpha-1-antitrypsin and pancreatic trypsin inhibitor), proteins of the immune system, such as immunoglobulin proteins (*e.g*., IgA, IgD, IgE, IgG, IgM, immunoglobulin light chains (kappa/lambda)), transport proteins (*e.g*., retinol binding protein, α-1 microglobulin), defensins (*e.g*., betadefensin 1, neutrophil defensin 1, neutrophil defensin 2 and neutrophil defensin 3) and the like.

Suitable proteins found at the blood brain barrier or in neural tissue include, for example, melanocortin receptor, myelin, ascorbate transporter and the like.

Suitable polypeptides that enhances serum half-life *in vivo* also include proteins localized to the kidney (*e.g*., polycystin, type IV collagen, organic anion transporter K1, Heymann's antigen), proteins localized to the liver (*e.g*., alcohol dehydrogenase, G250), proteins localized to the lung (*e.g*., secretory component, which binds IgA), proteins localized to the heart (*e.g*., HSP 27, which is associated with dilated cardiomyopathy), proteins localized to the skin (*e.g*., keratin), bone specific proteins such as morphogenic proteins (BMPs), which are a subset of the transforming growth factor P superfamily of proteins that demonstrate osteogenic activity (*e.g*., BMP-2, BMP-4, BMP-5, BMP-6, BMP-7, BMP-8), tumor specific proteins (*e.g*., trophoblast antigen, herceptin receptor, oestrogen receptor, cathepsins (*e.g*., cathepsin B, which can be found in liver and spleen)).

Suitable disease-specific proteins include, for example, antigens expressed only on activated T-cells, including LAG-3 (lymphocyte activation gene), osteoprotegerin ligand (OPGL; see Nature 402, 304-309 (1999)), OX40 (a member of the TNF receptor family, expressed on activated T cells and specifically up-regulated in human T cell leukemia virus type-I (HTLV-I)-producing cells; see Immunol. 165 (1):263-70 (2000)). Suitable disease-specific proteins also include, for example, metalloproteases (associated with arthritis/cancers) including CG6512 Drosophila, human paraplegin, human FtsH, human AFG3L2, murine ftsH; and angiogenic growth factors, including acidic fibroblast growth factor (FGF-1), basic fibroblast growth factor (FGF-2), vascular endothelial growth factor/vascular permeability factor (VEGF/VPF), transforming growth factor-α (TGF α), tumor necrosis factor-alpha (TNF-α), angiogenin, interleukin-3 (IL-3), interleukin-8 (IL-8), platelet-derived endothelial growth factor (PD-ECGF), placental growth factor (P1GF), midkine platelet-derived growth factor-BB (PDGF), and fractalkine.

Suitable polypeptides that enhance serum half-life *in vivo* also include stress proteins such as heat shock proteins (HSPs). HSPs are normally found intracellularly. When they are found extracellularly, it is an indicator that a cell has died and spilled out its contents. This unprogrammed cell death (necrosis) occurs when as a result of trauma, disease or injury, extracellular HSPs trigger a response from the immune system. Binding to extracellular HSP can result in localizing the compositions of the invention to a disease site.

Suitable proteins involved in Fc transport include, for example, Brambell receptor (also known as FcRB). This Fc receptor has two functions, both of which are potentially useful for delivery. The functions are (1) transport of IgG from mother to child across the placenta (2) protection of IgG from degradation thereby prolonging its serum half-life. It is thought that the receptor recycles IgG from endosomes. (See, Holliger et al, Nat Biotechnol 15(7):632-6 (1997).)

Methods for pharmacokinetic analysis and determination of ligand half-life will be familiar to those skilled in the art. Details may be found in Kenneth, A et al: Chemical Stability of Pharmaceuticals: A Handbook for Pharmacists and in Peters et al, Pharmacokinetc analysis: A Practical Approach (1996). Reference is also made to "Pharmacokinetics", M Gibaldi & D Perron, published by Marcel Dekker, 2nd Rev. ex edition (1982), which describes pharmacokinetic parameters such as t alpha and t beta half lives and area under the curve (AUC).

### Assays for Assessing Endogenous Target Compound Function.

The following assays, suitable variations thereof and other suitable assays can be used to assess activity of endogenous target compounds, and to assess the whether a ligand substantially inhibits the activity of an endogenous target compound to which it binds.

ABC-1 function may be assayed by measuring apolipoprotein-mediated lipid efflux from cultured cells (J Clin Invest 1999 Oct; 104(8): R25-31). aFGF1-pseudomonas exotoxin chimera function may be assayed in vitro using a cytotoxicity assay (Proc Natl Acad Sci USA 1989 Jun; 86(11): 4215-4219). Inhibin A activity may be assayed *in vitro* by measuring its differentiation-inducing activity toward mouse Friend erythroleukemia (MEL) cells and human K-562 cells (Proc Natl Acad Sci USA 1988 Apr; 85(8): 2434-2438); or its suppression of the secretion of pituitary follicle-stimulating hormone (Biol Reprod 2000 Sep; 63(3): 865-871). Inhibin beta C activity may be assayed *in vitro* by measuring its differentiation-inducing activity toward mouse Friend erythroleukemia (MEL) cells and human K-562 cells (Proc Natl Acad Sci USA 1988 Apr; 85(8): 2434-2438); or its suppression of the secretion of pituitary follicle-stimulating hormone (Biol Reprod 2000 Sep; 63(3): 865-871). Adenosine deaminase activity may be assayed *in vitro* by measuring purine catabolism (Mol Cell Biol 1985 Apr; 5(4): 762-767). Adiposin function may be assayed *in vitro* by measuring cAMP accumulation in adiposin stimulated mouse melanoma cells. (Hum Mol Genet 1995 Feb; 4(2): 223-230).

ASP function may be assayed *in vitro* by measuring inhibition of alpha-MSH-stimulated cAMP accumulation in mouse melanoma cells (Hum Mol Genet 1995 Feb; 4(2): 223-230). Myelin may be assayed *in vitro* by measurement of its phosphorylation by MAP kinase (J Neurochem 1999 Sep; 73(3): 1090-1097); or its effect on synaptic transmission between neurons (Eur Neurol. 1988; 28(2): 57-63). Myelin Basic Protein may be assayed in vitro by measurement of its phosphorylation by MAP kinase (J Neurochem 1999 Sep; 73(3): 1090-1097); or its effect on synaptic transmission between neurons (Eur Neurol. 1988; 28(2): 57-63). Collagen function may be measured using an *in vitro* collagen fibril stability assay (Cell Mol Life Sci 2000 May; 57(5): 859-863); or an *in vitro* cell adhesion assay (J Cell Biochem Oct. 1, 1997; 67(1): 75-83). Alpha glucosidase can be assayed by hydrolysis of chromogenic artificial substrate p-Nitrophenyl a-D-glucoside. Bergmeyer, H. U. (ed) Methods of Enzymatic Analysis, Second English Edition, 1, 459 (1974). Alpha-galactosidase can be assayed by hydrolysis of chromogenic artificial substrate p-Nitrophenyl a- D-galactosidase to p-Nitrophenol and D-galactose- Rietra et al., (1975) "Properties of the residual alpha-galactosidase activity in the tissues of a Fabry hemizygote". Clin Chim Acta; 62(3): 401-13. Alpha-L iduronidase can be assayed by hydrolysis of the substrate 4-methylumbelliferyl alpha-L-iduronide is followed in a fluorometric assay (Hopwood et al. (1979), Clin Chim Acta.; 92: 257-65); other assays are found in Thompson (1978) "Substrates for the assay of alpha-L-iduronidase". Clin Chim Acta; 89(3): 435-46

Angiopoeitin 1 activity may be assayed *in vitro* using a capillary sprouting assay. (Curr Biol Apr. 23, 1998; 8(9): 529-532). Angiopoeitin 2 activity may be assayed *in vitro* using a capillary sprouting assay (Curr Biol Apr. 23, 1998; 8(9): 529-532). Angiostatin can be assayed in an endothelial proliferation assay (Kringle domains of Human Angiostatin. Cao et al (1996) J. Biol. Chem. 271 29461-29467. ADMP activity may be assayed *in vitro* by measuring its ability to downregulate the dorsalizing factors noggin, goosecoid or follistatin. (Development 1995 Dec;121(12): 4293-4301). TRAIL can be assayed in an apoptosis assay (TRAIL-R2: a novel apoptosis-mediating receptor for TRAIL, Walczak et al. (1996) EMBOJ 16: 5386-5397). Arresten function may be assayed *in vitro* by measuring its ability to inhibit endothelial cell proliferation, migration, tube formation, and Matrigel neovascularization (Cancer Res May 1, 2000;60(9): 2520-6). Arylsulfatase B activity may be assayed by *in vitro* measurement of hydrolysis of sulfates ofN-Acetyl-D-galactosamine (J Biol Chem Feb. 25, 1990;265(6): 3374-3381). Asparaginase activity may be assayed *in vitro* using an asparaginase enzymatic assay (Anal Biochem Apr. 10, 2000;280(1): 42-45). rBPI activity may be assayed *in vitro* using an antibacterial assay (J Biol Chem Nov. 5, 1987;262(31): 14891-14894).

BDNF can be assayed in a neuronal growth and synaptic activity assay (BDNF enhances neuronal growth and synaptic activity in hippocampal cell cultures. Bartrupet al (1997) Neuroreport 1; 8(17): 3791-4 Daniels L B, Glew R H, Radin N S, Vunnam R R). B-glucocerebrosidase can be assayed using a fluorometric assay for Gaucher's disease using conduritol-beta-epoxide with liver as the source of Beta-glucosidase (Clin Chim Acta. Sep. 25, 1980; 106(2): 155-63; Johnson W G, Gal A E, Miranda A F, Pentchev P G. Diagnosis of adult Gaucher disease: use of a new chromogenic substrate, 2-hexadecanoylamino-4-nitrophenyl-beta-D-glucopyranoside, in cultured skin fibroblasts. Clin Chim Acta. Mar. 14, 1980;102(1): 91-7). BMP-2 can be assayed as described by Wang, E. A et al.:Recombinant human bone morphogenetic protein induces bone formation. Proc. Nat.Acad. Sci. 87: 2220-2224,1990. BT-SD function may beassayed *in vitro* by using a superoxide dismutase assay (Nucleic Acids Res Mar. 25, 1985; 13(6): 2017-34). BRCA1 activity may be assayed by measuring alterations in expression of p21 WAF1/CIP1 (Oncogene Jun. 11, 1998;16(23): 3069-82). BRCA2 activity may be assayed by measuring alterations in expression of p21 WAF1/CIP1 (Oncogene Jun. 11, 1998;16(23): 3069-82). The vasodilatory activity of CGRP can be assayed using the aortic ring vasodilation assay described in Pharmacol Res. 1999 Mar; 39(3): 217-20; endothelial and osteoblast cell proliferation activities can be measured *in vitro* (Eur J Pharmacol. Dec. 15, 2000; 409(3): 273-8; Proc Natl Acad Sci U.S.A. 1990 May; 87(9): 3299-303).

Calreticulin activity can be measured *in vitro* using calcium imaging assays (Cell. Sep. 8, 1995; 82(5): 765-71). CD4 function may be assayed *in vitro* by measuring gp120 binding (Viral Immunol 2000; 13(4): 547-554); or altered monocyte responses to cytokines following gp120 binding (J Immunol Oct. 15, 1998; 161(8): 4309-4317). CD40 ligand can be assayed as described by Hollenbaugh D, et al., The human T cell antigen gp39, a member of the TNF gene family, is a ligand for the CD40 receptor: expression of a soluble form of gp39 with B cell co-stimulatory activity. EMBO J. 1992 Dec; 11(12): 4313-21. Chemokine binding proteins can be assayed using receptor binding assays (J Biol Chem May 9, 1997; 272(19): 12495-12504). CNTF may be assayed in vitro using neuronal proliferation and survival assays (EMBO J. Apr. 2, 2001; 20(7) 1692-1703). Contortrostatin activity may be measured *in vitro* by measuring binding to integrins alphavbeta3 andnalphavbeta5, inhibition of platelet aggregation, and inhibition of cancer cell adhesion to fibronectin and vitronectin. (Arch Biochem Biophys Mar. 15, 2000;375(2): 278-288). The activity of CRF binding protein can be measured using a CRF binding assay (Peptides Jan. 22, 2001; 22(1): 47-56).

CTLA4 activity can be measured using a T cell activation assay (J Immunol Mar. 1, 2001; 166(5): 3143-3150). Decorin function may be measured using an *in vitro* collagen fibril stability assay (Cell Mol Life Sci 2000 May;57(5): 859-863); or an *in vitro* cell adhesion assay (J Cell Biochem Oct. 1, 1997;67(1): 75-83). Del-1 function may be assayed *in vitro* using an alphavbeta3 integrin adhesion assay. (Genes Dev Jan. 1, 1998; 12(1): 21-33). Desmoteplase can be assayed as described by Wallen, P., Biochemistry of plasminogen. In:Kline D. L., Reddy, K.N. N., eds. Fibrinolysis. Boca Raton, FL: CRC Press,1980: 1-25; Saksela, O., Rifkin, D. B., Cell-associated plasminogen activation: Regulation and physiological functions. Annu Rev Cell Biol 1988; 4: 93-126; Womack C J, Ivey F M, Gardner A W, Macko R F, Fibrinolytic response to acute exercise in patients with peripheral arterial disease. Med Sci Sports Exerc 2001 Feb; 33(2): 214-9. Dnase activity can be measured using the DNA degradation assay described in J Biochem (Tokyo). 1982 Oct; 92(4): 1297-303. Ectoapyrase activity may be assayed in vitro using an ectoapyrase assay (J Biol Chem Sep. 18, 1998; 273(38): 24814-24821). EGF can be assayed using a cell growth assay (J Biol Chem Mar. 31, 1995; 270(13): 7495-500) EMAP II activity may be assayed in *vitro* using a capillary sprouting assay (Curr Biol Apr. 23, 1998; 8(9): 529-532). FGF-1 can be assayed using a cell proliferation assay: Cell, vol.50, no.5, pp.729-737 (Aug. 1987). Proc Natl Acad Sci U.S.A, vol.86, no.3, pp.802-806 (1989). FGF-2 can be assayed using a proliferation assay using NR6R-3T3 cells (Rizzino 1988 Cancer Res. 48: 4266). Fibrolase activity may be assayed in vitro using a fibrinolytic assay. (Thromb Res May 15, 1994; 74(4): 355-367). FLT3 can be assayed in a proliferation assay using a Flt-3 transformed pro B-cell line (Hannum 1994 Nature 368: 643).

Follitropin activity may be assayed *in vitro* by measuring cAMP production in cells expressing the FSH receptor (J Reprod Immunol 2001 Jan; 49(1): 1-19). GDNF activity may be assayed *in vitro* by measuring increases in Ret tyrosine phosphorylation in response to GDNF treatment (Mol Cell Biol Mar. 15, 1995; (3): 1613-1619). Gelsolin activity may be assayed *in vitro* by measuring proteolysis of actin (Nature 1987 Jan 22-28; 325(6102): 362-364). GGF2 activity may be assayed *in vitro* by measuring activation of ERBB receptor tyrosine kinases in human rhabdomyosarcoma cells.(Int J Cancer Jul. 1, 2000;87(1): 29-36).

Glucagon's glucogenic activity is mediated by a high affinity glucagons receptor. Biological activity of recombinant glucagon can be assessed by direct binding assays (Science Mar. 12, 1993; 259(5101): 1614-6). HBNF activity may be assayed *in vitro* using a neurite outgrowth assay. (J Biol Chem Oct. 27, 1995; 270(43): 25992-25999). hCG receptor binding and activation can be measured to assess biological activity of recombinant hCG (J Biol Chem Oct. 5, 1993; 268(28): 20851-4). Heat shock proteins can be assayed by administration of HSP- peptide complexes in two UV-induced carcinoma models in mice (US 5,837,251). Interleukin 1 can be assayed by 1) binding to IL-1 receptors in YT-NCI or C3H/HeJ cells (Carter et al., Nature 344: 633-638, 1990); 2) induction of endothelial cell-leukocyte adhesion (Carter et al., Nature 344: 633-638, 1990); 3) proliferation assays on A375-C6 cells (Murai T et al., J. Biol. Chem. 276: 6797-6806, 2001); D10S proliferation: Orencole & Dinarello (1989) Cytokine 1, 14-20. IL-1ra can be assayed by 1) competition for IL-1 binding to IL-1 receptors in YT-NCI or C3H/HeJ cells (Carter et al., Nature 344: 633-638, 1990); 2) inhibition ofIL-1-induced endothelial cell-leukocyte adhesion (Carter et al., Nature 344: 633-638, 1990); 3) proliferation assays on A375-C6 cells, a human melanoma cell line highly susceptible to the antiproliferative action of IL-1 (Murai T et al., J. Biol. Chem. 276: 6797-6806, 2001). IL-10 can be assayed by 1) binding of IL-10 to NK cells (carson WE et al., Blood 85: 3577-3585, 1995); 2) inhibition of TNF-alpha production by macrophages (Riley J K et al., J. Biol. Chem. 274: 16513-16521, 1999); 3) inhibition of macrophage proliferation (O'Farrell A- M et al., EMBO 17: 1006-1018, 1998); MC-9 proliferation: Thompson- Snipes et al (1991) J. Exp. Med. 173, 507-510 IL-11 can be assayed in hematopoietic cell proliferation assay. "Interleukin-11 enhances human megakaryocytopoiesis in vitro." Blood. Jan. 15, 1992; 79(2): 327-31. "Synergistic effects of stem cell factor and interleukin 6 or interleukin 11 on the expansion of murine hematopoietic progenitors in liquid suspension culture." Stem Cells. 1995 Jul; 13(4): 404-13; B9-11 proliferation: Lu et al (1994) J immunol. Methods 173, 19. IL-12 can be assayed in a natural killer (NK) cell cytotoxicity assay and interferon-gamma (IFN- gamma) release assay. "Requirement for type 2 NO synthase for IL-12 signaling in innate immunity". Science 284: 951-955, 1999; KIT-225 proliferation: Hori et al (1987), Blood 70, 1069-1078.

IL18 binding protein activity may be assayed in vitro by measuring its ability to inhibit the early Th1 response (Immunity 1999 Jan; 10(1): 127-136). IL2-diphtheria toxin chimera function may be assayed in vitro using a cytotoxicity assay. (Exp Hematol 2000 Dec; 28(12): 1390-1400). IL-4 can be assayed in a cytotoxicity assay in normal T lymphocytes. (PMID: 8144944); RAMOS Augmentation of CD23 expression:Siegel & Mostowski (1990) J Immunol Methods 132, 287-295. IL-4 receptor activity can be measured by ability to inhibit IL-4 dependent proliferation of TF-1 cells (Kitamura, T et al., 1989, J. Cell. PPhysiol. 140:323). IL-8 can be assayed in an assay that monitors calcium reflux in IL8R bearing cells (Holmes et al (1991)Science 253, 1278-80). Interferon gamma can measure activity in anti-viral assay using Hela cells infected with EMC virus (Meager, A. 1987, Lymphokines and Interferons, A Practical Approach, Clemens, MJ et al., eds., IRL Press, p. 129); can measure modulation of MHC class II expression on Human colorectal carcinoma cell line COLO 205 (Gibson and Kramer, 1989, J. Immunol. Methods, 125: 105-113).

Interferon-omega activity can be measured using an *in vitro* antiviral assay (JMed Microbiol 1998 Nov; 47(11): 1015-8). IP-10 activity may beassayed *in vitro* using a rat artery smooth muscle cell chemotaxis assay. (J Biol Chem Sep. 27, 1996; 271 (39): 24286-24293). IL-3 activity may be measured *in vitro* using a haematopoietic cell differentiation assay(Science 1985; 228(4701): 810-815). KGF-1 activity may be assayed *in vitro* using an epithelial cell proliferation assay. (Proc Natl Acad Sci U.S.A. 1989 Feb; 86(3): 802-806). Kistrin activity may be assayed in vitro by assaying thrombolysis, reocclusion, and bleeding associated with administration of recombinant tissue-type plasminogen activator (rt-PA) in a canine model of coronary artery thrombosis (Circulation 1991 Mar; 83(3): 1038-1047). Kunitz protease inhibitor 1 activity may be assayed in vitro by measuring inhibitory activity toward HGF activator (J Biol Chem Mar. 7, 1997; 272(10): 6370-6376). Lactotransferrin activity can be measured using an in vitro viral inhibition assay (J Med Microbiol 1998 Nov; 47(11): 1015-8), and antimicrobial assays (J Clin Invest Sep. 1, 1998; 102(5): 874-80). Leptin can be assayed by in vivo modulation of food intake, reduction in body weight, and lowering of insulin and glucose levels in ob/ob mice, radioimmunoassay (RIA) and activation of the leptin receptor in a cell-based assay. Protein Expr Purif 1998 Dec; 14(3): 335-42. LIF activity may be measured in vitro using a haematopoietic cell differentiation assay. (Science 1985; 228(4701): 810-815). LFA-3 activity may be assayed in vitro by measuring its ability to inhibit T-cell function. (JExp Med Jul. 1, 1993;178(1): 211-222). Lys plasminogen activity can be measured using an in vitro fibrinolysis assay (J Biol Chem Dec. 25, 1992; 267(36): 26150-6).

Maspin activity can be measured using in vitro angiogenesis assays (Nat Med 2000 Feb; 6(2): 196-9). Methioninase activity may be assayed in vitro as described by Ito et al. (J Biochem (Tokyo), 1975 Nov; 78(5): 1105-1107). MTP activity may be assayed in vitro by using an apoB-containing lipoprotein secretion assay. (J Biol Chem Sep. 2, 1994; 269(35): 21951-21954). NGF activity may be assayed by measurement of CREB transcription factor activation in sympathetic neurons in culture (Science Dec. 17, 1999;286(5448): 2358-2361). Neutral endopeptidase activity may be assayed in vitro by measuring proteolysis of bombesin-,like peptides (Proc NatlmAcad Sci U.S.A. Dec. 1, 1991; 88(23): 10662-10666). NIF activity may be assayed in vitro using a polymorphonuclear leukocyte adhesion assay (Mol Pharm 1999 Nov; 56(5): 926-932). Noggin activity may be assayed by measuring TNF receptor responses to TNF stimulation of HeLa cells. (J Biol Chem Feb. 28, 1997; 272(9): 5861-5870). NT-3 activity may be assayed in vitro by measuring its ability to proliferate cultured NC progenitor cells grown in a serum-free defined medium (Proc Natl Acad Sci U.S.A Mar. 1, 1992; 89(5): 1661-1665). OP-1 activity may be assayed in vitro by measuring VEGF expression in fetal rat calvaria cells (Mol Cell Endocrinol Jul. 20, 1999;153(1-2): 113-124). Osteoprotegerin activity may be assayed in vitro using a coculture assay for osteoclastogenesis, a bone resorption assay using fetal long-bone organ culture system, a dentine resorption assay, or a fibroblast proliferation assay (FASEB J. 1998;12: 845-854). Plasma PAF acetylhydrolase activity may be determined by the method of Stafforini et al. (Stroke 1997 Dec; 28(12): 2417-20).

Patched function may be assayed in vitro by measuring binding of its ligand, Sonic hedgehog (Nature Nov. 14, 1996; 384(6605): 129-134). PDGF activity may be assayed in vitro by measuring its ability to induce tyrosine phosphorylation on the PDGF receptor (J Biol Chem May 25, 1989;264(15): 8905-8912). PEDF function may be assayed in vitro using a neurite outgrowth assay (J Biol Chem Oct. 27, 1995; 270(43): 25992-25999). Plasminogen activator inhibitor can be assayed as described by Wallen, P., Biochemistry of plasminogen. In: Kline D. L., Reddy, K. N. N., eds. Fibrinolysis. Boca Raton, FL: CRC Press, 1980: 1-25; Saksela, O., Rifkin, D. B., Cell- associated plasminogen activation: Regulation and physiological functions. Annu Rev Cell Biol 1988; 4: 93-126; Womack C J, Ivey F M, Gardner A W, Macko R F, Fibrinolytic response to acute exercise in patients with peripheral arterial disease. Med Sci Sports Exerc 2001 Feb; 33(2): 214-9. PGP activity may be assayed in vitro by measuring agonism of fetal liver tyrosine kinase- 3 and granulocyte colony- stimulating factor (Exp Hematol 2001 Jan; 29(1): 41-50). PMP activity may be assayed in vitro by measuring the ability to induce megakaryocytopoiesis in CD43+ cells purified from bone marrow, mobilized peripheral blood progenitor cells, or umbilical cord (J Hematother 1999 Apr; 8(2): 199-208). Prosaptide efficacy can be measured through multiple dose regimens using diabetic rats (Anesthesiology 2000 Nov; 93(5): 1271-8).

Ranpirnase activity in vitro may be assayed by using ribonuclease and cytotoxic activity assays (J Mol Biol Apr. 19, 1996; 257(5): 992-1007). Relaxin activity may be assayed in vitro using an inhibition of KCl-induced rat uterine contractions in vitro assay (Can J Physiol Pharmacol 1981 May; 59(5): 507-12). Retinal S-Antigen activity may be assayed in vitro by measuring activity of cyclic GMP- gated channels in rod photoreceptor cells (J Biol Chem Nov. 25, 1994; 269(47): 29765-29770). RhLH activity may be assayed in vitro by measuring Fura-2 fluorescence in isolated porcine thecal cells in response to RhLH stimulation (Endocrinology 2000 Jun; 141 (6): 2220-2228). Saruplase activity may be measured in vitro using a plasminogen cleavage assay (J Biol Chem Jan. 18, 2001; epub ahead of print). Complement receptor type 1 may be assayed in vitro by measuring C3b and C4b binding. (J Exp Med Nov. 1, 1988; 168(5): 1699-1717).

Sonic hedgehog function may be assayed in vitro by measuring its binding to patched (Nature 1996 Nov. 14; 384(6605): 129-134). Staphylokinase activity may be measured in vitro using a plasminogen cleavage assay (J Biol Chem Jan. 18, 2001; epub ahead of print). SCF activity may be assayed by measuring mast cell survival, proliferation, or production of pro- inflammatory cytokines (Immunol Rev 2001 Feb; 179: 57-60). Streptokinase activity may be measured in vitro using a plasminogen activation assay (J Biol Chem Jan. 18, 2001; epub ahead of print). Superoxide dismutase activity may be assayed in vitro using a superoxide dismutase assay (Nucleic Acids Res Mar. 25, 1985; 13(6): 2017-34). T1/ST2 function may be assayed in vitro using a Th2 cell activation assay (J Immunol Mar. 1, 2001; 166(5): 3143-3150). TGF beta 1 activity may be assayed in vitro by measuring induction of renal fibroblast proliferation via induction of FGF-2 (Kidney Int 2001 Feb; 59(2): 579-592). TGF Beta 2 function may be assayed in vitro by measuring inhibition of iNOS transcription (Inflamm Res 1997 Sep; 46(9): 327-331). TGF-beta 3 can be assayed for stimulation of production of prostaglandin E2 (PGE2) and bone resorption in neonatal mouse calvaria in organ culture (PNAS USA 1985 Jul; 82(13): 4535-4538), or stimulation of the synthesis of collagen, osteopontin, osteonectin, and alkaline phosphatase, and the ability to stimulate replication in osteoblast-like cells (J Biol Chem 1987;262: 2869, J Biol Chem 1988; 263: 13916, J Cell Biol 1988; 106: 915, J Cell Physiol 1987; 133: 426, Endocrinology 1987; 121: 212, Endocrinology 1986;19: 2306, and J Cell Biol 1987; 105: 457).

Thrombopoietin (TPO) can be assayed to determine regulation of growth and differentiation ofmegakaryocytes (Mol Cell Biol 2001 Apr; 21(8): 2659-2670; Exp Hematol 2001 Jan; 29(1): 51-58; Leukemia 2000 Oct; 14(10): 1751-1756). Tie-2/Tek function may be assayed by measuring its phosphorylation in response to stimulation by angiopoietin (Int Immunol 1998 Aug; 10(8): 1217-1227). Tissue factor pathway inhibitor may be assayed for inactivation of factor Xa and inhibition of the VIIa-tissue factor complex of the extrinsic coagulation pathway (J Biol Chem May 5, 1998; 263(13): 6001-6004; Thromb Haemost 1998; 79(2): 306-309). TNF binding protein activity may be assayed by measuring inhibition of PIP5K activation in response to TNF stimulation of HeLa cells. (J Biol Chem Feb. 28, 1997; 272(9): 5861-5870). TNF binding protein activity may be assayed by measuring inhibition of PIP5K activation in response to TNF stimulation of HeLa cells. (J Biol Chem Feb. 28, 1997;272(9): 5861-5870). TNF receptor activity may be assayed by measuring increases of PIP5K activation in response to TNF stimulation of HeLa cells (J Biol Chem Feb. 28, 1997; 272(9): 5861-5870). t-PA can be assayed as described in Wallen, P., Biochemistry of plasminogen. In: Kline D. L., Reddy, K. N. N., eds. Fibrinolysis. Boca Raton, FL: CRC Press, 1980: 1-25; Saksela, O., Rifkin, D. B., Cell- associated plasminogen activation: Regulation and physiological functions. Annu Rev Cell Biol 1988; 4: 93-126; Womack C J, Ivey FM, Gardner A W, Macko R F, Fibrinolytic response to acute exercise in patients with peripheral arterial disease. Med Sci Sports Exerc 2001 Feb; 33(2): 214-9. IFN-tau activity may be assayed in vitro by measuring IFN-tau induced production of an acidic 70 kD protein in cultured explants of endometrium prepared from ewes on day 13 of the estrous cycle. (Mol Endocrinol 1990 Oct; 4(10): 1506-1514).

Troponin I activity may be assayed in vitro using a myofibril binding assay (J Muscle Res Cell Motil 1999 Nov; 20(8): 755-760). Urate oxidase activity may be assayed in vitro using an assay for uricase-catalyzed oxidation of uric acid (Anal Chem May 15, 1999; 71(10): 1928-1934); or by immunoassay of recombinant urate oxidase (J Pharm Sci 1996 Sep; 85(9): 955-959). Urokinase activity may be measured in vitro using a plasminogen cleavage assay. Sazonova et al. (J Biol Chem Jan. 18, 2001, electronic publication prior to print). VEGF-1 activity may beassayed in vitro using an endothelial cell proliferation assay. (Proc Natl Acad Sci U.S.A. 1989 Feb; 86(3): 802-806). Activity of Viscumin may be assayed using granulocyte and neutrophil activation assays. (Anticancer Res 1999 Jul-Aug; 19(4B): 2925-2928; and J Leukoc Biol 2000 Dec; 68(6): 845-853).

### Preparation of Immunoglobulin Based Ligands

Binding ligands (e.g., dAbs) as described herein according to the invention can be prepared according to previously established techniques, used in the field of antibody engineering, for the preparation of scFv, "phage" antibodies and other engineered antibody molecules. Techniques for the preparation of antibodies are for example described in the following reviews and the references cited therein: Winter & Milstein, (1991) Nature 349:293-299; Pluckthun (1992) Immunological Reviews 130:151-188; Wright et al., (1992) Crit. Rev. Immunol.12:125-168; Holliger, P. & Winter, G. (1993) Curr. Op. Biotechn. 4, 446-449; Carter, et al. (1995) J. Hematother. 4, 463-470; Chester, K.A. & Hawkins, R.E. (1995) Trends Biotechn. 13, 294-300; Hoogenboom, H.R. (1997) Nature Biotechnol. 15, 125-126; Fearon, D. (1997) Nature Biotechnol. 15, 618-619; Plückthun, A. & Pack, P. (1997) Immunotechnology 3, 83-105; Carter, P. & Merchant, A.M. (1997) Curr. Opin. Biotechnol. 8, 449-454; Holliger, P. & Winter, G. (1997) Cancer Immunol. Immunother. 45,128-130.

Suitable techniques employed for selection of antibody variable domains with a desired specificity employ libraries and selection procedures which are known in the art. Natural libraries (Marks et al. (1991) J. Mol. Biol., 222: 581; Vaughan et al. (1996) Nature Biotech., 14: 309) which use rearranged V genes harvested from human B cells are well known to those skilled in the art. Synthetic libraries (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97) are prepared by cloning immunoglobulin V genes, usually using PCR. Errors in the PCR process can lead to a high degree of randomisation. V_{H} and/or V_{L} libraries may be selected against target antigens or epitopes separately, in which case single domain binding is directly selected for, or together.

### Library vector systems

A variety of selection systems are known in the art which are suitable for use in the present invention. Examples of such systems are described below.

Bacteriophage lambda expression systems may be screened directly as bacteriophage plaques or as colonies of lysogens, both as previously described (Huse et al. (1989) Science, 246: 1275; Caton and Koprowski (1990) Proc. Natl. Acad. Sci. U.S.A., 87; Mullinax et al. (1990) Proc. Natl. Acad. Sci. U.S.A., 87: 8095; Persson et al. (1991) Proc. Natl. Acad. Sci. U.S.A., 88: 2432) and are of use in the invention. Whilst such expression systems can be used to screen up to 10⁶ different members of a library, they are not really suited to screening of larger numbers (greater than 10⁶ members). Of particular use in the construction of libraries are selection display systems, which enable a nucleic acid to be linked to the polypeptide it expresses. As used herein, a selection display system is a system that permits the selection, by suitable display means, of the individual members of the library by binding the generic and/or target ligands.

Selection protocols for isolating desired members of large libraries are known in the art, as typified by phage display techniques. Such systems, in which diverse peptide sequences are displayed on the surface of filamentous bacteriophage (Scott and Smith (1990) Science, 249: 386), have proven useful for creating libraries of antibody fragments (and the nucleotide sequences that encoding them) for the *in vitro* selection and amplification of specific antibody fragments that bind a target antigen (McCafferty et al., WO 92/01047). The nucleotide sequences encoding the variable regions are linked to gene fragments which encode leader signals that direct them to the periplasmic space of *E*. *coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty et al. (1990) Nature, 348: 552; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad. Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al.* (1991) supra; Barbas *et al.* (1992) supra; Hawkins and Winter (1992) J. Immunol., 22: 867; Marks et al., 1992, J. Biol. Chem., 267: 16007; Lerner et al. (1992) Science, 258: 1313, incorporated herein by reference).

One particularly advantageous approach has been the use of scFv phage-libraries (Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al.* (1990) supra; Clackson et al. (1991) Nature, 352: 624; Marks et al. (1991) J. Mol. Biol., 222: 581; Chiswell et al. (1992) Trends Biotech., 10: 80; Marks et al. (1992) J. Biol. Chem., 267). Various embodiments of scFv libraries displayed on bacteriophage coat proteins have been described. Refinements of phage display approaches are also known, for example as described in WO96/0621 and WO92/01047 (Medical Research Council *et al*.) and WO97/08320 (Morphosys), which are incorporated herein by reference.

Other systems for generating libraries of polypeptides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bach (1990) Nucleic Acids Res., 18: 3203; Beaudry and Joyce (1992) Science, 257: 635; WO92/05258 and WO92/14843). In a similar way, *in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection.

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

### Library Construction

Libraries intended for selection, may be constructed using techniques known in the art, for example as set forth above, or may be purchased from commercial sources. Libraries which are useful in the present invention are described, for example, in WO99/20749. Once a vector system is chosen and one or more nucleic acid sequences encoding polypeptides of interest are cloned into the library vector, one may generate diversity within the cloned molecules by undertaking mutagenesis prior to expression; alternatively, the encoded proteins may be expressed and selected, as described above, before mutagenesis and additional rounds of selection are performed. Mutagenesis of nucleic acid sequences encoding structurally optimised polypeptides is carried out by standard molecular methods. Of particular use is the polymerase chain reaction, or PCR, (Mullis and Faloona (1987) Methods Enzymol., 155: 335, herein incorporated by reference). PCR, which uses multiple cycles of DNA replication catalysed by a thermostable, DNA-dependent DNA polymerase to amplify the target sequence of interest, is well known in the art. The construction of various antibody libraries has been discussed in Winter et al. (1994) Ann. Rev. Immunology 12, 433-55, and references cited therein.

PCR is performed using template DNA (at least 1fg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionized water to a total volume of 25 µl. Mineral oil is overlaid and the PCR is performed using a programmable thermal cycler. The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenised, mismatch is required, at least in the first round of synthesis. The ability to optimise the stringency of primer annealing conditions is well within the knowledge of one of moderate skill in the art. An annealing temperature of between 30 °C and 72 °C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1-5 minutes, depending on the length of the amplified product). Final extension is generally for 4 minutes at 72°C, and may be followed by an indefinite (0-24 hour) step at 4°C.

### Combining Single Variable Domains

Domains useful in the invention, once selected, may be combined by a variety of methods known in the art, including covalent and non-covalent methods. Preferred methods include the use of polypeptide linkers, as described, for example, in connection with scFv molecules (Bird et al., (1988) Science 242:423-426). Discussion of suitable linkers is provided in Bird et al. Science 242, 423-426; Hudson et al, Journal Immunol Methods 231 (1999) 177-189; Hudson et al, Proc Nat Acad Sci USA 85, 5879-5883. Linkers are preferably flexible, allowing the two single domains to interact. One linker example is a (Gly₄ Ser)ₙ linker, where n=1 to 8, eg, 2, 3, 4, 5 or 7. The linkers used in diabodies, which are less flexible, may also be employed (Holliger et al., (1993) PNAS (USA) 90:6444-6448). In one embodiment, the linker employed is not an immunoglobulin hinge region.

Variable domains may be combined using methods other than linkers. For example, the use of disulphide bridges, provided through naturally-occurring or engineered cysteine residues, may be exploited to stabilise V_{H}-V_{H},V_{L}-V_{L} or V_{H}-V_{L} dimers (Reiter et al., (1994) Protein Eng. 7:697-704) or by remodelling the interface between the variable domains to improve the "fit" and thus the stability of interaction (Ridgeway et al., (1996) Protein Eng. 7:617-621; Zhu et al., (1997) Protein Science 6:781-788). Other techniques for joining or stabilising variable domains of immunoglobulins, and in particular antibody V_{H} domains, may be employed as appropriate.

### Characterisation of Ligands

The binding of a ligand (e.g., dAb monomer, dual-specific ligand) to its specific antigen(s) or epitope(s) can be tested by methods which will be familiar to those skilled in the art and include ELISA. In a preferred embodiment of the invention binding is tested using monoclonal phage ELISA. Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12, 433-55 and references cited therein.

The diversity of the selected phage monoclonal antibodies may also be assessed by gel electrophoresis of PCR products (Marks *et al.* 1991, *supra;* Nissim *et al.* 1994 *supra*), probing (Tomlinson et al., 1992) J. Mol. Biol. 227, 776) or by sequencing of the vector DNA.

### Structure of Ligands

In the case that the variable domains are selected from V-gene repertoires selected for instance using phage display technology as herein described, then these variable domains comprise a universal framework region, such that is they may be recognised by a specific generic ligand as herein defined. The use of universal frameworks, generic ligands and the like is described in WO99/20749.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair. DNA shuffling is known in the art and taught, for example, by Stemmer, 1994, Nature 370: 389-391 and U.S. Patent No. 6,297,053, both of which are incorporated herein by reference. Other methods of mutagenesis are well known to those of skill in the art.

In general, nucleic acid molecules and vector constructs required for selection, preparation and formatting ligands may be constructed and manipulated as set forth in standard laboratory manuals, such as Sambrook et al. (1989) Molecular Cloning: A Laboratory Manual, Cold Spring Harbor, USA.

The manipulation of nucleic acids useful in the present invention is typically carried out in recombinant vectors.

As used herein, vector refers to a discrete element that is used to introduce heterologous DNA into cells for the expression and/or replication thereof. Methods by which to select or construct and, subsequently, use such vectors are well known to one of ordinary skill in the art. Numerous vectors are publicly available, including bacterial plasmids, bacteriophage, artificial chromosomes and episomal vectors. Such vectors may be used for simple cloning and mutagenesis; alternatively gene expression vector is employed. A vector of use according to the invention may be selected to accommodate a polypeptide coding sequence of a desired size, typically from 0.25 kilobase (kb) to 40 kb or more in length A suitable host cell is transformed with the vector after *in vitro* cloning manipulations. Each vector contains various functional components, which generally include a cloning (or "polylinker") site, an origin of replication and at least one selectable marker gene. If given vector is an expression vector, it additionally possesses one or more of the following: enhancer element, promoter, transcription termination and signal sequences, each positioned in the vicinity of the cloning site, such that they are operatively linked to the gene encoding a ligand according to the invention.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available in the growth media.

Since the replication of vectors encoding a ligand according to the present invention is most conveniently performed in *E. coli,* an *E. coli*-selectable marker, for example, the β-lactamase gene that confers resistance to the antibiotic ampicillin, is of use. These can be obtained from *E*. *coli* plasmids, such as pBR322 or a pUC plasmid such as pUC18 or pUC19.

Expression vectors usually contain a promoter that is recognised by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Delgarno sequence operably linked to the coding sequence.

The preferred vectors are expression vectors that enables the expression of a nucleotide sequence corresponding to a polypeptide library member. Thus, selection with the first and/or second antigen or epitope can be performed by separate propagation and expression of a single clone expressing the polypeptide library member or by use of any selection display system. As described above, the preferred selection display system is bacteriophage display. Thus, phage or phagemid vectors may be used, eg pIT1 or pIT2. Leader sequences useful in the invention include pelB, stII, ompA, phoA, bla and pe1A. One example are phagemid vectors which have an *E*. *coli.* origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector contains a β-lactamase gene to confer selectivity on the phagemid and a lac promoter upstream of a expression cassette that consists (N to C terminal) of a pelB leader sequence (which directs the expressed polypeptide to the periplasmic space), a multiple cloning site (for cloning the nucleotide version of the library member), optionally, one or more peptide tag (for detection), optionally, one or more TAG stop codon and the phage protein pIII. Thus, using various suppressor and non-suppressor strains of *E*. *coli* and with the addition of glucose, iso-propyl thio-β-D-galactoside (IPTG) or a helper phage, such as VCS M13, the vector is able to replicate as a plasmid with no expression, produce large quantities of the polypeptide library member only or produce phage, some of which contain at least one copy of the polypeptide-pIII fusion on their surface.

Construction of vectors encoding ligands according to the invention employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, *in situ* hybridisation, immunocytochemistry or sequence analysis of nucleic acid or protein molecules. Those skilled in the art will readily envisage how these methods may be modified, if desired.

### Skeletons

Skeletons may be based on immunoglobulin molecules or may be non-immunoglobulin in origin as set forth above. Preferred immunoglobulin skeletons as herein defined includes any one or more of those selected from the following: an immunoglobulin molecule comprising at least (i) the CL (kappa or lambda subclass) domain of an antibody; or (ii) the CH1 domain of an antibody heavy chain; an immunoglobulin molecule comprising the CH1 and CH2 domains of an antibody heavy chain; an immunoglobulin molecule comprising the CH1, CH2 and CH3 domains of an antibody heavy chain; or any of the subset (ii) in conjunction with the CL (kappa or lambda subclass) domain of an antibody. A hinge region domain may also be included.. Such combinations of domains may, for example, mimic natural antibodies, such as IgG or IgM, or fragments thereof, such as Fv, scFv, Fab or F(ab')₂ molecules. Those skilled in the art will be aware that this list is not intended to be exhaustive.

### Protein Scaffolds

Each epitope binding domain comprises a protein scaffold and one or more CDRs which are involved in the specific interaction of the domain with one or more epitopes. Advantageously, an epitope binding domain according to the present invention comprises three CDRs. Suitable protein scaffolds include any of those selected from the group consisting of the following: those based on immunoglobulin domains, those based on fibronectin, those based on affibodies, those based on CTLA4, those based on chaperones such as GroEL, those based on lipocallin and those based on the bacterial Fc receptors SpA and SpD. Those skilled in the art will appreciate that this list is not intended to be exhaustive.

### Scaffolds for use in Constructing Ligands

### Selection of the Main-chain Conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol., 196: 901; Chothia et al. (1989) Nature, 342: 877). Analysis of loop lengths and key residues has therefore enabled prediction of the main-chain conformations of H1, H2, L1, L2 and L3 found in the majority of human antibodies (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1).

Libraries of ligands and/or domains can be designed in which certain loop lengths and key residues have been chosen to ensure that the main-chain conformation of the members is known. Advantageously, these are real conformations of immunoglobulin superfamily molecules found in nature, to minimise the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use to assess the number of different main-chain conformations encoded by ligands, to predict the main-chain conformation based on ligand sequences and to choose residues for diversification which do not affect the canonical structure. It is known that, in the human V_{κ} domain, the L1 loop can adopt one of four canonical structures, the L2 loop has a single canonical structure and that 90% of human V_{κ} domains adopt one of four or five canonical structures for the L3 loop (Tomlinson *et al.* (1995) supra); thus, in the V_{κ} domain alone, different canonical structures can combine to create a range of different main-chain conformations. Given that the V_{λ} domain encodes a different range of canonical structures for the L1, L2 and L3 loops and that V_{κ} and V_{λ} domains can pair with any V_{H} domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a preferred aspect, the dual-specific ligands of the invention possess a single known main-chain conformation.

The single main-chain conformation that is chosen is preferably commonplace among molecules of the immunoglobulin superfamily type in question. A conformation is commonplace when a significant number of naturally occurring molecules are observed to adopt it. Accordingly, in a preferred aspect of the invention, the natural occurrence of the different main-chain conformations for each binding loop of an immunoglobulin domain are considered separately and then a naturally occurring variable domain is chosen which possesses the desired combination of main-chain conformations for the different loops. If none is available, the nearest equivalent may be chosen. It is preferable that the desired combination of main-chain conformations for the different loops is created by selecting germline gene segments which encode the desired main-chain conformations. It is more preferable, that the selected germline gene segments are frequently expressed in nature, and most preferable that they are the most frequently expressed of all natural germline gene segments.

In designing ligands (e.g., dAbs) or libraries thereof the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of V_{κ} (39%), L2 - CS 1 (100%), L3 - CS 1 of V_{κ} (36%) (calculation assumes a κ:λ ratio of 70:30, Hood et al. (1967) Cold Spring Harbor Symp. Quant. Biol., 48: 133). For H3 loops that have canonical structures, a CDR3 length (Kabat *et al.* (1991) *Sequences of proteins of immunological interest,* U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the V_{H} segment 3-23 (DP-47), the J_{H} segment JH4b, the V_{κ} segment O2/01 (DPK9) and the J_{κ} segment J_{κ}1. V_{H} segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five or for all six of the antigen binding loops can be determined. Here, it is preferable that the chosen conformation is commonplace in naturally occurring antibodies and most preferable that it observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

### Diversification of the Canonical Sequence

Having selected several known main-chain conformations or, preferably a single known main-chain conformation, ligands (e.g., dAbs) or libraries for use in the invention can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or are preferably selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins et al. (1992) J. Mol. Biol., 226: 889), chemical mutagenesis (Deng et al. (1994) J. Biol. Chem., 269: 9533) or bacterial mutator strains (Low et al. (1996) J. Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas et al. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97). Such diversification has been extended to include some or all of the other antigen binding loops (Crameri et al. (1996) Nature Med., 2: 100; Riechmann et al. (1995) Bio/Technology, 13: 475; Morphosys, WO97/08320, supra).

Since loop randomisation has the potential to create approximately more than 10¹⁵ structures for H3 alone and a similarly large number of variants for the other five loops, it is not feasible using current transformation technology or even by using cell free systems to produce a library representing all possible combinations. For example, in one of the largest libraries constructed to date, 6 x 10¹⁰ different antibodies, which is only a fraction of the potential diversity for a library of this design, were generated (Griffiths *et al.* (1994) supra).

Preferably, only the residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main-chain conformation.

### Diversification of the Canonical Sequence as it Applies to Antibody Domains

In the case of antibody based ligands (e.g., dAbs), the binding site for the target is most often the antigen binding site. Thus, preferably only those residues in the antigen binding site are varied. These residues are extremely diverse in the human antibody repertoire and are known to make contacts in high-resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat *et al.* (1991, supra), some seven residues compared to the two diversified in the library for use according to the invention. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the centre of the antigen binding site whereas somatic hypermutation spreads diversity to regions at the periphery of the antigen binding site that are highly conserved in the primary repertoire (see Tomlinson et al. (1996) J. Mol. Biol., 256: 813). This complementarity has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire can be created where some, but not all, of the residues in the antigen binding site are diversified. As used herein in this context, the term "naive" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

Naive repertoires of binding domains for the construction of ligands in which some or all of the residues in the antigen binding site are varied are known in the art. (See, WO 2004/058821, WO 2004/003019, and WO 03/002609). The "primary" library mimics the natural primary repertoire, with diversity restricted to residues at the centre of the antigen binding site that are diverse in the germline V gene segments (germline diversity) or diversified during the recombination process (junctional diversity). Those residues which are diversified include, but are not limited to, H50, H52, H52a, H53, H55, H56, H58, H95, H96, H97, H98, L50, L53, L91, L92, L93, L94 and L96. In the "somatic" library, diversity is restricted to residues that are diversified during the recombination process (junctional diversity) or are highly somatically mutated). Those residues which are diversified include, but are not limited to: H31, H33, H35, H95, H96, H97, H98, L30, L31, L32, L34 and L96. All the residues listed above as suitable for diversification in these libraries are known to make contacts in one or more antibody-antigen complexes. Since in both libraries, not all of the residues in the antigen binding site are varied, additional diversity is incorporated during selection by varying the remaining residues, if it is desired to do so. It shall be apparent to one skilled in the art that any subset of any of these residues (or additional residues which comprise the antigen binding site) can be used for the initial and/or subsequent diversification of the antigen binding site.

In the construction of libraries for use in the invention, diversification of chosen positions is typically achieved at the nucleic acid level, by altering the coding sequence which specifies the sequence of the polypeptide such that a number of possible amino acids (all 20 or a subset thereof) can be incorporated at that position. Using the IUPAC nomenclature, the most versatile codon is NNK, which encodes all amino acids as well as the TAG stop codon. The NNK codon is preferably used in order to introduce the required diversity. Other codons which achieve the same ends are also of use, including the NNN codon, which leads to the production of the additional stop codons TGA and TAA.

A feature of side-chain diversity in the antigen binding site of human antibodies is a pronounced bias which favours certain amino acid residues. If the amino acid composition of the ten most diverse positions in each of the V_{H}, V_{κ} and V_{λ} regions are summed, more than 76% of the side-chain diversity comes from only seven different residues, these being, serine (24%), tyrosine (14%), asparagine (11%), glycine (9%), alanine (7%), aspartate (6%) and threonine (6%). This bias towards hydrophilic residues and small residues which can provide main-chain flexibility probably reflects the evolution of surfaces which are predisposed to binding a wide range of antigens or epitopes and may help to explain the required promiscuity of antibodies in the primary repertoire.

Since it is preferable to mimic this distribution of amino acids, the distribution of amino acids at the positions to be varied preferably mimics that seen in the antigen binding site of antibodies. Such bias in the substitution of amino acids that permits selection of certain polypeptides (not just antibody polypeptides) against a range of target antigens is easily applied to any polypeptide repertoire. There are various methods for biasing the amino acid distribution at the position to be varied (including the use of tri-nucleotide mutagenesis, see WO97/08320), of which the preferred method, due to ease of synthesis, is the use of conventional degenerate codons. By comparing the amino acid profile encoded by all combinations of degenerate codons (with single, double, triple and quadruple degeneracy in equal ratios at each position) with the natural amino acid use it is possible to calculate the most representative codon. The codons (AGT)(AGC)T, (AGT)(AGC)C and
(AGT)(AGC)(CT) - that is, DVT, DVC and DVY, respectively using IUPAC nomenclature - are those closest to the desired amino acid profile: they encode 22% serine and 11 % tyrosine, asparagine, glycine, alanine, aspartate, threonine and cysteine. Preferably, therefore, libraries are constructed using either the DVT, DVC or DVY codon at each of the diversified positions.

### L929 Cytotoxicity Assay

Antagonists of TNFR1 (e.g., ligands, dAb monomers) can be identified by the ability to inhibit TNF-induced cytotoxicity in mouse L929 fibroblasts (Evans, T. (2000) Molecular Biotechnology 15, 243-248). Briefly, L929 cells plated in microtitre plates are incubated overnight with antagonist of TNFR1, 100 pg/ml TNF and 1mg/ml actinomycin D (Sigma, Poole, UK). Then cell viability is measured by reading absorbance at 490nm following an incubation with 3-(4,5-dimethylthiazol-2-yl)-5-(3-carbboxymethoxyphenyl)-2-(4-sulfophenyl)-2H-tetrazolium (Promega, Madison, USA). Antagonists of TNFR1 will inhibit cytotoxicity and therefore produce an increase in absorbance compared with TNF only control.

### HeLa IL-8 assay

Antagonists of TNFR1 (e.g., ligands, dAb monomers) can be identified by the ability to inhibit TNF-induced secretion of IL-8 by human HeLa cells (method adapted from that of Akeson, L. et al (1996) Journal of Biological Chemistry 271, 30517-30523, describing the induction of IL-8 by IL-1 in HUVEC; here we look at induction by human TNF alpha and we use HeLa cells instead of the HUVEC cell line). Briefly, HeLa cells can be plated in microtitre plates were incubated overnight with antagonist of TNFR1 and 300pg/ml TNF. Post incubation, the supernatant is aspirated off the cells and IL-8 concentration is measured using a sandwich ELISA (R&D Systems), or other suitable method. Antagonists of TNFR1 inhibit IL-8 secretion, and less IL-8 is detected in the supernatant compared with the TNF only control.

### EXAMPLES

### Example 1. Extension of the serum half-life of tumour necrosis factor (TNF).

Mice transgenic for human TNF (Tg197) were administered in groups of 10 animals with weekly intraperitoneal doses of anti-TNF dAbs which had been reformatted either as dimers with a 40k branched PEG (termed PEG TAR1-5-19) or as a fusion with an anti-serum albumin dAb (termed TAR1-5-19/MSA trimer). Saline administration was used as a control. After 7 weeks of drug administration the serum of the animals was analysed for circulating TNF levels using an anti-TNF sandwich ELISA kit. Animals administered saline had a circulating TNF level of 25.6pg/ml whereas animals receiving TAR1-5-19/MSA trimer had TNF levels elevated to 28.8pg/ml and PEG TAR1-5-19 had levels elevated to 2315pg/ml. This clearly demonstrates that dAbs with an extended serum half-life can increase the circulating levels of a molecule with a short serum half-life such as a cytokine and therefore increase the serum levels.

### Example 2. Extension of the serum half-life of soluble tumour necrosis factor receptor 1 (sTNFR1).

CD1 mice were administered a single intravenous 1mg/kg dose of an anti-mouse TNFR1 dAb which had been reformatted (extended half-life formats) with either a 40k branched PEG (termed PEG anti-TNFR1 dAb) or as a fusion with an anti-serum albumin dAb (termed anti-TNFR1/anti-SA dimer). Serum was examined at various time points for levels of soluble TNFR1 using a anti-TNFR1 sandwich ELISA as detailed below.

### Measurement of TNFR1

F96 Maxisorp 96 well flat bottom immunoplates (Nunc, Cat No: 439454) were coated with anti-mouse sTNF R1/TNFRSF1A antibody (50ul per well at 1ug/ml in PBS). Plates were incubated for 1hr at room temperature. Plates were washed three times with 200ul PBS. Non-specific binding was blocked with 3% BSA/PBS for 1hr at room temperature. Plates were washed three times with 200ul PBS/0.05% Tween-20.

Dilutions of recombinant mouse sTNF R1 (R & D Systems, cat# 4254-R1) were prepared in PBS/1% BSA at 5000ng ml⁻¹, 500ng ml⁻¹, 50ng ml⁻¹ and 5ng ml⁻¹ and 50ul added to the well. Alternatively, serum from mice immunised with TAR2m-21-23/40K branched PEG was prepared at 1/5, 1/50, 1/500 and 1/5000 dilutions in PBS/1%BSA and 50ul added to the well. Plates were incubated for 1 hour, room temperature. Plates were washed three times with 200ul PBS/0.05% Tween-20.

Biotinylated anti-mouse sTNF R1 antibody (R & D Systems, cat # AHF01) was prepared at 1ug/ml and 50ul added to the well. Plates were incubated for 1 hour, room temperature. Plates were washed three times with 200ul PBS/0.05% Tween-20.

Peroxidase-conjugated IgG fraction monoclonal mouse anti-biotin (Stratech, Cat No: 200-032-096) was prepared at 160ng ml⁻¹ and 50ul added to the well. Plates were incubated for 1 hour, room temperature. Plates were washed three times with 200ul PBS/0.05% Tween-20 then three times with PBS.

SureBlue TMB 1-component microwell peroxidase substrate (KPL, Cat No: 52-00-00) was added to all wells. The plate was incubated at room temperature for 15 minutes and the reaction stopped with 1M HCl. The absorbance was read at 450nM.

All samples were assayed in duplicate. Wells without sample were included.

The OD results for the 1/5 dilutions of serum from the mice dosed with the PEG anti-TNFR1 dAb were plotted against time (Figure 1). The results clearly show the increasing levels of TNFR1 in the serum before falling back to basal levels following clearance of the dAb.

### Example 3. dAbs That Bind Receptor Molecules But Do Not Bind The Active Site.

This example illustrates a method for identify dAbs that bind a desired receptor molecule but does not bind the active site of the receptor. The approach is illustrated using tumor necrosis factor 1 receptor (TNFR1), and is generally applicable to receptor molecules.

Chimeric receptor molecules made up of different murine TNFR1 domains and human TNFR1 domains (Banner DW, et al. Cell, 73(3):431-45 (1993).) such that the molecule contains the four defined extracellular domains of TNFR1 but that these vary in origin between mouse and human TNFR1 proteins were produced. The produced chimeric receptors shared properties of both human TNFR1 and mouse TNFR1 according to the differing domain roles and functionality. The molecules provided a means for the assessment of the domain specificity of dAbs, antibodies and antigen-binding fragments thereof and other molecules (eg protein domains such as affibodies, LDL receptor domains, or EGF domains) that bind human or mouse TNFR1.

### Methods

Human and mouse TNFR1 sequences were previously cloned into the *Pichia* expression vector pPicZalpha (Invitrogen) via EcoR1 and NotI restriction endonuclease sites. The template mouse TNFR1 DNA (and consequently chimeric receptor constructs ending with a murine domain 4) contained a 3' 6x Histidine tag. Human TNFR1 (and consequently chimeric receptor constructs ending with a human domain 4) contained both Myc and 6x Histidine tags in sequence at the 3' end.

Initial PCRs were performed according to standard PCR conditions using RubyTaq DNA polymerase (USB Corporation, Cleveland, Ohio), 100 ng of template DNA (comprising the relevant DNA miniprep template of either full length mTNFR1 or hTNFR1 DNA).

Typical PCR reacts were set up was as follows: 25 µl of 10X RubyTaq PCR buffer containing polymerase; 2 µl of first primer (from 10 µM stock); 2 µl of second primer (from 10 µM stock); 1 µl (100 ng) full length TNFR1 template DNA; 20 µl of dH₂O (to a final volume of 50 µl). The reactions were set up in thin walled tubes and placed into a thermocycler where the reaction was performed according to the following parameters.

| | | |
|---|---|---|
| Initial Denaturation | 3 minutes | 94°C |
| | | |
| Denaturation | 30 seconds | 94°C |
| Annealing (25 cycles) | 30 seconds | 55°C |
| Extension | 1 minute | 72°C |
| | | |
| Final Extension | 10 minutes | 72°C |

Summary of initial PCR reactions used in generation of chimeric constructs

| Construct* | PCR number - primers used | Template |
|---|---|---|
| MHHH | PCR 1 - 1 and 12 | Mouse |
| | PCR 2 - 2 and 3 | Human |
| HMHH | PCR1 -1 and 9 | Human |
| | PCR 2-6 and 13 | Mouse |
| | PCR 3 - 2 and 4 | Human |
| HHMH | PCR 1 - 1 and 10 | Human |
| | PCR 2 - 7 and 14 | Mouse |
| | PCR 3 - 2 and 5 | Human |
| HHHM | PCR 1 - 1 and 11 | Human |
| | PCR 2 - 2 and 8 | Mouse |
| HMMM | PCR 1 -1 and 9 | Human |
| | PCR 2 - 2 and 6 | Mouse |

| | | |
|---|---|---|
| *Notation: H=human domain; M=mouse domain; eg, MHHH = mouse Domain 1, human domains 2-4. | | |

PCR products generated these initial PCRs were cut out from a 1% agarose gel and purified using a gel purification kit (Qiagen) before elution into 50 µl dH₂O.

Primers used for Chimeric TNFR1 construct generation

| Primer Number | Primer sequence |
|---|---|
| Primer 1 | GCCAGCATTGCTGCTAAAGAA (SEQ ID NO:5) |
| Primer 2 | GGTCGACGGCGCTATTCAG (SEQ ID NO:6) |
| Primer 3 | CTGCAGGGAGTGTGAGAGCGGC (SEQ ID NO:7) |
| Primer 4 | GTGTGTGGCTGCAGGAAGAAC (SEQ ID NO: 8) |
| Primer 5 | CTGCCATGCAGGTTTCTTTC (SEQ ID NO:9) |
| Primer 6 | CTGCAGGGAGTGTGAAAAGGG (SEQ ID NO: 10) |
| Primer 7 | GTGTGTGGCTGTAAGGAGAACC (SEQ ID NO:11) |
| Primer 8 | CTGCCATGCAGGGTTCTTTC (SEQ ID NO:12) |
| Primer 9 | TCACACTCCCTGCAGTCCG (SEQ ID NO:13) |
| Primer 10 | CAGCCACACACGGTGTCCCGG (SEQ ID NO:14) |
| Primer 11 | CCTGCATGGCAGGTGCACACGG (SEQ ID NO:15) |
| Primer 12 | TCACACTCCCTGCAGACTG (SEQ ID NO:16) |
| Primer 13 | CAGCCACACACCGTGTCCTTG (SEQ ID NO:17) |
| Primer 14 | CCTGCATGGCAGTTACACACGG (SEQ ID NO:18) |

### SOE PCR

Assembly PCR (also known as 'pull-through' or Splicing by Overlap Extension (SOE) see Gene, 15:77(1):61-8 (1989)) allows the primary PCR products to be brought together without digest or ligation, making use of the complementary ends of the Primary PCR products. During this process the primary products are brought together and denatured before their complementary ends are allowed to anneal together in the presence of Taq DNA polymerase and dNTPs. Several cycles of reannealing and extension result in fill-in of the complementary strands and the production of a full-length template. Primers that flank the now full-length construct cassette are added and a conventional PCR was run to amplify the assembled product. SOE PCRs were performed in order to anneal together and amplify the various TNFR1 domains derived from the initial PCRs described above. Assembly SOE PCRs were set up as follows: 40 µl 10 x PCR buffer containing MgCl₂; ∼2 µl (100 ng) cleaned product of initial PCR 1; -2 µl (100 ng) cleaned product of initial PCR 2; 36 µl dH₂O (to final volume of 80 µl). SOE primer mix was added after the assembly step as follows: 2 µl 5' flanking primer (Primer 1); 2 µl 3' flanking primer (Primer 2); 10 µl 10x PCR Buffer; 6 µl dH2O (to final volume 20 µl).

The PCR reactions were performed using the program described below. The initial assembly cycles required approximately 45 minutes after which the thermocycler was set to pause at 94°C. 20 µl of primer mix was added to each reaction and mixed.

### Assembly conditions

| | | |
|---|---|---|
| Initial Denaturation | 5 minutes | 94°C |
| Denaturation | 1 minute | 94°C |
| Annealing (15 cycles) | 1 minute | 55°C |
| Extension | 1 minute | 72°C |

Amplification conditions (Pause at 94°C, Primers mix then added)

| | | |
|---|---|---|
| Denaturation | 1 minute | 94°C |
| Annealing (25 cycles) | 1 minute | 55°C |
| Extension | 1 minute | 72°C |

PCR products were checked by running 3-5 µl of each reaction on a 1% agarose gel.

Cloning of assembled TNFR1 chimeras into the *Pichia* expression vector.

pPicZalpha vector (Invitrogen) was sequentially digested with EcoRI and NotI enzymes prior to Chromaspin TE-1000 gel filtration column (Clontech, Mountain View, CA) purification.

### Transformation of TNFR1 chimeric constructs into E. coli.

The ligated chimeric constructs were transformed into HB2151 electrocompetent *E*. *coli* cells and recovered for an hour in low salt LB media prior to plating on low salt LB agar with 0.25 µg/ml ZEOCIN, antibiotic formulation containing Phleomycin D (Cayla, Toulouse, France), for 24 hrs at 37°C. Individual colonies were then sequence verified to ensure the correct sequence of the chimeric construct within the expression vector and large scale Maxiprep plasmid preparations made of each chimeric construct vector.

The nucleotide sequences of prepared chimeric constructs are presented below. The chimeric constructs were named according to origin of their domains (running from Domain 1 on the left to Domain 4 on the right). For example, HMMM contains human Domain 1 and mouse Domains 2-4. Chimeric proteins that contain mouse domain 4 have only His tags and lack the spacer region between the transmembrane region and Domain 4.

HMMM

AGTGTGTGTCCCCAAGGAAAATATATCCACCCTCAAAATAATTCGATTTGCTGTACCAAGTGCCACAA
AGGAACCTACTTGTACAATGACTGTCCAGGCCCGGGGCAGGATACGGACTGCAGGGAGTGTGAAAAGG
GCACCTTTACGGCTTCCCAGAATTACCTCAGGCAGTGCCTCAGTTGCAAGACATGTCGGAAAGAAATG
TCCCAGGTGGAGATCTCTCCTTGCCAAGCTGACAAGGACACGGTGTGTGGCTGTAAGGAGAACCAGTT
CCAACGCTACCTGAGTGAGACACACTTCCAGTGCGTGGACTGCAGCCCCTGCTTCAACGGCACCGTGA
CAATCCCCTGTAAGGAGACTCAGAACACCGTGTGTAACTGCCATGCAGGGTTCTTTCTGAGAGAAAGT
GAGTGCGTCCCTTGCAGCCACTGCAAGAAAAATGAGGAGTGTATGAAGTTGTGCCTAAGCGCTCATCA
TCATCATCATCATTAATGA (SEQ ID NO:19)

HHHM

AGTGTGTGTCCCCAAGGAAAATATATCCACCCTCAAAATAATTCGATTTGCTGTACCAAGTGCCACAA
AGGAACCTACTTGTACAATGACTGTCCAGGCCCGGGGCAGGATACGGACTGCAGGGAGTGTGAGAGCG
GCTCCTTCACCGCTTCAGAAAACCACCTCAGACACTGCCTCAGCTGCTCCAAATGCCGAAAGGAAATG
GGTCAGGTGGAGATCTCTTCTTGCACAGTGGACCGGGACACCGTGTGTGGCTGCAGGAAGAACCAGTA
CCGGCATTATTGGAGTGAAAACCTTTTCCAGTGCTTCAATTGCAGCCTCTGCCTCAATGGGACCGTGC
ACCTCTCCTGCCAGGAGAAACAGAACACCGTGTGCACCTGCCATGCAGGGTTCTTTCTGAGAGAAAGT
GAGTGCGTCCCTTGCAGCCACTGCAAGAAAAATGAGGAGTGTATGAAGTTGTGCCTAAGCGCTCATCA
TCATCATCATCATTAATGA (SEQ ID NO:20)

HHMH

AGTGTGTGTCCCCAAGGAAAATATATCCACCCTCAAAATAATTCGATTTGCTGTACCAAGTGCCACAA
AGGAACCTACTTGTACAATGACTGTCCAGGCCCGGGGCAGGATACGGACTGCAGGGAGTGTGAGAGCG
GCTCCTTCACCGCTTCAGAAAACCACCTCAGACACTGCCTCAGCTGCTCCAAATGCCGAAAGGAAATG
GGTCAGGTGGAGATCTCTTCTTGCACAGTGGACCGGGACACCGTGTGTGGCTGTAAGGAGAACCAGTT
CCAACGCTACCTGAGTGAGACACACTTCCAGTGCGTGGACTGCAGCCCCTGCTTCAACGGCACCGTGA
CAATCCCCTGTAAGGAGACTCAGAACACCGTGTGTAACTGCCATGCAGGTTTCTTTCTAAGAGAAAAC
GAGTGTGTCTCCTGTAGTAACTGTAAGAAAAGCCTGGAGTGCACGAAGTTGTGCCTACCCCAGATTGA
GAATGTTAAGGGCACTGAGGACTCAGGCACCACAGCGGCCGCCAGCTTTCTAGAACAAAAACTCATCT
CAGAAGAGGATCTGAATAGCGCCGTCGACCATCATCATCATCATCATTGA (SEQ ID NO:21)

HMHH

AGTGTGTGTCCCCAAGGAAAATATATCCACCCTCAAAATAATTCGATTTGCTGTACCAAGTGCCACAA
AGGAACCTACTTGTACAATGACTGTCCAGGCCCGGGGCAGGATACGGACTGCAGGGAGTGTGAAAAGG
GCACCTTTACGGCTTCCCAGAATTACCTCAGGCAGTGTCTCAGTTGCAAGACATGTCGGAAAGAAATG

TCCCAGGTGGAGATCTCTCCTTGCCAAGCTGACAAGGACACGGTGTGTGGCTGCAGGAAGAACCAGTA
CCGGCATTATTGGAGTGAAAACCTTTTCCAGTGCTTCAATTGCAGCCTCTGCCTCAATGGGACCGTGC
ACCTCTCCTGCCAGGAGAAACAGAACACCGTGTGCACCTGCCATGCAGGTTTCTTTCTAAGAGAAAAC
GAGTGTGTCTCCTGTAGTAACTGTAAGAAAAGCCTGGAGTGCACGAAGTTGTGCCTACCCCAGATTGA
GAATGTTAAGGGCACTGAGGACTCAGGCACCACAGCGGCCGCCAGCTTTCTAGAACAAAAACTCATCT
CAGAAGAGGATCTGAATAGCGCCGTCGACCATCATCATCATCATCATTGA (SEQ ID NO:22)

MHHH

AGCTTGTGTCCCCAAGGAAAGTATGTCCATTCTAAGAACAATTCCATCTGCTGCACCAAGTGCCACAA
AGGAACCTACTTGGTGAGTGACTGTCCGAGCCCAGGGCGGATACAGTCTGCAGGGAGTGTGAGAGCG
GCTCCTTCACCGCTTCAGAAAACCACCTCAGACACTGCCTCAGCTGCTCCAAATGCCGAAAGGAAATG
GGTCAGGTGGAGATCTCTTCTTGCACAGTGGACCGGGACACCGTGTGTGGCTGCAGGAAGAACCAGTA
CCGGCATTATTGGAGTGAAAACCTTTTCCAGTGCTTCAATTGCAGCCTCTGCCTCAATGGGACCGTGC
ACCTCTCCTGCCAGGAGAAACAGAACACCGTGTGCACCTGCCATGCAGGTTTCTTTCTAAGAGAAAAC
GAGTGTGTCTCCTGTAGTAACTGTAAGAAAAGCCTGGAGTGCACGAAGTTGTGCCTACCCCAGATTGA
GAATGTTAAGGGCACTGAGGACTCAGGCACCACAGCGGCCGCCAGCTTTCTAGAACAAAAACTCATCT
CAGAAGAGGATCTGAATAGCGCCGTCGACCATCATCATCATCATCATTGA (SEQ ID NO:23)

### Preparation of TNFR1 chimeric construct and transformation into Pichia pastoris.

The plasmid DNA generated by each maxiprep was digested with the infrequent cutting restriction endonuclease PmeI in order to linearise the DNA prior to *pichia* transformation. The linearised DNA was subsequently cleaned by phenol/chloroform extraction and ethanol precipitation, before resuspension in 30 µl of dH₂O. 10 µl of the linearised DNA solution was mixed with 80 µl of electro-competent KM71H *Pichia* cells for 5 minutes prior to electroporation at 1.5kV, 200Ω, 25 µF. Cells were immediately recovered with YPDS and incubated for 2 hours at 30°C before plating on YPDS agar plates containing 100 µg/ml ZEOCIN, antibiotic formulation containing Phleomycin D (Cayla, Toulouse, France), for 2 days.

### Expression of constructs in Pichia.

An individual transformant colony for each construct was picked into 5 ml of BMGY as a starter culture and grown for 24 hrs at 30°C. This culture was used to inoculate 500 ml of BMGY media which was grown for 24 hrs at 30°C before cells were harvested by centrifugation at 1500-3000g for 5 minutes at room temp. Cells were then resuspended in 100 ml of BMMY and grown for 4 days with staggered increases in methanol concentration (0.5% day 1, 1% day 2, 1.5% day 3 and 2% day 4). After expression supernatant was recovered after centrifugation of the cultures at 3300g for 15 minutes.

### Purification of TNFR1 chimeric constructs using Nickel resin.

Culture supernatants were initially buffered through addition of 10 mM final concentration imidazole and 2x PBS. His-tagged protein was batch absorbed for 4 hours (shaking) at room temperature through addition of Nickel-NTA resin. The supernatant/resin mix was then flowed into a poly-prep column (Biorad). Resin was then washed with 10 column volumes of 2xPBS before elution using 250 mM imidazole 1x PBS. After buffer exchange the chimeric construct expression was deglycosylated using the EndoH deglycosylase before verification by SDS-PAGE.

### Template DNA sequences used during PCR

Human (*Homo sapiens*) TNFR1 (extracellular region Genbank accession 33991418)

CTGGTCCCTCACCTAGGGGACAGGGAGAAGAGAGATAGTGTGTGTCCCCAAGGAAAATA
TATCCACCCTCAAAATAATTCGATTTGCTGTACCAAGTGCCACAAAGGAACCTACTTGTA
CAATGACTGTCCAGGCCCGGGGCAGGATACGGACTGCAGGGAGTGTGAGAGCGGCTCCT
TCACCGCTTCAGAAAACCACCTCAGACACTGCCTCAGCTGCTCCAAATGCCGAAAGGAA
ATGGGTCAGGTGGAGATCTCTTCTTGCACAGTGGACCGGGACACCGTGTGTGGCTGCAG
GAAGAACCAGTACCGGCATTATTGGAGTGAAAACCTTTTCCAGTGCTTCAATTGCAGCCT
CTGCCTCAATGGGACCGTGCACCTCTCCTGCCAGGAGAAACAGAACACCGTGTGCACCT
GCCATGCAGGTTTCTTTCTAAGAGAAAACGAGTGTGTCTCCTGTAGTAACTGTAAGAAAA
GCCTGGAGTGCACGAAGTTGTGCCTACCCCAGATTGAGAATGTTAAGGGCACTGAGGAC
TCAGGCACCACA (SEQ ID NO:1)

The encoded extracellular region of human TNFR1 has the following amino acid sequence.

LVPHLGDREKRDSVCPQGKYIHPQNNSICCTKCHKGTYLYNDCPGPGQDTDCRECESGSFTA
SENHLRHCLSCSKCRKEMGQVEISSCTVDRDTVCGCRKNQYRHYWSENLFQCFNCSLCLNG
TVHLSCQEKQNTVCTCHAGFFLRENECVSCSNCKKSLECTKLCLPQIENVKGTEDSGTT
(SEQ ID NO:2)

Murine (*Mus musculus*) TNFR1 (extracellular region Genbank accession 31560798)

CTAGTCCCTTCTCTTGGTGACCGGAGAAGAGGGATAGCTTGTGTCCCCAAGGAAAGTA
TGTCCATTCTAAGAACAATTCCATCTGCTGCACCAAGTGCCACAAAGGAACCTACTTGGT
GAGTGACTGTCCGAGCCCAGGGCGGATACAGTCTGCAGGGAGTGTGAAAAGGGCACC
TTTACGGCTTCCCAGAATTACCTCAGGCAGTGTCTCAGTTGCAAGACATGTCGGAAAGA
AATGTCCCAGGTGGAGATCTCTCCTTGCCAAGCTGACAAGGACACGGTGTGTGGCTGTA
AGGAGAACCAGTTCCAACGCTACCTGAGTGAGACACACTTCCAGTGCGTGGACTGCAGC
CCCTGCTTCAACGGCACCGTGACAATCCCCTGTAAGGAGACTCAGAACACCGTGTGTAA
CTGCCATGCAGGGTTCTTTCTGAGAGAAAGTGAGTGCGTCCCTTGCAGCCACTGCAAGA
AAAATGAGGAGTGTATGAAGTTGTGCCTACCTCCTCCGCTTGCAAATGTCACAAACCCCC
AGGACTCAGGTACTGCG (SEQ ID NO:3)

The encoded extracellular region of murine (*Mus musculus*) TNFR1 has the following amino acid sequence.

LVPSLGDREKRDSLCPQGKYVHSKNNSICCTKCHKGTYLVSDCPSPGRDTVCRECEKGTFTA
SQNYLRQCLSCKTCRKEMSQVEISPCQADKDTVCGCKENQFQRYLSETHFQCVDCSPCFNGT
VTIPCKETQNTVCNCHAGFFLRESECVPCSHCKKNEECMKLCLPPPLANVTNPQDSGTA
(SEQ ID NO:4)

### Domain specificity of anti-TNFR1 dAbs

The domain specificity of anti-TNFR1 dAbs was determined using surface plasmon resonance (SPR) ('Detection of immuno-complex formation via surface plasmon resonance on gold-coated diffraction gratings.' Biosensors. 1987-88;3(4):211-25.) to determine the ability of antibodies to bind fully human or mouse biotinylated TNFR1 that was immobilized on a SPR chip surface, after the antibodies had been incubated and equilibrated with an excess of the chimeric receptors described above. In this assay, flow of an anti- TNFR1 dAb over the TNFR1 surface generates an SPR signal indicating that amount of dAb that binds TNFR1 immoblized on the SPR chip. If the dAb is pre-incubated and equilibrated with a chimeric molecule that comprises the domain(s) of TNFR1 that the particular dAb binds, then flow of this mixture over the TNFR1 surface will produce a smaller SPR signal relative to the dAb alone. However, if the dAb is pre-incubated and equilibrated with a chimeric molecule that does not comprises the domain(s) of TNFR1 that the particular dAb binds, then flow of this mixture over the TNFR1 surface will produce a SPR signal that is about the same as the signal obtained using dAb alone.

### Method

### Generation of an SPR chip TNFR1 surface.

The choice of TNFR1 surface is determined by the species specificity of the anti-TNFR1 dAb to be tested. Therefore anti-human TNFR1 dAbs were evaluated using a surface coated with human TNFR1 and anti-mouse TNFR1 dAbs were evaluated using a chip coated with mouse TNFR1.

Biotinylated TNFR1 was diluted in the appropriate SPR buffer and run across a streptavidin (SA) sensor chip in a BIACORE 3000 SPR instrument (Biacore International AB, Uppsala, Sweden). A low flow-rate (5-10 µl/minute) was used in order to maximise the contact time between the biotinylated TNFR1 and the streptavidin surface. Flow continued until the streptavidin surface was saturated with biotinylated material, in order to generate a chip with maximal TNFR1 surface. The chip typically bound several hundred to several thousand SPR response units of the biotinylated material.

### Titration of the anti- TNFR1 response on the SPR chip

A successful competition experiment requires initial optimisation of the concentration of anti- TNFR1 dAb such that the minimum amount of dAb is flowed over the surface that gives a significant SPR signal. Within a certain concentration range, the dAb will bind the surface in a dose dependent manner so that the number of RUs of dAb bound reflect the concentration of dAb flowed across the chip surface.

In order to ascertain the concentration range of this dose-dependency, the anti-TNFR1 dAbs were titrated in a 10-fold dilution series of Biacore buffer ranging from a 1 in 10 dilution to a 1 in 1,000,000 dilution. The dilutions were then individually and sequentially injected across the TNFR1 chip surface, starting with the most dilute sample. The maximal number of RUs achieved at each dilution were measured. After each injection the TNFR1 surface was regenerated to remove bound anti-TNFR1 dAb where necessary using a suitable SPR regeneration buffer. Using this method the minimal concentration of anti-TNFR1 dAb required to generate a signal representing approximately 100RU was determined.

### Pre-equilibration of anti-TNFR1 dAbs/chimerics.

Once the optimum anti-TNFR1 dAb concentration was determined, anti-TNFR1 dAb/chimeric TNFR1 mixes were set up. Mixes were set up such that the final concentration of anti-TNFR1 dAb was identical to the optimal concentration determined previously. Reactions were typically set up in 100 µl volumes containing 50 microliters of a 2 x concentrate of anti-TNFR1 dAb, 40 microliters of Biacore buffer and 10 microliters of neat, purified chimeric protein. Typical concentrations for the final mix were about 10-100 µM of chimeric protein and about 10-100 nM anti-TNFR1 dAb. Mixtures were allowed to equilibrate for 30 minutes at room temperature.

### Competition Biacore experiment

After equilibration, each anti-TNFR1 dAb/chimeric TNFR1 mixture was sequentially run over the TNFR1 SPR surface and the number of response units measured. After each mixture was injected, the surface was regenerated to remove bound anti-TNFR1 dAb on before the next mixture was injected. The different responses generated using the different chimerics enabled determination of the TNFR1 domains bound by particular dAbs.

These studies revealed that TAR2m-21-23 binds Domain 1 of mouse TNFR1, and that TAR2h-205 binds Domain 1 of human TNFR1.
TAR2m-21-23 
EVQLLESGGGLVQPGGSLRLSCAASGFTFN~RYSMG~WLRQAPGKGLEWVS~~RIDSYGRGT
YYEDPVKG~~RFSISRDNSKNTLYLQMNSLRAEDTAVYYCAK~~~ISQFGSNAFDY~~~WGQ
GTQVTVSS (SEQ ID NO:24)
 TAR2h-205

EVQLLESGGGLVQPGGSLRLSCAASGFTFV~KYSMG~WVRQAPGKGLEWVS~~QISNTGGHT
YYADSVKG~~RFTISRDNSKNTLYLQMNSLRAEDTAVYYCAK~~~YTGRWEPFDY~~~WGQG
TLVTVSS  (SEQ ID NO:25)

In the amino acid sequences of TAR2M-21-23 and TAR2h-205 CDR1 is flanked by ∼ CDR2 is flanked by ∼∼, and CDR3 is flanked by ∼∼∼. The presented amino acid sequences are continuous with not gaps.

### Example 4. Screening Methods

Chimeric receptor proteins such as the chimeric TNFR1 proteins described herein can be used in assays or screens to isolate agents (e.g., antibodies, dAbs) that bind to particular domains within a desired receptor. Using TNFR1 as a model for a desired receptor protein, these methods describe the addition of chimeric proteins to crude antibody preparations prior to their screening for receptor binding either by ELISA or surface plasmon resonance. Additionally they describe the use of chimeric proteins coated on a surface (*e.g.*, ELISA plate or SPR chip) and the screening of antibodies through testing of their binding to chimeric proteins on this surface. Similar methods can be used to identify agents (e.g., antibodies, dAbs) that bind to desired domains (e.g., outside of the active site) of other receptors.

### Soluble ELISA Screen

This method can be used to rapidly isolate antibodies or antibody fragments (e.g., dAbs) that bind specific domains of TNFR1 from a large repertoire of antibodies or antibody fragments of unknown specificity.

A 96 well assay plate will be coated overnight at 4°C with 100µl per well of chimeric TNFR1. Wells will be washed 3 times with 0.1% TPBS (Phosphate buffered saline containing Tween-20 at a concentration of 0.1%). 200µl per well of 1% TPBS will be added to block the plate, and the plate incubated for 1-2 hours at room temperature. Wells will then be washed 3 times with PBS before addition of 50µl of bacterial supernatant or periprep, containing the soluble antibody or antibody fragment (that contain the c-Myc epitope tag), in 50µl 0.2% TPBS. The plate will then be incubated for 1 hour at room temperature. After this the plate will be washed 5 times with 0.1% TPBS (0.1% Tween-20 in PBS). 100 µl of a primary anti-c-Myc mouse monoclonal will then be added in 0.1 % TPBS to each well and the plate will be incubated for 1 hour at room temperature. This primary antibody solution will be discarded and the plate will then be washed 5 times with 0.1% TPBS. 100 µl of prediluted anti-mouse IgG (Fc specific) HRP conjugate from goat will then be added (Sigma Cat No: A0168) and the plate will be incubated for 1 hour at room temperature. The secondary antibody will then be discarded and the plate will be washed 6 times with 0.1 % TPBS followed by 2 washes with PBS. 50µl of TMB peroxidase solution will then be added to each well and the plate will be left at room temperature for 2-60 minutes. The reaction will be stopped by the addition of 50µl of 1M hydrochloric acid. The OD at 450 nm of the plate will be read in a 96-well plate reader within 30 minutes of acid addition. Those antibodies present in crude bacterial supernatant or peripreps that bind the domains of TNFR1 present within the chimeric protein will give a stronger ELISA signal than those that do not.

### Competitive ELISA Screen

This method can be used to rapidly screen a diverse sets of crude antibody or antibody fragment preparations that bind TNFR1 in order to determine their domain binding specificity.

A 96 well assay plate will be coated overnight at 4°C with 100µl per well of murine or human TNFR1 (either human or mouse). Wells will be washed 3 times with 0.1 % TPBS (Phosphate buffered saline containing Tween-20 at a concentration of 0.1%). 200µl per well of 1% TPBS (1% Tween-20 in PBS) will be added to block the plate, and the plate incubated for 1-2 hours at room temperature. Wells will then be washed 3 times with PBS. At the same time bacterial supernatants or peripreps will be pre-equilibrated with a pre-optimised concentration of chimeric TNFR1 protein in solution. 50µl of this crude bacterial preparation/chimeric protein mix, containing the soluble antibody or antibody fragment will then be added to the ELISA plate. The plate will be incubated for 1 hour at room temperature. Then, the plate will be washed 5 times with 0.1 % TPBS (0.1 % Tween-20 in PBS), and 100µl of a primary detecting antibody (or Protein A-HRP or Protein L HRP) will be added in 0.1% TPBS, to each well and the plate will be incubated for 1 hour at room temperature. This primary antibody solution will be discarded and the plate will be washed 5 times with 0.1 % TPBS. If required 100µl of a prediluted secondary antibody/HRP conjugate from goat will then be added, and the plate will be incubated for 1 hour at room temperature. The secondary antibody will then be discarded and the plate will be washed 6 times with 0.1% TPBS followed by 2 washes with PBS. 50µl of TMB peroxidase solution will be added to each well and the plate will be left at room temperature for 2-60 minutes. The reaction will be stopped by the addition of 50 µl of 1M hydrochloric acid. The OD at 450 nm of the plate will be read in a 96-well plate reader within 30 minutes of acid addition. A reduction in ELISA signal will be indicative of the antibody binding the chimeric TNFR1 domains rather than the full TNFR1 coated on the plate, and therefore, that the antibody binds one of the domains within the chimeric protein.

### Competitive ELISA Screen for Antibodies and Antibody Fragments that Compete with a Reference Antibody or Antibody Fragment for Binding to TNFR1

This method can be used to rapidly screen diverse sets of crude antibody or antibody fragment preparations that bind TNFR1 for those antibodies or antibody fragments that compete with a reference antibody or antibody fragment (*e.g*., TAR2m-21-23) for binding to TNFR1 or bind a desired domain of TNFR1 (*e.g*., domain 1). The method uses a reference antibody or antibody fragment and test antibody or antibody fragment (e.g., a population of antibodies to be screened) that contain different detectable tags (epitope tags).

A 96 well assay plate will be coated overnight at 4°C with 100µl per well of murine or human TNFR1. Wells will be washed 3 times with 0.1% TPBS (Phosphate buffered saline containing Tween-20 at a concentration of 0.1%). 200µl per well of 1% TPBS (1% Tween-20 in PBS) will be added to block the plate, and the plate will be incubated for 1-2 hours at room temperature. Wells will then be washed 3 times with PBS. At the same time the crude antibody preparations to be tested will be mixed with a pre-optimised concentration of reference antibody or antibody fragment (*e.g*., domain 1-binding antibody; TAR2m-21-23) in solution. As already stated is important that this antibody does not include the same detection tags as present in the antibodies being screened for domain binding specificity. 50µl of this crude antibody/reference antibody mix, will be added to the ELISA plate. The plate will then be incubated for 1 hour at room temperature. After, the plate will be washed 5 times with 0.1 % TPBS, 100µl of a primary detecting antibody (that binds the tag present only on the antibody population being screened) will be added in 0.1 % TPBS to each well, and the plate will be incubated for 1 hour at room temperature. This primary antibody solution will be discarded and the plate will then be washed 5 times with 0.1% TPBS. 100µl of a prediluted secondary antibody-HRP conjugate that recognises the primary detection antibody will then be added, and the plate will be incubated for 1 hour at room temperature. The secondary antibody solution will then be discarded and the plate washed 6 times with 0.1% TPBS followed by 2 washes with PBS. 50µl of TMB peroxidase solution will then be added to each well and the plate will be left at room temperature for 2-60 minutes. The reaction will be stopped by the addition of 50 µl of 1M hydrochloric acid. The OD at 450 nm of the plate will be read in a 96-well plate reader within 30 minutes of acid addition. A separate and parallel ELISA using this method but without addition of the reference antibody or antibody fragment should be done in parallel. A reduction in ELISA signal in the presence of the reference antibody or antibody fragment, in comparison to the ELISA signal for the same antibody preparation without competing reference antibody or antibody fragment, will be indicative that the particular antibody or antibody fragment competes with the reference antibody or antibody fragment for binding to TNFR1, and binds the same domain of TNFR1 as the reference antibody or antibody fragment.

### SPR Screening

The ELISA methods described above can be readily adapted to a format that uses surface plasmon resonance, for example using a BIACORE 3000 SPR instrument (Biacore International AB, Uppsala, Sweden). Generally, the chimeric protein will be either immobilized on the SPR chip, or the chimeric protein will be equilibrated with crude bacterial supernatant containing anti- TNFR1 antibodies or antibody fragments, and the resultant mixture flowed over a SPR chip coated with full length human TNFR1 or murine TNFR1.

### Example 5. PEGylation of dAbs

The effects of PEGylation of dAbs on the ability of the dAbs to bind TNFR1 in a cell free receptor binding assay and to inhibit function of TNFR1 on the surface of cells in a cell based assay was studied.

### Cell assay

### MRC-5 IL-8 release assay

The activities of certain dAbs that bind human TNFR1 were assessed in the following MRC-5 cell assay. The assay is based on the induction of IL-8 secretion by TNF in MRC-5 cells and is adapted from the method described in Alceson, L. et al. Journal of Biological Chemistry 271:30517-30523 (1996), describing the induction of IL-8 by IL-1 in HUVEC. The activity of the dAbs was assayed by assessing IL-8 induction by human TNFa using MRC-5 cells instead of the HUVEC cell line. Briefly, MRC-5 cells were plated in microtitre plates and the plates were incubated overnight with dAb and human TNFa (300 pg/ml). Following incubation, the culture supernatant was aspirated and the IL-8 concentration in the supernatant was measured via a sandwich ELISA (R&D Systems). Anti-TNFR1 dAb activity resulted in a decrease in IL-8 secretion into the supernatant compared with control wells that were incubated with TNFa only.

### Receptor binding assay

Anti-TNF dAbs were tested for the ability to inhibit the binding of TNF to recombinant TNF receptor 1 (p55). Briefly, Maxisorp plates were incubated overnight with 30mg/ml anti-human Fc mouse monoclonal antibody (Zymed, San Francisco, USA). The wells were washed with phosphate buffered saline (PBS) containing 0.05% Tween-20 and then blocked with 1% BSA in PBS before being incubated with 100ng/ml TNF receptor 1 Fc fusion protein (R&D Systems, Minneapolis, USA). Anti-TNF dAb was mixed with TNF which was added to the washed wells at a final concentration of 10ng/ml. TNF binding was detected with 0.2mg/ml biotinylated anti-TNF antibody (HyCult biotechnology, Uben, Netherlands) followed by 1 in 500 dilution of horse radish peroxidase labelled streptavidin (Amersham Biosciences, UK) and then incubation with TMB substrate (KPL, Gaithersburg, USA). The reaction was stopped by the addition of HCl and the absorbance was read at 450nm. Anti-TNF dAb activity lead to a decrease in TNF binding and therefore a decrease in absorbance compared with the TNF only control.

The comparative effects of PEGylation dAbs on recombinant and cell surface TNFR1 are illustrated in the data presented in Table 5.

**Table 5.**

| dAb Format | RBA IC50 (nM) | Cell assay ND50 (nM) |
|---|---|---|
| anti-TNFR dAb | 3 | 30 |
| (no PEG) | | |
| 5K PEG | 3 | 200 |
| 20K PEG | 6 | 600 |
| 2x10K PEG | 10 | 1000 |
| 30K PEG | 10 | 600 |
| 2x20K PEG | 10 | 1000 |

The receptor binding assay (RBA), which utilises a recombinant form of receptor, containing only the extracellular portion of the receptor in a non-cell based assay format, is shown on the left, where the effects on PEGylation are minimal, and in the case of 5K PEG, no difference in binding was observed.

In contrast, as seen in the data obtained in the cell assay, PEGylation of dAbs substantially reduced the effectiveness of the dAbs in the assay. In some cases, effectiveness with reduced up to a factor of 33-fold. The data collected in cell based assay where based on the ability of the dAbs to block receptor activity at the cell surface.

The results of the study show that PEGylation of dAbs had a much greater effect on the ability of the dAbs to inhibit function of TNFR1 on the cell surface than on the ability to bind TNFR1 in a cell free assay.

Example 6. dAbs That Bind Endogenous Agonist Molecules But Do Not Bind The Active Site.

This example illustrates a method for identify dAbs that bind a desired endogenous agonist molecule but do not bind the active site of the endogenous agonist. The approach is illustrated using erythropoietin (epo), and is generally applicable to endogenous agonists.

Domain antibodies that bind EPO can be identified and isolated using any suitable screening method. A cell line that expresses recombinant EPO receptor and that is dependent upon hematopoietic factors for growth can be used in an assay to identify dAbs that bind EPO but do not bind in the active site, and therefore do not prevent binding of EPO to the EPO receptor. For example Ba/F3 cells, which is a murine IL-3 dependent cell line that has been transfected with human EPO receptor and undergoes epo-dependent growth can be used. (D'Andrea et al., Blood 82:46-52 (1993). Ba/F3 cells can be cultured in a suitable culture dish, the culture media can be removed and the cells can be washed using a suitable buffer such as PBS. The cells can then be cultured for about 48 hours in culture media that contains EPO. Domain antibodies to be assayed can be added to individual cultures (e.g., individual wells of a suitable cell culture assay plate). After about 48 hours, cell proliferation can be assessed using any suitable method, such as the dimethylthiazol diphenyl-tetrazolium brimide (MTT) reduction assay. A lack of inhibition of cell proliferation is wells that contain a dAb relative to control wells that contain culture media but not dAb will indicate that the dAb does not bind the active site of epo.

Representative embodiments of the invention include:
1. Use of a ligand comprising a moiety that has a binding site for an endogenous target compound for the manufacture of a medicament for increasing the amount of said endogenous target compound in a subject, wherein said ligand binds said endogenous target compound and does not substantially inhibit the activity of said endogenous target compound.
2. Use of a ligand comprising a moiety that has a binding site for an endogenous target compound for the manufacture of a medicament for increasing the bioavailability said endogenous target compound in a subject, wherein said ligand binds said endogenous target compound and does not substantially inhibit the activity of said endogenous target compound.
3. Use of a ligand comprising a moiety that has a binding site for an endogenous target compound for the manufacture of a medicament for increasing the *in vivo* half-life of said endogenous target compound, wherein said ligand binds said endogenous target compound, and does not substantially inhibit the activity of said endogenous target compound.
4. Use of a ligand comprising a moiety that has a binding site for an endogenous target compound for the manufacture of a medicament for local administration that increases the amount of said endogenous target compound at the site of administration, wherein said ligand binds said endogenous target compound, and does not substantially inhibit the activity of said endogenous target compound.
5. The use of any one of embodiments 1 through 4 wherein the amount of endogenous target ligand in said subject about 4 hours after administration of said medicament is increased by at least 1.5 times, relative to the amount before administration of said medicament.
6. The use of embodiment 5 wherein the amount of endogenous target ligand in said subject about 4 hours after administration of said medicament is increased by at least 10 times, relative to the amount before administration of said medicament.
7. The use of any one of embodiments 1 through 6 wherein said ligand binds said endogenous target compound and increases the *in vivo* half life of said endogenous target compound by at least 1.5 times.
8. The use of embodiment 7 wherein said ligand binds said endogenous target compound and increases the *in vivo* half life of said endogenous target compound by at least 10 times.
9. The use of any one of embodiments 1 through 6 wherein said ligand inhibits the activity of said endogenous target compound by no more than about 10%.
10. The use of any one of embodiments 1 through 6 wherein the inhibitory concentration 50 (IC50) of said ligand is at least 1 micromolar.
11. The use of any one of embodiments 1 through 10 wherein said ligand binds said endogenous target compound but does not bind to the active site of said endogenous target compound.
12. The use of any one of embodiments 1 through 11 wherein said ligand comprises two or more copies of said moiety that has a binding site for an endogenous target.
13. The use of embodiment 12 wherein said ligand is a dimer of said moiety that has a binding site for an endogenous target.
14. The use of any one of embodiments 1-13 wherein said moiety that has a binding site for an endogenous target compound is selected from the group consisting of an affibody, an SpA domain, an LDL receptor class A domain, an EGF domain, and an avimer.
15. The use of any one of embodiments 1-14 wherein said moiety that has a binding site for an endogenous target compound is an antibody or antibody fragment.
16. The use of embodiment 15 wherein said moiety that has a binding site for an endogenous target compound is an antibody fragment selected from the group consisting of a Fv fragment, a single chain Fv fragment, a disulfide bonded Fv fragment, a Fab fragment, a Fab' fragment, a F(ab')₂ fragment, a diabody, an immunoglobulin single variable domain.
17. The use of embodiment 16 wherein said moiety that has a binding site for an endogenous target compound is an immunoglobulin single variable domain.
18. The use of embodiment 17 wherein said immunoglobulin single variable domain is selected from the group consisting of a human V_{H}, and a human V_{L}.
19. The use of any one of embodiments 1 through 18 wherein said ligand further comprises a half-life extending moiety.
20. The use of embodiment 19 wherein said half-life extending moiety is a polyalkylene glycol moiety, serum albumin or a fragment thereof, transferrin receptor or a transferrin-binding portion thereof, or a moiety comprising a binding site for a polypeptide that enhances half-life *in vivo.*
21. The use of embodiment 20 wherein said half-life extending moiety is an antibody or antibody fragment that has a binding site for a polypeptide that enhances half-life *in vivo.*
22. The use of embodiment 21 wherein said antibody or antibody fragment is a dAb.
23. The use of embodiment 20 wherein said half-life extending moiety is an moiety comprising a binding site for a polypeptide that enhances half-life *in vivo* selected from the group consisting of an affibody, an SpA domain, an LDL receptor class A domain, an EGF domain, and an avimer.
24. The use of any one of embodiments 21 through 23 wherein polypeptide that enhances half-life *in vivo* is serum albumin or neonatal Fc receptor.
25. The use of embodiment 20, wherein said half-life extending moiety is a polyethylene glycol moiety.
26. The use of any one of embodiments 1 through 25 wherein said medicament is substantially non-immunogenic.
27. The use of any one of embodiments 1 through 26 wherein said medicament is a depot formulation.
28. The use of any one of embodiments 1 through 27 wherein said endogenous target compound is selected from the group consisting of a soluble cytokine receptor, an endogenous receptor antagonist, an enzyme, a cytokine, a growth factor, and a hormone.
29. The use of embodiment 28 wherein said endogenous target compound is a soluble cytokine receptor.
30. The use of embodiment 28 wherein said soluble cytokine receptor is soluble TNFR1
31. The use of any one of embodiments 1-30 wherein the moiety that has a binding site for an endogenous target compound binds said endogenous target compound with a Kd of 1 x 10⁻⁷ M or less.
32. The use of any one of embodiments 1-30 wherein the ligand binds said endogenous target compound with a Kd of 1 x 10⁻⁷ M or less.
33. Use of a ligand comprising a moiety that has a binding site for an endogenous target compound for the manufacture of a medicament for increasing the activity of said endogenous target, wherein said ligand binds said endogenous target and does not substantially inhibit the activity of said endogenous target compound.
34. The use of embodiment 33 wherein said ligand does not bind to the active site of said endogenous target compound.
35. The use of embodiment 33 or embodiment 34 wherein said ligand inhibits the activity of said endogenous target compound by no more than about 10%.
36. The use of embodiment 33 or embodiment 34 wherein the inhibitory concentration 50 (IC50) of said ligand is at least 1 micromolar.
37. Use of any one of embodiments 33-36 wherein said ligand comprises two or more moieties that have a binding site for said endogenous target.
38. The use of any one of embodiments 33-36 wherein said ligand comprises two or more copies of said moiety that has a binding site for an endogenous target.
39. The use of embodiment 38 wherein said ligand is a dimer of said moiety that has a binding site for an endogenous target.
40. The use of any one of embodiments 33-39 wherein said moiety that has a binding site for an endogenous target compound is selected from the group consisting of an affibody, an SpA domain, an LDL receptor class A domain, an EGF domain, and an avimer.
41. The use of any one of embodiments 33-40 wherein said moiety that has a binding site for an endogenous target compound is an antibody or antibody fragment.
42. The use of embodiment 41 wherein said moiety that has a binding site for an endogenous target compound is an antibody fragment selected from the group consisting of a Fv fragment, a single chain Fv fragment, a disulfide bonded Fv fragment, a Fab fragment, a Fab' fragment, a F(ab')₂ fragment, a diabody, an immunoglobulin single variable domain.
43. The use of embodiment 42 wherein said moiety that has a binding site for an endogenous target compound is an immunoglobulin single variable domain.
44. The use of embodiment 43 wherein said immunoglobulin single variable domain is selected from the group consisting of a human V_{H}, and a human V_{L}.
45. The use of any one of embodiments 33 through 44 wherein said ligand further comprises a half-life extending moiety.
46. The use of embodiment 45 wherein said half-life extending moiety is a polyalkylene glycol moiety, serum albumin or a fragment thereof, transferrin receptor or a transferrin-binding portion thereof, or a moiety comprising a binding site for a polypeptide that enhances half-life *in vivo.*
47. The use of embodiment 46 wherein said half-life extending moiety is an antibody or antibody fragment that has a binding site for a polypeptide that enhances half-life *in vivo.*
48. The use of embodiment 47 wherein said antibody or antibody fragment is a dAb.
49. The use of embodiment 46 wherein said half-life extending moiety is an moiety comprising a binding site for a polypeptide that enhances half-life *in vivo* selected from the group consisting of an affibody, an SpA domain, an LDL receptor class A domain, an EGF domain, and an avimer.
50. The use of any one of embodiments 47 through 49 wherein polypeptide that enhances half-life *in vivo* is serum albumin or neonatal Fc receptor.
51. The use of embodiment 46 wherein said half-life extending moiety is a polyethylene glycol moiety.
52. The use of any one of embodiments 33 through 51 wherein said medicament is substantially non-immunogenic.
53. The use of any one of embodiments 1 through 27 wherein said medicament is a depot formulation.
54. The use of any one of embodiments 33 through 53 wherein said endogenous target compound is selected from the group consisting of a soluble cytokine receptor, an endogenous receptor antagonist, an enzyme, a cytokine, a growth factor, and a hormone.
55. The use of embodiment 54 wherein said endogenous target compound is a soluble cytokine receptor.
56. The use of embodiment 55 wherein said soluble cytokine receptor is soluble TNFR1
57. The use of any one of embodiments 33-56 wherein the moiety that has a binding site for an endogenous target compound binds said endogenous target compound with a Kd of 1 x 10⁻⁷ M or less.
58. The use of any one of embodiments 33-56 wherein the ligand binds said endogenous target compound with a Kd of 1 x 10⁻⁷ M or less.
59. Use of a ligand comprising two or more moieties that have a binding site for an endogenous target compound for the manufacture of a medicament for increasing the binding activity of said endogenous target compound, wherein said ligand binds said endogenous target, does not bind the active site of said endogenous target, and does not substantially inhibit the binding activity of said endogenous target compound.
60. The use of embodiment 59 wherein said ligand comprises two copies of a moiety that has a binding site for an endogenous target.
61. The use of embodiment 60 wherein said ligand is a dimer of said moiety that has a binding site for an endogenous target.
62. The use of any one of embodiments 59-61 wherein said binding activity is increased by at least a factor of 10.
63. The use of any one of embodiments 59-62 wherein said endogenous target compound is a soluble cytokine receptor.
64. The use of embodiment 63 wherein said soluble cytokine receptor is soluble TNFR1, and said moieties that have a binding site for an endogenous target compound independently bind a domain of TNFR1 selected from the group consisting of Domain 1 and Domain 4.
65. The use of any one of embodiments 59-64 wherein said moiety that has a binding site for an endogenous target compound is selected from the group consisting of an affibody, an SpA domain, an LDL receptor class A domain, an EGF domain, and an avimer.
66. The use of any one of embodiments 59-64 wherein said moiety that has a binding site for an endogenous target compound is an antibody or antibody fragment.
67. The use of embodiment 66 wherein said moiety that has a binding site for an endogenous target compound is an antibody fragment selected from the group consisting of a Fv fragment, a single chain Fv fragment, a disulfide bonded Fv fragment, a Fab fragment, a Fab' fragment, a F(ab')₂ fragment, a diabody, an immunoglobulin single variable domain.
68. The use of embodiment 67 wherein said moiety that has a binding site for an endogenous target compound is an immunoglobulin single variable domain.
69. The use of embodiment 68 wherein said immunoglobulin single variable domain is selected from the group consisting of a human V_{H}, and a human V_{L}.
70. The use of any one of embodiments 59 through 69 wherein said ligand further comprises a half-life extending moiety.
71. The use of embodiment 70 wherein said half-life extending moiety is a polyalkylene glycol moiety, serum albumin or a fragment thereof, transferrin receptor or a transferrin-binding portion thereof, or a moiety comprising a binding site for a polypeptide that enhances half-life *in vivo.*
72. The use of embodiment 71 wherein said half-life extending moiety is an antibody or antibody fragment that has a binding site for a polypeptide that enhances half-life *in vivo.*
73. The use of embodiment 72 wherein said antibody or antibody fragment is a dAb.
74. The use of embodiment 71 wherein said half-life extending moiety is an moiety comprising a binding site for a polypeptide that enhances half-life *in vivo* selected from the group consisting of an affibody, an SpA domain, an LDL receptor class A domain, an EGF domain, and an avimer.
75. The use of any one of embodiments 72 through 74 wherein polypeptide that enhances half-life *in vivo* is serum albumin or neonatal Fc receptor.
76. The use of embodiment 71 wherein said half-life extending moiety is a polyethylene glycol moiety.
77. The use of any one of embodiments 59 through 76 wherein said medicament is substantially non-immunogenic.
78. The use of any one of embodiments 59 through 77 wherein said medicament is a depot formulation.
79. The use of any one of embodiments 59-78 wherein the moiety that has a binding site for an endogenous target compound binds said endogenous target compound with a Kd of 1 x 10⁻⁷ M or less.
80. The use of any one of embodiments 59-78 wherein the ligand binds said endogenous target compound with a Kd of 1 x 10⁻⁷ M or less.
81. A ligand that binds an endogenous target compound having activity suitable for treating a disease in a subject, wherein said ligand does not bind the active site of said endogenous target compound or substantially inhibit the activity of said endogenous target compound, for use in therapy of a disease amenable to treatment with said endogenous target compound.
82. The ligand of embodiment 81 wherein said ligand increases the in vivo half-life of said endogenous target compound.
83. The ligand of embodiment 81 or embodiment 82 wherein said ligand increases the amount of said endogenous target compound in a subject.
84. The ligand of any one of embodiments 81-83 wherein said ligand increases the bioavailability of said endogenous target compound.
85. The ligand of any one of embodiments 81-84 wherein said ligand increases the binding activity of said endogenous compound.
86. The ligand of any one of embodiments 81-85 wherein said ligand is as described in any one of embodiments 1-80.
87. A pharmaceutical composition comprising a ligand that binds an endogenous target compound having activity suitable for treating a disease in a subject and a physiologically acceptable carrier, wherein said ligand does not bind the active site of said endogenous target compound or substantially inhibit the activity of said endogenous target compound.
88. The pharmaceutical composition of embodiment 87 wherein said ligand increases the in vivo half-life of said endogenous target compound.
89. The pharmaceutical composition of embodiment 87 or embodiment 88 wherein said ligand increases the amount of said endogenous target compound in a subject.
90. The pharmaceutical composition of any one of embodiments 87-89 wherein said ligand increases the bioavailability of said endogenous target compound.
91. The pharmaceutical composition of any one of embodiments 87-90 wherein said ligand increases the binding activity of said endogenous compound.
92. The pharmaceutical composition of any one of embodiments 87-91 wherein said ligand is as described in any one of embodiments 1-80.
93. A drug delivery device comprising a pharmaceutical composition of any one of embodiments 87-92.
94. The drug delivery device of embodiment 93 wherein said drug delivery device is selected from the group consisting of a parenteral delivery device, intravenous delivery device, intramuscular delivery device, intraperitoneal delivery device, transdermal delivery device, pulmonary delivery device, intraarterial delivery device, intrathecal delivery device, intraarticular delivery device, subcutaneous delivery device, intranasal delivery device, vaginal delivery device, and rectal delivery device.
95. The drug delivery device of embodiment 94 wherein said device is selected from the group consisting of a syringe, a transdermal delivery device, a capsule, a tablet, a nebulizer, an inhaler, an atomizer, an aerosolizer, a mister, a dry powder inhaler, a metered dose inhaler, a metered dose sprayer, a metered dose mister, a metered dose atomizer, a catheter.
96. Use of a ligand comprising a binding moiety that has a binding site for an endogenous target compound for increasing the half-life, bioavailability or activity of said endogenous compound, wherein said binding moiety that has a binding site for an endogenous compound is said endogenous compound or a portion or variant thereof, and wherein said ligand binds said endogenous target compound and does not substantially inhibit the activity of said endogenous target compound.
97. The use of embodiment 96 wherein said endogenous compound is a soluble receptor that is a member of the TNF receptor superfamily.
98. Use of a ligand comprising a binding moiety that has a binding site for a member of the TNF receptor superfamily for increasing the half-life, bioavailability or activity of said member of the TNF receptor superfamily, wherein said ligand binds said member of the TNF receptor superfamily and does not substantially inhibit the activity of said member of the TNF receptor superfamily, and wherein said binding moiety that has a binding site for a member of the TNF receptor superfamily is a pre-ligand assembly domain (PLAD) or a variant thereof.
99. A domain antibody comprising a binding site for soluble tumor necrosis factor receptor 1 for use as a medicament wherein the domain antibody binds soluble tumor necrosis factor receptor 1 and does not substantially inhibit the activity of soluble tumor necrosis factor receptor 1.
100. A domain antibody as defined in embodiment 1 wherein the medicament is for the treatment of a disease which is an acute and/or chronic inflammatory disease, cancer, such as lymphomas (including B cell lymphomas, T cell lymphomas, acute myeloid lymphoma), multiple myeloma, lung cancer, carcinomas), a metabolic disease (including diabetes, obesity), allergic hypersensitivity, bacterial or viral infection, autoimmune disorders (including Type I diabetes, asthma, multiple sclerosis, arthritis, rheumatoid arthritis, juvenile rheumatoid arthritis, psoriatic arthritis, spondylarthropathy, ankylosing spondylitis), systemic lupus erythematosus, inflammatory bowel disease, Crohn's disease, ulcerative colitis, myasthenia gravis and Behcet's syndrome, psoriasis, endometriosis, abdominal adhesions, post abdominal surgery, osteoarthritis or chronic obstructive pulmonary disease.
101. Use of a domain antibody comprising a binding site for soluble tumor necrosis factor receptor 1; wherein the domain antibody binds soluble tumor necrosis factor receptor 1 and does not substantially inhibit the activity of soluble tumor necrosis factor receptor 1 for the manufacture of a medicament for use in the treatment of a disease wherein the disease is an acute and/or chronic inflammatory disease, cancer, such as lymphomas (including B cell lymphomas, T cell lymphomas, acute myeloid lymphoma), multiple myeloma, lung cancer, carcinomas), a metabolic disease (including diabetes, obesity), allergic hypersensitivity, bacterial or viral infection, autoimmune disorders (including Type I diabetes, asthma, multiple sclerosis, arthritis, rheumatoid arthritis, juvenile rheumatoid arthritis, psoriatic arthritis, spondylarthropathy, ankylosing spondylitis), systemic lupus erythematosus, inflammatory bowel disease, Crohn's disease, ulcerative colitis, myasthenia gravis and Behcet's syndrome, psoriasis, endometriosis, abdominal adhesions, post abdominal surgery, osteoarthritis or chronic obstructive pulmonary disease.
102. The use of embodiment 101 wherein the amount of soluble tumor necrosis factor receptor 1 about 4 hours after administration of said medicament to a subject is increased by at least 1.5 times, relative to the amount before administration of said medicament.
103. The use of embodiment 102 wherein the amount of soluble tumor necrosis factor receptor 1 about 4 hours after administration of said medicament to a subject is increased by at least 10 times, relative to the amount before administration of said medicament.
104. A domain antibody or use as defined in any one of embodiments 99 through 103 wherein said domain antibody binds soluble tumor necrosis factor receptor 1 and increases the *in vivo* half life of soluble tumor necrosis factor receptor 1 by at least 1.5 times.
105. A domain antibody or use as defined in embodiment 104 wherein said domain antibody binds soluble tumor necrosis factor receptor 1 and increases the *in vivo* half life of soluble tumor necrosis factor receptor 1 by at least 10 times.
106. A domain antibody or use as defined in any one of embodiments 99 through 103 wherein said domain antibody inhibits the activity of soluble tumor necrosis factor receptor 1 compound by no more than about 10%.
107. A domain antibody or use as defined in any one of embodiments 99 through 103 wherein the inhibitory concentration 50 (IC50) of said domain antibody is at least 1 micromolar.
108. A domain antibody or use as defined in any one of embodiments 99 through 107 wherein said domain antibody binds soluble tumor necrosis factor receptor 1 but does not bind to the active site of soluble tumor necrosis factor receptor 1.
109. A domain antibody or use as defined in any of embodiments 99 to 108 wherein said domain antibody binds a domain of TNFR1 selected from the group consisting of Domain 1 and Domain 4.
110. A domain antibody or use as defined in any of embodiments 99 to 109 wherein said domain antibody is selected from the group consisting of a human VH, and a human VL.
111. A domain antibody or use as defined in any one of embodiments 99 to 110 wherein said domain antibody further comprises a half-life extending moiety.
112. A domain antibody or use as defined in embodiment 111 wherein said half-life extending moiety is a polyalkylene glycol moiety, serum albumin or a fragment thereof, transferring receptor or a transferrin-binding portion thereof, an antibody Fc region or a moiety comprising a binding site for a polypeptide that enhances half-life *in vivo.*
113. A domain antibody or use as defined in embodiment 112 wherein said half-life extending moiety is an antibody or antibody fragment that has a binding site for a polypeptide that enhances half-life *in vivo.*
114. A domain antibody or use as defined in embodiment 113 wherein said antibody or antibody fragment is a dAb.
115. A domain antibody or use as defined in embodiment 112 wherein said half-life extending moiety is an moiety comprising a binding site for a polypeptide that enhances half-life *in vivo* selected from the group consisting of an affibody, an SpA domain, an LDL receptor class A domain, an EGF domain, and an avimer.
116. A domain antibody or use as defined in any one of embodiments 112 through 115 wherein the polypeptide that enhances half-life *in vivo* is serum albumin or neonatal Fc receptor.
117. A domain antibody or use as defined in embodiment 112 wherein said half-life extending moiety is a polyethylene glycol moiety.
118. A domain antibody or use as defined in any one of embodiments 99 to 117 wherein said medicament is substantially non-immunogenic.
119. A domain antibody or use as defined in any one of embodiments 99 to 118 wherein said medicament is a depot formulation.
120. A domain antibody or use as defined in any one of embodiments 99 to 119 wherein said medicament comprises a dimer of the domain antibody.
121. A domain antibody or use as defined in any one of embodiments 99 to 120 wherein the moiety that has a binding site for an endogenous target compound binds said endogenous target compound with a Kd of 1 x 10-7 M or less.
122. A domain antibody or use as defined in any one of embodiments 99 to 120 wherein the ligand binds said endogenous target compound with a Kd of 1 x 10-7 M or less.
123. A domain antibody or use as defined in any of embodiments 99 to 122 wherein the ligand further comprises a half-life extending moiety that binds albumin.
124. A domain antibody or use as defined in any of embodiments 99 to 123 wherein the domain antibody comprises a framework selected from the group consisting of a DPK9 VL framework and a DP47 VH framework.
125. A domain antibody or use as defined in any of embodiments 99 to 124 wherein the domain antibody comprises a binding site for soluble tumor necrosis
126. A domain antibody or use as defined in embodiment 125, wherein the pre-ligand assembly domain comprises at least 10 contiguous amino acid residues of an amino acid sequence selected from the group consisting of SEQ ID NO: 28.
127. A pharmaceutical composition comprising a domain antibody as defined in any of embodiments 99 to 126 and a physiologically acceptable carrier.
128. A drug delivery device comprising a pharmaceutical composition of embodiment 127.
129. The drug delivery device of embodiment 128 wherein said drug delivery device is selected from the group consisting of a parenteral delivery device, intravenous delivery device, intramuscular delivery device, intraperitoneal delivery device, transdermal delivery device, pulmonary delivery device, intraarterial delivery device, intrathecal delivery device, intraarticular delivery device, subcutaneous delivery device, intranasal delivery device, vaginal delivery device, and rectal delivery device.
130. The drug delivery device of embodiment 129 wherein said device is selected from the group consisting of a syringe, a transdermal delivery device, a capsule, a tablet, a nebulizer, an inhaler, an atomizer, an aerosolizer, a mister, a dry powder inhaler, a metered dose inhaler, a metered dose sprayer, a metered dose mister, a metered dose atomizer, a catheter.

All publications mentioned in the present specification, and references cited in said publications, are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. Use of a ligand comprising a moiety that has a binding site for an endogenous target compound for the manufacture of a medicament for increasing the amount of said endogenous target compound in a subject, wherein said ligand binds said endogenous target compound and does not substantially inhibit the activity of said endogenous target compound.

2. Use of a ligand comprising a moiety that has a binding site for an endogenous target compound for the manufacture of a medicament for increasing the bioavailability said endogenous target compound in a subject, wherein said ligand binds said endogenous target compound and does not substantially inhibit the activity of said endogenous target compound.

3. Use of a ligand comprising a moiety that has a binding site for an endogenous target compound for the manufacture of a medicament for increasing the *in vivo* half-life of said endogenous target compound, wherein said ligand binds said endogenous target compound, and does not substantially inhibit the activity of said endogenous target compound.

4. Use of a ligand comprising a moiety that has a binding site for an endogenous target compound for the manufacture of a medicament for local administration that increases the amount of said endogenous target compound at the site of administration, wherein said ligand binds said endogenous target compound, and does not substantially inhibit the activity of said endogenous target compound.

5. Use of any of claims 1 to 4 wherein said endogenous target compound is a soluble cytokine receptor, such as a member of the TNF receptor superfamily.

6. A ligand comprising a moiety that has a binding site for an endogenous target compound for use in increasing the activity of said endogenous target, wherein said ligand binds said endogenous target and does not substantially inhibit the activity of said endogenous target compound.

7. A ligand comprising two or more moieties that have a binding site for an endogenous target compound for use in increasing the binding activity of said endogenous target compound, wherein said ligand binds said endogenous target, does not bind the active site of said endogenous target, and does not substantially inhibit the binding activity of said endogenous target compound.

8. A ligand that binds an endogenous target compound having activity suitable for treating a disease in a subject, wherein said ligand does not bind the active site of said endogenous target compound or substantially inhibit the activity of said endogenous target compound, for use in therapy of a disease amenable to treatment with said endogenous target compound.

9. A ligand comprising a binding moiety that has a binding site for an endogenous target compound for use in increasing the half-life, bioavailability or activity of said endogenous compound, wherein said binding moiety that has a binding site for an endogenous compound is said endogenous compound or a portion or variant thereof, and wherein said ligand binds said endogenous target compound and does not substantially inhibit the activity of said endogenous target compound.

10. A ligand of any of claims 6 to 9 wherein said endogenous target compound is a soluble cytokine receptor, such as a member of the TNF receptor superfamily.

11. A pharmaceutical composition comprising a ligand that binds an endogenous target compound having activity suitable for treating a disease in a subject and a physiologically acceptable carrier, wherein said ligand does not bind the active site of said endogenous target compound or substantially inhibit the activity of said endogenous target compound.

12. A pharmaceutical composition of claim 11 wherein said endogenous target compound is a soluble cytokine receptor, such as a member of the TNF receptor superfamily.

13. A drug delivery device comprising a pharmaceutical composition of claim11 or 12.

14. A ligand comprising a binding moiety that has a binding site for a member of the TNF receptor superfamily for use in increasing the half-life, bioavailability or activity of said member of the TNF receptor superfamily, wherein said ligand binds said member of the TNF receptor superfamily and does not substantially inhibit the activity of said member of the TNF receptor superfamily, and wherein said binding moiety that has a binding site for a member of the TNF receptor superfamily is a pre-ligand assembly domain (PLAD) or a variant thereof.

15. A domain antibody comprising a binding site for soluble tumor necrosis factor receptor 1 for use as a medicament wherein the domain antibody binds soluble tumor necrosis factor receptor 1 and does not substantially inhibit the activity of soluble tumor necrosis factor receptor 1.
